# EUROPEAN PATENT APPLICATION

(11) **EP 3 501 299 A1**
(43) Date of publication of application: **26.06.2019**
(21) Application number: 17843572.3
(22) Date of filing: 22.08.2017
(51) Int. Cl.: A24B 15/00, A24B 3/04, A01H 1/00, A01H 1/06, A01H 5/00, A01H 5/02, A01H 5/04, A01H 5/06, A01H 5/10, A01H 5/12

(54) **METHOD FOR PRODUCING CURED TOBACCO MATERIAL**

(30) Priority: 22.08.2016 JP 2016162276
(71) Applicant: JAPAN TOBACCO INC., Minato-ku Tokyo 105-8422 (JP)
(72) Inventor: TSUKUI, Satomi, Tokyo 130-8603 (JP); SANDA, Satoko, Tokyo 130-8603 (JP); UDAGAWA, Hisashi, Tokyo 130-8603 (JP); FUJIMURA, Tomoya, Tokyo 130-8603 (JP); ARAI, Masao, Tokyo 130-8603 (JP); KOIKE, Akiko, Tokyo 130-8603 (JP)
(74) Representative: Isarpatent
(86) International application number: PCT/JP2017/029901
(87) International publication number: WO 2018/038092

(57) **Abstract**

Provided is a method of producing a cured tobacco material having an enhanced threonine content. The present invention provides a method of producing a cured tobacco material having an enhanced threonine content, wherein the cured tobacco material is produced from a modified tobacco plant which has been modified such that expression or activity of an endogenous threonine aldolase is reduced or from a part of the modified tobacco plant.

SEQUENCE LISTING

<110>  JAPAN TOBACCO INC.

<120>  Methods for producing dried tabacco materials

<130>  JT17271

<150>  JP2016-162276
<151>  2016-08-22

<160>  51

<170>  PatentIn version 3.5


<210>  1
<211>  1080
<212>  DNA
<213>  Nicotiana tabacum

<400>  1
atggtgatga gaaccgtgga tcttcgctcg gacacagtca ctaaaccaac tgaagccatg      60

cggaacgcga tggccaatgc tgaagtggat gacgatgtat tgggttatga tccaactgcc     120

caacgccttg aagccgagat cgcaaggata atgggcaaag aagcagggct atttgttcct     180

tcaggcacta tgggcaacct tatcagtgtg ctgactcatt gccaaattag aggcagtgaa     240

attattcttg gtgattattc gcatatccat atttatgaaa atggaggcat tgccactctc     300

ggaggtgttc atccgaggac agtgaagaac aatgaagatg gaacaatgga tcttgatcta     360

attgaagctg caattcgaga tcctagcttt gagatatgtt acccaaccac taggctgatc     420

tgcttggaga attcacatgc acactcagga ggccgatgcc tttctgcaga gtatacagac     480

aaagtcggag agctagcaaa gaagtatggt ctgaagcttc acattgatgg agctcgtata     540

ttcaatgcat cagttgcact cggagtgcct gttcatagac ttgtacgggc tgctgattca     600

gtttcggtat gcttatcaaa aggtcttggt gctccagtcg ggtctgtggt tgttggttca     660

aagagcttca ttgccaaggc gaaaattctc aggaagactc taggcggtgg aatgaggcag     720

gttggtgtcc tttgtgctgc tgcttttatc gctttccaag agaatcttgt taagctggaa     780

ggagatcaca aaaaggctaa gattttggct gaggaactta acaaaatcaa agggctgaaa     840

gttgatattg ctgccgtaga gactaacatt gtatattgtg atatactgaa ggggtcaaag     900

ataagcgaaa cagagatgtg caagactttg gaacaacatg gtttacttat actaccagaa     960

gggccacaga gaatcagatt tgttattcat caccagattt cagaaaatga tgtgcactat    1020

gcagcgtcgt gcgttcagcg agctttggca ggagtggccg aagaaaatgg tgacaagtaa    1080


<210>  2
<211>  359
<212>  PRT
<213>  Nicotiana tabacum

<400>  2

Met Val Met Arg Thr Val Asp Leu Arg Ser Asp Thr Val Thr Lys Pro
1               5                   10                  15

Thr Glu Ala Met Arg Asn Ala Met Ala Asn Ala Glu Val Asp Asp Asp
            20                  25                  30

Val Leu Gly Tyr Asp Pro Thr Ala Gln Arg Leu Glu Ala Glu Ile Ala
        35                  40                  45

Arg Ile Met Gly Lys Glu Ala Gly Leu Phe Val Pro Ser Gly Thr Met
    50                  55                  60

Gly Asn Leu Ile Ser Val Leu Thr His Cys Gln Ile Arg Gly Ser Glu
65                  70                  75                  80

Ile Ile Leu Gly Asp Tyr Ser His Ile His Ile Tyr Glu Asn Gly Gly
                85                  90                  95

Ile Ala Thr Leu Gly Gly Val His Pro Arg Thr Val Lys Asn Asn Glu
            100                 105                 110

Asp Gly Thr Met Asp Leu Asp Leu Ile Glu Ala Ala Ile Arg Asp Pro
        115                 120                 125

Ser Phe Glu Ile Cys Tyr Pro Thr Thr Arg Leu Ile Cys Leu Glu Asn
    130                 135                 140

Ser His Ala His Ser Gly Gly Arg Cys Leu Ser Ala Glu Tyr Thr Asp
145                 150                 155                 160

Lys Val Gly Glu Leu Ala Lys Lys Tyr Gly Leu Lys Leu His Ile Asp
                165                 170                 175

Gly Ala Arg Ile Phe Asn Ala Ser Val Ala Leu Gly Val Pro Val His
            180                 185                 190

Arg Leu Val Arg Ala Ala Asp Ser Val Ser Val Cys Leu Ser Lys Gly
        195                 200                 205

Leu Gly Ala Pro Val Gly Ser Val Val Val Gly Ser Lys Ser Phe Ile
    210                 215                 220

Ala Lys Ala Lys Ile Leu Arg Lys Thr Leu Gly Gly Gly Met Arg Gln
225                 230                 235                 240

Val Gly Val Leu Cys Ala Ala Ala Phe Ile Ala Phe Gln Glu Asn Leu
                245                 250                 255

Val Lys Leu Glu Gly Asp His Lys Lys Ala Lys Ile Leu Ala Glu Glu
            260                 265                 270

Leu Asn Lys Ile Lys Gly Leu Lys Val Asp Ile Ala Ala Val Glu Thr
        275                 280                 285

Asn Ile Val Tyr Cys Asp Ile Leu Lys Gly Ser Lys Ile Ser Glu Thr
    290                 295                 300

Glu Met Cys Lys Thr Leu Glu Gln His Gly Leu Leu Ile Leu Pro Glu
305                 310                 315                 320

Gly Pro Gln Arg Ile Arg Phe Val Ile His His Gln Ile Ser Glu Asn
                325                 330                 335

Asp Val His Tyr Ala Ala Ser Cys Val Gln Arg Ala Leu Ala Gly Val
            340                 345                 350

Ala Glu Glu Asn Gly Asp Lys
        355

<210>  3
<211>  1080
<212>  DNA
<213>  Nicotiana tabacum

<400>  3
atggtgatga gaaccgtgga tcttcgctcg gacacagtca ctaaaccaac tgaagccatg      60

cggaacgcaa tggccaacgc tgaagtggat gacgatgtat tgggttatga tccaactgcc     120

caacgccttg aagccgagat cgcaaggata atgggcaaag aagcagggct atttgttcct     180

tcaggcacta tgggcaacct tatcagtgtg ctgactcatt gccaaattag aggcagtgaa     240

attattcttg gtgattattc acatatccat atttatgaaa atggaggcat tgccactctc     300

ggaggtgttc atccgaggac agtgaagaac aatgaagatg gaacaatgga tcttgattta     360

attgaagctg caattcgaga tcctagcttt gagatatgtt acccgaccac taggctgatc     420

tgcttggaga attcacatgc acactcagga ggcagatgcc tttctgcaga gtatacagac     480

aaagtcggag agctagcaaa gaagtatggt ctgaagcttc acattgatgg agctcgtata     540

ttcaatgcat cagttgcact tggagtgcct gttcatagac ttgtacgtgc tgctgattca     600

gtttcggtat gcttatcaaa aggtcttggt gctccagttg ggtctgtggt tgttggttca     660

aagagcttca ttgccaaggc aaaaattctg aggaagactc taggcggtgg aatgaggcag     720

gttggtgtcc tttgtgctgc tgcttttatc ggtttccaag agaatcttgt taagctggaa     780

ggagatcaca aaaaggctaa gattttggct gaggaactaa acaaaatcaa agggctgaaa 840

gttgatattg ctgccgtaga gactaacatt gtatattgcg atatactgaa ggggtcaaag 900

atcagcgaaa cagagatgtg caagattttg gagcaacatg gtttacttat actaccagaa 960

ggcctaatga gaatcagatt tgttattcat caccagattt cagaaaatga tgtgcactat 1020

gcggtatctt gtgttcagcg agctttagca ggagtggccg aagaaaatgg tgacaagtaa 1080

<210> 4
<211> 359
<212> PRT
<213> Nicotiana tabacum

<400> 4

Met Val Met Arg Thr Val Asp Leu Arg Ser Asp Thr Val Thr Lys Pro
1               5                   10                  15

Thr Glu Ala Met Arg Asn Ala Met Ala Asn Ala Glu Val Asp Asp Asp
            20                  25                  30

Val Leu Gly Tyr Asp Pro Thr Ala Gln Arg Leu Glu Ala Glu Ile Ala
        35                  40                  45

Arg Ile Met Gly Lys Glu Ala Gly Leu Phe Val Pro Ser Gly Thr Met
    50                  55                  60

Gly Asn Leu Ile Ser Val Leu Thr His Cys Gln Ile Arg Gly Ser Glu
65                  70                  75                  80

Ile Ile Leu Gly Asp Tyr Ser His Ile His Ile Tyr Glu Asn Gly Gly
                85                  90                  95

Ile Ala Thr Leu Gly Gly Val His Pro Arg Thr Val Lys Asn Asn Glu
            100                 105                 110

Asp Gly Thr Met Asp Leu Asp Leu Ile Glu Ala Ala Ile Arg Asp Pro
        115                 120                 125

Ser Phe Glu Ile Cys Tyr Pro Thr Thr Arg Leu Ile Cys Leu Glu Asn
    130                 135                 140

Ser His Ala His Ser Gly Gly Arg Cys Leu Ser Ala Glu Tyr Thr Asp
145                 150                 155                 160

Lys Val Gly Glu Leu Ala Lys Lys Tyr Gly Leu Lys Leu His Ile Asp
                165                 170                 175

Gly Ala Arg Ile Phe Asn Ala Ser Val Ala Leu Gly Val Pro Val His
            180                 185                 190

Arg Leu Val Arg Ala Ala Asp Ser Val Ser Val Cys Leu Ser Lys Gly
        195                 200                 205

Leu Gly Ala Pro Val Gly Ser Val Val Val Gly Ser Lys Ser Phe Ile
    210                 215                 220

Ala Lys Ala Lys Ile Leu Arg Lys Thr Leu Gly Gly Gly Met Arg Gln
225                 230                 235                 240

Val Gly Val Leu Cys Ala Ala Ala Phe Ile Gly Phe Gln Glu Asn Leu
                245                 250                 255

Val Lys Leu Glu Gly Asp His Lys Lys Ala Lys Ile Leu Ala Glu Glu
            260                 265                 270

Leu Asn Lys Ile Lys Gly Leu Lys Val Asp Ile Ala Ala Val Glu Thr
        275                 280                 285

Asn Ile Val Tyr Cys Asp Ile Leu Lys Gly Ser Lys Ile Ser Glu Thr
    290                 295                 300

Glu Met Cys Lys Ile Leu Glu Gln His Gly Leu Leu Ile Leu Pro Glu
305                 310                 315                 320

Gly Leu Met Arg Ile Arg Phe Val Ile His His Gln Ile Ser Glu Asn
                325                 330                 335

Asp Val His Tyr Ala Val Ser Cys Val Gln Arg Ala Leu Ala Gly Val
            340                 345                 350

Ala Glu Glu Asn Gly Asp Lys
        355

<210>  5
<211>  1119
<212>  DNA
<213>  Nicotiana tabacum

<400>  5
atggcggtaa gaactgtgga ttttcgatca gacacagtta ctaaaccaac tgaatccatg      60

cgtaacgcaa tggccaatgc agaagtagat gatgatgtct taggttatga tccaacggcc     120

caacgcctcg aagcagagat ggcaagaatc atgggcaaag aagcagggct atttgttcct     180

tcaggcacaa tgggcaatct tatttgcttg cttactcatt gccaaattag ggggagtgaa     240

gtaattcttg gtgataattc acatattcat gtatatgaaa atggagggat ttctacaatt     300

ggaggtgttc atcctaggac agtgaagaac aatgaagatg gaacaatgga tcttcatctg 360

attgaagctg caattcgaga ttctagtttt gaaatacttt atccaaccac taggcttatc 420

tgcttagaga attcacatgc acgatcggga ggtagatgcc ttactgtaga gtatacagac 480

aaagttggag agctagcaaa gaaatatggt ctgaagctgc atattgatgg cgctcgtata 540

ttcaatgcat cagttgcact tggtgtgccc gttcacaggc ttgtacaagc ggctgattca 600

gtttcggcat gcttatcgaa aggtcttggt gctccagcgg gatctgtgat tgttggttca 660

aagagcttca ttgccagggc aaaaattctg aggaagactc taggtggagg aatgcgacag 720

attggtgtac tctgtgctgc tgcttctgtc gctttacaag aaaatcttgt taagttggaa 780

ggagaccaca gaaaggctaa acttttagca gaggaactta acaaaatcaa agggcttaaa 840

gttgatagtg ctgctgtaga gaccaacatt gtatattgtc atatactgaa agggtcaaag 900

gtcagtagaa cacatttgtg taagattttg gaacaacatg gtatacttgt actaccagaa 960

gggccaatga gaatcaggtt tgttatacac caccagattt cagaaagtga tgtgcactat 1020

actgtctctt gccttcagca aactttggcg ggagaggcag aagaaaatgg tgatattaag 1080

catcttatga acggtttcaa acataatctt tatctgtaa 1119

<210> 6
<211> 372
<212> PRT
<213> Nicotiana tabacum

<400> 6

Met Ala Val Arg Thr Val Asp Phe Arg Ser Asp Thr Val Thr Lys Pro
1               5                   10                  15

Thr Glu Ser Met Arg Asn Ala Met Ala Asn Ala Glu Val Asp Asp Asp
            20                  25                  30

Val Leu Gly Tyr Asp Pro Thr Ala Gln Arg Leu Glu Ala Glu Met Ala
        35                  40                  45

Arg Ile Met Gly Lys Glu Ala Gly Leu Phe Val Pro Ser Gly Thr Met
    50                  55                  60

Gly Asn Leu Ile Cys Leu Leu Thr His Cys Gln Ile Arg Gly Ser Glu
65                  70                  75                  80

Val Ile Leu Gly Asp Asn Ser His Ile His Val Tyr Glu Asn Gly Gly
                85                  90                  95

Ile Ser Thr Ile Gly Gly Val His Pro Arg Thr Val Lys Asn Asn Glu
            100                 105                 110

Asp Gly Thr Met Asp Leu His Leu Ile Glu Ala Ala Ile Arg Asp Ser
        115                 120                 125

Ser Phe Glu Ile Leu Tyr Pro Thr Thr Arg Leu Ile Cys Leu Glu Asn
    130                 135                 140

Ser His Ala Arg Ser Gly Gly Arg Cys Leu Thr Val Glu Tyr Thr Asp
145                 150                 155                 160

Lys Val Gly Glu Leu Ala Lys Lys Tyr Gly Leu Lys Leu His Ile Asp
                165                 170                 175

Gly Ala Arg Ile Phe Asn Ala Ser Val Ala Leu Gly Val Pro Val His
            180                 185                 190

Arg Leu Val Gln Ala Ala Asp Ser Val Ser Ala Cys Leu Ser Lys Gly
        195                 200                 205

Leu Gly Ala Pro Ala Gly Ser Val Ile Val Gly Ser Lys Ser Phe Ile
    210                 215                 220

Ala Arg Ala Lys Ile Leu Arg Lys Thr Leu Gly Gly Gly Met Arg Gln
225                 230                 235                 240

Ile Gly Val Leu Cys Ala Ala Ala Ser Val Ala Leu Gln Glu Asn Leu
                245                 250                 255

Val Lys Leu Glu Gly Asp His Arg Lys Ala Lys Leu Leu Ala Glu Glu
            260                 265                 270

Leu Asn Lys Ile Lys Gly Leu Lys Val Asp Ser Ala Ala Val Glu Thr
        275                 280                 285

Asn Ile Val Tyr Cys His Ile Leu Lys Gly Ser Lys Val Ser Arg Thr
    290                 295                 300

His Leu Cys Lys Ile Leu Glu Gln His Gly Ile Leu Val Leu Pro Glu
305                 310                 315                 320

Gly Pro Met Arg Ile Arg Phe Val Ile His His Gln Ile Ser Glu Ser
                325                 330                 335

Asp Val His Tyr Thr Val Ser Cys Leu Gln Gln Thr Leu Ala Gly Glu
            340                 345                 350

Ala Glu Glu Asn Gly Asp Ile Lys His Leu Met Asn Gly Phe Lys His

355                 360                 365

Asn Leu Tyr Leu
    370

<210>  7
<211>  26
<212>  DNA
<213>  Artificial sequence

<220>
<223>  Forward primer

<400>  7
ggggtcaaag atcagcgaaa cagaga                                            26

<210>  8
<211>  25
<212>  DNA
<213>  Artificial sequence

<220>
<223>  Reverse primer

<400>  8
tcttcggcca ctcctgctaa agctc                                             25

<210>  9
<211>  25
<212>  DNA
<213>  Artificial sequence

<220>
<223>  Forward primer

<400>  9
tactgaaagg gtcaaaggtc agtag                                             25

<210>  10
<211>  26
<212>  DNA
<213>  Artificial sequence

<220>
<223>  Reverse primer

<400>  10
ttcttctgcc tctcccgcca aagttt                                            26

<210>  11
<211>  23
<212>  DNA
<213>  Artificial sequence

<220>
<223>  Forward primer

<400>  11
cagggaaaat ggtgatgaga acc                                              23


<210>  12
<211>  23
<212>  DNA
<213>  Artificial sequence

<220>
<223>  Reverse primer

<400>  12
atgcaccaat atgaggatcg ctg                                              23


<210>  13
<211>  23
<212>  DNA
<213>  Artificial sequence

<220>
<223>  Forward primer

<400>  13
cagggaaaat ggtgatgaga acc                                              23


<210>  14
<211>  25
<212>  DNA
<213>  Artificial sequence

<220>
<223>  Reverse primer

<400>  14
accagataat gtaccaaata gagag                                            25


<210>  15
<211>  23
<212>  DNA
<213>  Artificial sequence

<220>
<223>  tha1_s1_F primer

<400>  15
gcactatggg caaccttatc agt                                              23


<210>  16
<211>  21
<212>  DNA
<213>  Artificial sequence

<220>
<223>  tha1_s1_R primer

<400>  16
aacacctccg agagtggcaa t                                                21

<210>  17
<211>  16
<212>  DNA
<213>  Artificial sequence

<220>
<223>  tha1_s1_W probe

<400>  17
tcattgccaa attaga                                                        16


<210>  18
<211>  17
<212>  DNA
<213>  Artificial sequence

<220>
<223>  tha1_s1_M probe

<400>  18
tgctgactca ttgctaa                                                       17


<210>  19
<211>  24
<212>  DNA
<213>  Artificial sequence

<220>
<223>  tha1_s2_F primer

<400>  19
cgaggacagt gaagaacaat gaag                                               24


<210>  20
<211>  22
<212>  DNA
<213>  Artificial sequence

<220>
<223>  tha1_s2_R primer

<400>  20
tcagcctagt ggttgggtaa ca                                                 22


<210>  21
<211>  16
<212>  DNA
<213>  Artificial sequence

<220>
<223>  tha1_s2_W probe

<400>  21
aagctgcaat tcgaga                                                        16


<210>  22

<211>  14
<212>  DNA
<213>  Artificial sequence

<220>
<223>  tha1_s2_M probe

<400>  22
aagctgcaat ttga                                                          14


<210>  23
<211>  19
<212>  DNA
<213>  Artificial sequence

<220>
<223>  tha1_t_F primer

<400>  23
caatggccaa cgctgaagt                                                     19


<210>  24
<211>  21
<212>  DNA
<213>  Artificial sequence

<220>
<223>  tha1_t_R primer

<400>  24
ggttgcccat agtgcctgaa g                                                  21


<210>  25
<211>  19
<212>  DNA
<213>  Artificial sequence

<220>
<223>  tha1_t_W probe

<400>  25
ccaactgccc aacgccttg                                                     19


<210>  26
<211>  19
<212>  DNA
<213>  Artificial sequence

<220>
<223>  tha1_t_M probe

<400>  26
ccaactgcct aacgccttg                                                     19


<210>  27
<211>  23
<212>  DNA
<213>  Artificial sequence

<220>
<223>  THA1_F primer

<400>  27
taggtcaggg aaaatggtga tga                                              23


<210>  28
<211>  18
<212>  DNA
<213>  Artificial sequence

<220>
<223>  THA1_R primer

<400>  28
cgttccgcat ggcttcag                                                    18


<210>  29
<211>  24
<212>  DNA
<213>  Artificial sequence

<220>
<223>  THA1 probe

<400>  29
accgtggatc ttcgctcgga caca                                             24


<210>  30
<211>  20
<212>  DNA
<213>  Artificial sequence

<220>
<223>  EF-1_F primer

<400>  30
ctaagggtgc tgccagcttt                                                  20


<210>  31
<211>  22
<212>  DNA
<213>  Artificial sequence

<220>
<223>  EF-1_R primer

<400>  31
gtcaagcact ggagcatatc ca                                               22


<210>  32
<211>  26
<212>  DNA
<213>  Artificial sequence

<220>
<223>  EF-1 Probe

<400>  32
atcatgaacc atccaggaca gattgg                                          26

<210>  33
<211>  1080
<212>  DNA
<213>  Nicotiana tabacum

<400>  33
atggtgatga gaaccgtgga tcttcgctcg gacacagtca ctaaaccaac tgaagccatg      60

cggaacgcga tggccaatgc tgaagtggat gacgatgtat tgggttatga tccaactgcc     120

caacgccttg aagccgagat cgcaaggata atgggcaaag aagcagggct atttgttcct     180

tcaggcacta tgggcaacct tatcagtgtg ctgactcatt gctaaattag aggcagtgaa     240

attattcttg gtgattattc gcatatccat atttatgaaa atggaggcat tgccactctc     300

ggaggtgttc atccgaggac agtgaagaac aatgaagatg gaacaatgga tcttgatcta     360

attgaagctg caattcgaga tcctagcttt gagatatgtt acccaaccac taggctgatc     420

tgcttggaga attcacatgc acactcagga ggccgatgcc tttctgcaga gtatacagac     480

aaagtcggag agctagcaaa gaagtatggt ctgaagcttc acattgatgg agctcgtata     540

ttcaatgcat cagttgcact cggagtgcct gttcatagac ttgtacgggc tgctgattca     600

gtttcggtat gcttatcaaa aggtcttggt gctccagtcg ggtctgtggt tgttggttca     660

aagagcttca ttgccaaggc gaaaattctc aggaagactc taggcggtgg aatgaggcag     720

gttggtgtcc tttgtgctgc tgcttttatc gctttccaag agaatcttgt taagctggaa     780

ggagatcaca aaaaggctaa gattttggct gaggaactta acaaaatcaa agggctgaaa     840

gttgatattg ctgccgtaga gactaacatt gtatattgtg atatactgaa ggggtcaaag     900

ataagcgaaa cagagatgtg caagactttg gaacaacatg gtttacttat actaccagaa     960

gggccacaga gaatcagatt tgttattcat caccagattt cagaaaatga tgtgcactat    1020

gcagcgtcgt gcgttcagcg agctttggca ggagtggccg aagaaaatgg tgacaagtaa    1080


<210>  34
<211>  1080
<212>  DNA
<213>  Nicotiana tabacum

<400>  34
atggtgatga gaaccgtgga tcttcgctcg gacacagtca ctaaaccaac tgaagccatg      60

cggaacgcga tggccaatgc tgaagtggat gacgatgtat tgggttatga tccaactgcc     120

caacgccttg aagccgagat cgcaaggata atgggcaaag aagcagggct atttgttcct     180

tcaggcacta tgggcaacct tatcagtgtg ctgactcatt gccaaattag aggcagtgaa     240

attattcttg gtgattattc gcatatccat atttatgaaa atggaggcat tgccactctc     300

ggaggtgttc atccgaggac agtgaagaac aatgaagatg gaacaatgga tcttgatcta 360

attgaagctg caatttgaga tcctagcttt gagatatgtt acccaaccac taggctgatc 420

tgcttggaga attcacatgc acactcagga ggccgatgcc tttctgcaga gtatacagac 480

aaagtcggag agctagcaaa gaagtatggt ctgaagcttc acattgatgg agctcgtata 540

ttcaatgcat cagttgcact cggagtgcct gttcatagac ttgtacgggc tgctgattca 600

gtttcggtat gcttatcaaa aggtcttggt gctccagtcg ggtctgtggt tgttggttca 660

aagagcttca ttgccaaggc gaaaattctc aggaagactc taggcggtgg aatgaggcag 720

gttggtgtcc tttgtgctgc tgcttttatc gctttccaag agaatcttgt taagctggaa 780

ggagatcaca aaaaggctaa gattttggct gaggaactta acaaaatcaa agggctgaaa 840

gttgatattg ctgccgtaga gactaacatt gtatattgtg atatactgaa ggggtcaaag 900

ataagcgaaa cagagatgtg caagactttg gaacaacatg gtttacttat actaccagaa 960

gggccacaga gaatcagatt tgttattcat caccagattt cagaaaatga tgtgcactat 1020

gcagcgtcgt gcgttcagcg agctttggca ggagtggccg aagaaaatgg tgacaagtaa 1080

<210> 35
<211> 1080
<212> DNA
<213> Nicotiana tabacum

<400> 35
atggtgatga gaaccgtgga tcttcgctcg gacacagtca ctaaaccaac tgaagccatg 60

cggaacgcaa tggccaacgc tgaagtggat gacgatgtat tgggttatga tccaactgcc 120

taacgccttg aagccgagat cgcaaggata atgggcaaag aagcagggct atttgttcct 180

tcaggcacta tgggcaacct tatcagtgtg ctgactcatt gccaaattag aggcagtgaa 240

attattcttg gtgattattc acatatccat atttatgaaa atggaggcat tgccactctc 300

ggaggtgttc atccgaggac agtgaagaac aatgaagatg gaacaatgga tcttgattta 360

attgaagctg caattcgaga tcctagcttt gagatatgtt acccgaccac taggctgatc 420

tgcttggaga attcacatgc acactcagga ggcagatgcc tttctgcaga gtatacagac 480

aaagtcggag agctagcaaa gaagtatggt ctgaagcttc acattgatgg agctcgtata 540

ttcaatgcat cagttgcact tggagtgcct gttcatagac ttgtacgtgc tgctgattca 600

gtttcggtat gcttatcaaa aggtcttggt gctccagttg ggtctgtggt tgttggttca 660

aagagcttca ttgccaaggc aaaaattctg aggaagactc taggcggtgg aatgaggcag 720

gttggtgtcc tttgtgctgc tgcttttatc ggtttccaag agaatcttgt taagctggaa 780

ggagatcaca aaaaggctaa gattttggct gaggaactaa acaaaatcaa agggctgaaa 840

gttgatattg ctgccgtaga gactaacatt gtatattgcg atatactgaa ggggtcaaag 900

atcagcgaaa cagagatgtg caagattttg gagcaacatg gtttacttat actaccagaa     960

ggcctaatga gaatcagatt tgttattcat caccagattt cagaaaatga tgtgcactat    1020

gcggtatctt gtgttcagcg agctttagca ggagtggccg aagaaaatgg tgacaagtaa    1080

<210>  36
<211>  552
<212>  PRT
<213>  Nicotiana tabacum

<400>  36

Met Ala Ser Leu Ser His Phe Cys Gly Val Lys Thr Pro Cys Ser Ser
1               5                   10                  15

Leu Phe Lys Arg Ser Val His Phe Arg Gln Leu Asp Phe Val Ala Ser
            20                  25                  30

Val Pro Phe Ala Ser Ala Pro Ser Lys Tyr Val Lys Ala Ser Cys Cys
        35                  40                  45

Glu Arg Val Thr Cys Lys Ala Gln Ala Ala Asp Val Val Glu Leu Lys
    50                  55                  60

Glu Thr Lys Asn Glu Ala Phe Gly Glu Phe Thr Cys Val Met Lys Phe
65                  70                  75                  80

Gly Gly Ser Ser Val Ala Ser Ala Glu Arg Met Arg Glu Val Ala Asp
                85                  90                  95

Leu Ile Leu Ser Phe Pro Glu Glu Arg Pro Val Ile Val Leu Ser Ala
            100                 105                 110

Met Gly Lys Thr Thr Asn Asn Leu Leu Leu Ala Gly Glu Lys Ala Val
        115                 120                 125

Thr Cys Gly Val Ser Asn Val Cys Asp Leu Gln Glu Leu Asp Phe Ile
    130                 135                 140

Lys Asp Leu His Phe Arg Thr Ile Asp Gln Leu Gly Val Glu Lys Ser
145                 150                 155                 160

Thr Ile Ser Lys His Leu Leu Glu Leu Glu Gln Leu Leu Asn Gly Ile
                165                 170                 175

Ala Met Met Lys Glu Leu Thr Pro Arg Thr Arg Asp Tyr Leu Val Ser
            180                 185                 190

Phe Gly Glu Cys Met Ser Thr Arg Ile Phe Thr Ala Tyr Leu Asn Lys

195                 200                 205

Leu Gly Val Lys Ala Arg Gln Tyr Asp Ala Phe Glu Met Gly Ile Ile
    210                 215                 220

Thr Thr Asp Glu Phe Thr Asn Ala Asp Ile Leu Glu Ala Thr Tyr Pro
225                 230                 235                 240

Ala Val Ala Lys Arg Leu Ser Gly Asp Trp Met Asn Asn Pro Ala Ile
                245                 250                 255

Pro Ile Val Thr Gly Phe Leu Gly Lys Gly Trp Arg Thr Cys Ala Val
            260                 265                 270

Thr Thr Leu Gly Arg Gly Gly Ser Asp Leu Thr Ala Thr Thr Ile Gly
        275                 280                 285

Lys Ala Leu Gly Leu Arg Glu Ile Gln Val Trp Lys Asp Val Asp Gly
    290                 295                 300

Val Leu Thr Cys Asp Pro Asn Ile Tyr Pro Arg Ala Gln Pro Val Pro
305                 310                 315                 320

Tyr Leu Thr Phe Asp Glu Ala Ala Glu Leu Ala Tyr Phe Gly Ala Gln
                325                 330                 335

Val Leu His Pro Gln Ser Met Arg Pro Ala Arg Glu Gly Asp Ile Pro
            340                 345                 350

Val Arg Val Lys Asn Ser Tyr Asn Pro Lys Ala Pro Gly Thr Leu Ile
        355                 360                 365

Cys Arg Ala Arg Asp Met Ser Lys Ala Val Leu Thr Ser Ile Val Leu
    370                 375                 380

Lys Arg Asn Val Thr Met Leu Asp Ile Val Ser Thr Arg Met Leu Gly
385                 390                 395                 400

Gln Phe Gly Phe Phe Ala Lys Val Phe Ser Ile Phe Glu Asp Leu Gly
                405                 410                 415

Ile Ser Val Asp Val Val Ala Thr Ser Glu Val Ser Ile Ser Leu Thr
            420                 425                 430

Leu Asp Pro Ser Lys Leu Trp Ser Arg Glu Leu Ile Gln Gln Glu Leu
        435                 440                 445

Asp His Val Val Glu Glu Leu Glu Lys Ile Ala Val Val Asn Leu Leu
    450                 455                 460

Gln His Arg Ser Ile Ile Ser Leu Ile Gly Asn Val Gln Arg Ser Ser
465                 470                 475                 480

Leu Ile Leu Glu Lys Ala Phe His Val Leu Arg Thr Asn Gly Val Asn
                485                 490                 495

Val Gln Met Ile Ser Gln Gly Ala Ser Lys Val Asn Ile Ser Leu Ile
            500                 505                 510

Val Asn Asp Ser Glu Ala Glu Gln Cys Val Arg Ala Leu His Lys Thr
        515                 520                 525

Phe Phe Glu Ser Asp Leu Ser Glu Leu Val Trp Glu Asn Gly His Val
    530                 535                 540

Thr Pro Leu Ser Thr Leu Ser Thr
545                 550

<210>  37
<211>  18
<212>  DNA
<213>  Artificial sequence

<220>
<223>  Forward primer

<400>  37
actaccagaa gggccaca                                                    18

<210>  38
<211>  17
<212>  DNA
<213>  Artificial sequence

<220>
<223>  Reverse primer

<400>  38
ccaaagctcg ctgaacg                                                     17

<210>  39
<211>  21
<212>  DNA
<213>  Artificial sequence

<220>
<223>  Forward primer

<400>  39
ctaccagaag gcctaatgag a                                                21

<210>  40
<211>  20
<212>  DNA
<213>  Artificial sequence

<220>
<223>  Reverse primer

<400>  40
ctgctaaagc tcgctgaaca                                                    20


<210>  41
<211>  28
<212>  DNA
<213>  Artificial sequence

<220>
<223>  FAM/TAMRA probe

<400>  41
tcaccagatt tcagaaaatg atgtgcac                                           28


<210>  42
<211>  23
<212>  DNA
<213>  Artificial sequence

<220>
<223>  Target region for CRISPR-Cas9

<400>  42
ggaggcattg ccactctcgg agg                                                23


<210>  43
<211>  23
<212>  DNA
<213>  Artificial sequence

<220>
<223>  Forward primer

<400>  43
taggtcaggg aaaatggtga tga                                                23


<210>  44
<211>  18
<212>  DNA
<213>  Artificial sequence

<220>
<223>  Reverse primer

<400>  44
cgttccgcat ggcttcag                                                      18


<210>  45
<211>  22

<212>  DNA
<213>  Artificial sequence

<220>
<223>  Forward primer

<400>  45
ttggttcaaa gagcttcatt gc                                                22


<210>  46
<211>  21
<212>  DNA
<213>  Artificial sequence

<220>
<223>  Reverse primer

<400>  46
ccgataaaag cagcagcaca a                                                 21


<210>  47
<211>  24
<212>  DNA
<213>  Artificial sequence

<220>
<223>  FAM/TAMRA probe

<400>  47
accgtggatc ttcgctcgga caca                                              24


<210>  48
<211>  18
<212>  DNA
<213>  Artificial sequence

<220>
<223>  FAM/NFQ (MGB) probe

<400>  48
aagactctag gcggtgga                                                     18


<210>  49
<211>  1081
<212>  DNA
<213>  Nicotiana tabacum

<400>  49
atggtgatga gaaccgtgga tcttcgctcg gacacagtca ctaaaccaac tgaagccatg      60

cggaacgcga tggccaatgc tgaagtggat gacgatgtat tgggttatga tccaactgcc     120

caacgccttg aagccgagat cgcaaggata atgggcaaag aagcagggct atttgttcct     180

tcaggcacta tgggcaacct tatcagtgtg ctgactcatt gccaaattag aggcagtgaa     240

attattcttg gtgattattc gcatatccat atttatgaaa tatggaggca ttgccactct     300

cggaggtgtt catccgagga cagtgaagaa caatgaagat ggaacaatgg atcttgatct     360

aattgaagct gcaattcgag atcctagctt tgagatatgt tacccaacca ctaggctgat 420

ctgcttggag aattcacatg cacactcagg aggccgatgc ctttctgcag agtatacaga 480

caaagtcgga gagctagcaa agaagtatgg tctgaagctt cacattgatg gagctcgtat 540

attcaatgca tcagttgcac tcggagtgcc tgttcataga cttgtacggg ctgctgattc 600

agtttcggta tgcttatcaa aaggtcttgg tgctccagtc gggtctgtgg ttgttggttc 660

aaagagcttc attgccaagg cgaaaattct caggaagact ctaggcggtg gaatgaggca 720

ggttggtgtc ctttgtgctg ctgcttttat cgctttccaa gagaatcttg ttaagctgga 780

aggagatcac aaaaaggcta agattttggc tgaggaactt aacaaaatca aagggctgaa 840

agttgatatt gctgccgtag agactaacat tgtatattgt gatatactga aggggtcaaa 900

gataagcgaa acagagatgt gcaagacttt ggaacaacat ggtttactta tactaccaga 960

agggccacag agaatcagat ttgttattca tcaccagatt tcagaaaatg atgtgcacta 1020

tgcagcgtcg tgcgttcagc gagctttggc aggagtggcc gaagaaaatg gtgacaagta 1080

a 1081

<210> 50
<211> 1081
<212> DNA
<213> Nicotiana tabacum

<400> 50
atggtgatga gaaccgtgga tcttcgctcg gacacagtca ctaaaccaac tgaagccatg 60

cggaacgcaa tggccaacgc tgaagtggat gacgatgtat tgggttatga tccaactgcc 120

caacgccttg aagccgagat cgcaaggata atgggcaaag aagcagggct atttgttcct 180

tcaggcacta tgggcaacct tatcagtgtg ctgactcatt gccaaattag aggcagtgaa 240

attattcttg gtgattattc acatatccat atttatgaaa tatggaggca ttgccactct 300

cggaggtgtt catccgagga cagtgaagaa caatgaagat ggaacaatgg atcttgattt 360

aattgaagct gcaattcgag atcctagctt tgagatatgt tacccgacca ctaggctgat 420

ctgcttggag aattcacatg cacactcagg aggcagatgc ctttctgcag agtatacaga 480

caaagtcgga gagctagcaa agaagtatgg tctgaagctt cacattgatg gagctcgtat 540

attcaatgca tcagttgcac ttggagtgcc tgttcataga cttgtacgtg ctgctgattc 600

agtttcggta tgcttatcaa aaggtcttgg tgctccagtt gggtctgtgg ttgttggttc 660

aaagagcttc attgccaagg caaaaattct gaggaagact ctaggcggtg gaatgaggca 720

ggttggtgtc ctttgtgctg ctgcttttat cggtttccaa gagaatcttg ttaagctgga 780

aggagatcac aaaaaggcta agattttggc tgaggaacta aacaaaatca aagggctgaa 840

agttgatatt gctgccgtag agactaacat tgtatattgc gatatactga aggggtcaaa 900

gatcagcgaa acagagatgt gcaagatttt ggagcaacat ggtttactta tactaccaga 960

aggcctaatg agaatcagat ttgttattca tcaccagatt tcagaaaatg atgtgcacta 1020

tgcggtatct tgtgttcagc gagctttagc aggagtggcc gaagaaaatg gtgacaagta 1080

a 1081

<210> 51
<211> 1081
<212> DNA
<213> Nicotiana tabacum

<400> 51
atggtgatga gaaccgtgga tcttcgctcg gacacagtca ctaaaccaac tgaagccatg 60

cggaacgcaa tggccaacgc tgaagtggat gacgatgtat tgggttatga tccaactgcc 120

caacgccttg aagccgagat cgcaaggata atgggcaaag aagcagggct atttgttcct 180

tcaggcacta tgggcaacct tatcagtgtg ctgactcatt gccaaattag aggcagtgaa 240

attattcttg gtgattattc acatatccat atttatgaaa gatggaggca ttgccactct 300

cggaggtgtt catccgagga cagtgaagaa caatgaagat ggaacaatgg atcttgattt 360

aattgaagct gcaattcgag atcctagctt tgagatatgt tacccgacca ctaggctgat 420

ctgcttggag aattcacatg cacactcagg aggcagatgc ctttctgcag agtatacaga 480

caaagtcgga gagctagcaa agaagtatgg tctgaagctt cacattgatg gagctcgtat 540

attcaatgca tcagttgcac ttggagtgcc tgttcataga cttgtacgtg ctgctgattc 600

agtttcggta tgcttatcaa aaggtcttgg tgctccagtt gggtctgtgg ttgttggttc 660

aaagagcttc attgccaagg caaaaattct gaggaagact ctaggcggtg gaatgaggca 720

ggttggtgtc ctttgtgctg ctgcttttat cggtttccaa gagaatcttg ttaagctgga 780

aggagatcac aaaaaggcta agattttggc tgaggaacta aacaaaatca aagggctgaa 840

agttgatatt gctgccgtag agactaacat tgtatattgc gatatactga aggggtcaaa 900

gatcagcgaa acagagatgt gcaagatttt ggagcaacat ggtttactta tactaccaga 960

aggcctaatg agaatcagat ttgttattca tcaccagatt tcagaaaatg atgtgcacta 1020

tgcggtatct tgtgttcagc gagctttagc aggagtggcc gaagaaaatg gtgacaagta 1080

a 1081

SEQUENCE LISTING

<110>  JAPAN TOBACCO INC.

<120>  METHOD FOR PRODUCING CURED TOBACCO MATERIAL

<130>  P47777-WOEP

<140>  17843572.3
<141>  2017-08-22

<150>  JP2016-162276
<151>  2016-08-22

<160>  51

<170>  PatentIn version 3.5


<210>  1
<211>  1080
<212>  DNA
<213>  Nicotiana tabacum

<400>  1
atggtgatga gaaccgtgga tcttcgctcg gacacagtca ctaaaccaac tgaagccatg      60

cggaacgcga tggccaatgc tgaagtggat gacgatgtat tgggttatga tccaactgcc     120

caacgccttg aagccgagat cgcaaggata atgggcaaag aagcagggct atttgttcct     180

tcaggcacta tgggcaacct tatcagtgtg ctgactcatt gccaaattag aggcagtgaa     240

attattcttg gtgattattc gcatatccat atttatgaaa atggaggcat tgccactctc     300

ggaggtgttc atccgaggac agtgaagaac aatgaagatg gaacaatgga tcttgatcta     360

attgaagctg caattcgaga tcctagcttt gagatatgtt acccaaccac taggctgatc     420

tgcttggaga attcacatgc acactcagga ggccgatgcc tttctgcaga gtatacagac     480

aaagtcggag agctagcaaa gaagtatggt ctgaagcttc acattgatgg agctcgtata     540

ttcaatgcat cagttgcact cggagtgcct gttcatagac ttgtacgggc tgctgattca     600

gtttcggtat gcttatcaaa aggtcttggt gctccagtcg ggtctgtggt tgttggttca     660

aagagcttca ttgccaaggc gaaaattctc aggaagactc taggcggtgg aatgaggcag     720

gttggtgtcc tttgtgctgc tgcttttatc gctttccaag agaatcttgt taagctggaa     780

ggagatcaca aaaaggctaa gattttggct gaggaactta acaaaatcaa agggctgaaa     840

gttgatattg ctgccgtaga gactaacatt gtatattgtg atatactgaa ggggtcaaag     900

ataagcgaaa cagagatgtg caagactttg gaacaacatg gtttacttat actaccagaa     960

gggccacaga gaatcagatt tgttattcat caccagattt cagaaaatga tgtgcactat    1020

gcagcgtcgt gcgttcagcg agctttggca ggagtggccg aagaaaatgg tgacaagtaa    1080


<210>  2

<211>  359
<212>  PRT
<213>  Nicotiana tabacum

<400>  2

Met Val Met Arg Thr Val Asp Leu Arg Ser Asp Thr Val Thr Lys Pro
1               5                   10                  15

Thr Glu Ala Met Arg Asn Ala Met Ala Asn Ala Glu Val Asp Asp Asp
            20                  25                  30

Val Leu Gly Tyr Asp Pro Thr Ala Gln Arg Leu Glu Ala Glu Ile Ala
        35                  40                  45

Arg Ile Met Gly Lys Glu Ala Gly Leu Phe Val Pro Ser Gly Thr Met
    50                  55                  60

Gly Asn Leu Ile Ser Val Leu Thr His Cys Gln Ile Arg Gly Ser Glu
65                  70                  75                  80

Ile Ile Leu Gly Asp Tyr Ser His Ile His Ile Tyr Glu Asn Gly Gly
                85                  90                  95

Ile Ala Thr Leu Gly Gly Val His Pro Arg Thr Val Lys Asn Asn Glu
            100                 105                 110

Asp Gly Thr Met Asp Leu Asp Leu Ile Glu Ala Ala Ile Arg Asp Pro
        115                 120                 125

Ser Phe Glu Ile Cys Tyr Pro Thr Thr Arg Leu Ile Cys Leu Glu Asn
    130                 135                 140

Ser His Ala His Ser Gly Gly Arg Cys Leu Ser Ala Glu Tyr Thr Asp
145                 150                 155                 160

Lys Val Gly Glu Leu Ala Lys Lys Tyr Gly Leu Lys Leu His Ile Asp
                165                 170                 175

Gly Ala Arg Ile Phe Asn Ala Ser Val Ala Leu Gly Val Pro Val His
            180                 185                 190

Arg Leu Val Arg Ala Ala Asp Ser Val Ser Val Cys Leu Ser Lys Gly
        195                 200                 205

Leu Gly Ala Pro Val Gly Ser Val Val Val Gly Ser Lys Ser Phe Ile
    210                 215                 220

Ala Lys Ala Lys Ile Leu Arg Lys Thr Leu Gly Gly Gly Met Arg Gln

225                 230                 235                 240

Val Gly Val Leu Cys Ala Ala Ala Phe Ile Ala Phe Gln Glu Asn Leu
                245                 250                 255

Val Lys Leu Glu Gly Asp His Lys Lys Ala Lys Ile Leu Ala Glu Glu
            260                 265                 270

Leu Asn Lys Ile Lys Gly Leu Lys Val Asp Ile Ala Ala Val Glu Thr
        275                 280                 285

Asn Ile Val Tyr Cys Asp Ile Leu Lys Gly Ser Lys Ile Ser Glu Thr
    290                 295                 300

Glu Met Cys Lys Thr Leu Glu Gln His Gly Leu Leu Ile Leu Pro Glu
305                 310                 315                 320

Gly Pro Gln Arg Ile Arg Phe Val Ile His His Gln Ile Ser Glu Asn
                325                 330                 335

Asp Val His Tyr Ala Ala Ser Cys Val Gln Arg Ala Leu Ala Gly Val
            340                 345                 350

Ala Glu Glu Asn Gly Asp Lys
        355

<210>  3
<211>  1080
<212>  DNA
<213>  Nicotiana tabacum

<400>  3
atggtgatga gaaccgtgga tcttcgctcg gacacagtca ctaaaccaac tgaagccatg      60

cggaacgcaa tggccaacgc tgaagtggat gacgatgtat tgggttatga tccaactgcc     120

caacgccttg aagccgagat cgcaaggata atgggcaaag aagcagggct atttgttcct     180

tcaggcacta tgggcaacct tatcagtgtg ctgactcatt gccaaattag aggcagtgaa     240

attattcttg gtgattattc acatatccat atttatgaaa atggaggcat tgccactctc     300

ggaggtgttc atccgaggac agtgaagaac aatgaagatg gaacaatgga tcttgattta     360

attgaagctg caattcgaga tcctagcttt gagatatgtt acccgaccac taggctgatc     420

tgcttggaga attcacatgc acactcagga ggcagatgcc tttctgcaga gtatacagac     480

aaagtcggag agctagcaaa gaagtatggt ctgaagcttc acattgatgg agctcgtata     540

ttcaatgcat cagttgcact tggagtgcct gttcatagac ttgtacgtgc tgctgattca     600

gtttcggtat gcttatcaaa aggtcttggt gctccagttg ggtctgtggt tgttggttca     660

aagagcttca ttgccaaggc aaaaattctg aggaagactc taggcggtgg aatgaggcag 720

gttggtgtcc tttgtgctgc tgcttttatc ggtttccaag agaatcttgt taagctggaa 780

ggagatcaca aaaaggctaa gattttggct gaggaactaa acaaaatcaa agggctgaaa 840

gttgatattg ctgccgtaga gactaacatt gtatattgcg atatactgaa ggggtcaaag 900

atcagcgaaa cagagatgtg caagattttg gagcaacatg gtttacttat actaccagaa 960

ggcctaatga gaatcagatt tgttattcat caccagattt cagaaaatga tgtgcactat 1020

gcggtatctt gtgttcagcg agctttagca ggagtggccg aagaaaatgg tgacaagtaa 1080

<210> 4
<211> 359
<212> PRT
<213> Nicotiana tabacum

<400> 4

Met Val Met Arg Thr Val Asp Leu Arg Ser Asp Thr Val Thr Lys Pro
1               5                   10                  15

Thr Glu Ala Met Arg Asn Ala Met Ala Asn Ala Glu Val Asp Asp Asp
            20                  25                  30

Val Leu Gly Tyr Asp Pro Thr Ala Gln Arg Leu Glu Ala Glu Ile Ala
        35                  40                  45

Arg Ile Met Gly Lys Glu Ala Gly Leu Phe Val Pro Ser Gly Thr Met
    50                  55                  60

Gly Asn Leu Ile Ser Val Leu Thr His Cys Gln Ile Arg Gly Ser Glu
65                  70                  75                  80

Ile Ile Leu Gly Asp Tyr Ser His Ile His Ile Tyr Glu Asn Gly Gly
                85                  90                  95

Ile Ala Thr Leu Gly Gly Val His Pro Arg Thr Val Lys Asn Asn Glu
            100                 105                 110

Asp Gly Thr Met Asp Leu Asp Leu Ile Glu Ala Ala Ile Arg Asp Pro
        115                 120                 125

Ser Phe Glu Ile Cys Tyr Pro Thr Thr Arg Leu Ile Cys Leu Glu Asn
    130                 135                 140

Ser His Ala His Ser Gly Gly Arg Cys Leu Ser Ala Glu Tyr Thr Asp
145                 150                 155                 160

Lys Val Gly Glu Leu Ala Lys Lys Tyr Gly Leu Lys Leu His Ile Asp

                165                 170                 175

Gly Ala Arg Ile Phe Asn Ala Ser Val Ala Leu Gly Val Pro Val His
            180                 185                 190

Arg Leu Val Arg Ala Ala Asp Ser Val Ser Val Cys Leu Ser Lys Gly
        195                 200                 205

Leu Gly Ala Pro Val Gly Ser Val Val Val Gly Ser Lys Ser Phe Ile
    210                 215                 220

Ala Lys Ala Lys Ile Leu Arg Lys Thr Leu Gly Gly Gly Met Arg Gln
225                 230                 235                 240

Val Gly Val Leu Cys Ala Ala Ala Phe Ile Gly Phe Gln Glu Asn Leu
                245                 250                 255

Val Lys Leu Glu Gly Asp His Lys Lys Ala Lys Ile Leu Ala Glu Glu
            260                 265                 270

Leu Asn Lys Ile Lys Gly Leu Lys Val Asp Ile Ala Ala Val Glu Thr
        275                 280                 285

Asn Ile Val Tyr Cys Asp Ile Leu Lys Gly Ser Lys Ile Ser Glu Thr
    290                 295                 300

Glu Met Cys Lys Ile Leu Glu Gln His Gly Leu Leu Ile Leu Pro Glu
305                 310                 315                 320

Gly Leu Met Arg Ile Arg Phe Val Ile His His Gln Ile Ser Glu Asn
                325                 330                 335

Asp Val His Tyr Ala Val Ser Cys Val Gln Arg Ala Leu Ala Gly Val
            340                 345                 350

Ala Glu Glu Asn Gly Asp Lys
        355

<210> 5
<211> 1119
<212> DNA
<213> Nicotiana tabacum

<400> 5
atggcggtaa gaactgtgga ttttcgatca gacacagtta ctaaaccaac tgaatccatg 60

cgtaacgcaa tggccaatgc agaagtagat gatgatgtct taggttatga tccaacggcc 120

caacgcctcg aagcagagat ggcaagaatc atgggcaaag aagcagggct atttgttcct 180

tcaggcacaa tgggcaatct tatttgcttg cttactcatt gccaaattag ggggagtgaa 240

gtaattcttg gtgataattc acatattcat gtatatgaaa atggagggat ttctacaatt 300

ggaggtgttc atcctaggac agtgaagaac aatgaagatg gaacaatgga tcttcatctg 360

attgaagctg caattcgaga ttctagtttt gaaatacttt atccaaccac taggcttatc 420

tgcttagaga attcacatgc acgatcggga ggtagatgcc ttactgtaga gtatacagac 480

aaagttggag agctagcaaa gaaatatggt ctgaagctgc atattgatgg cgctcgtata 540

ttcaatgcat cagttgcact tggtgtgccc gttcacaggc ttgtacaagc ggctgattca 600

gtttcggcat gcttatcgaa aggtcttggt gctccagcgg gatctgtgat tgttggttca 660

aagagcttca ttgccagggc aaaaattctg aggaagactc taggtggagg aatgcgacag 720

attggtgtac tctgtgctgc tgcttctgtc gctttacaag aaaatcttgt taagttggaa 780

ggagaccaca gaaaggctaa acttttagca gaggaactta acaaaatcaa agggcttaaa 840

gttgatagtg ctgctgtaga gaccaacatt gtatattgtc atatactgaa agggtcaaag 900

gtcagtagaa cacatttgtg taagattttg gaacaacatg gtatacttgt actaccagaa 960

gggccaatga gaatcaggtt tgttatacac caccagattt cagaaagtga tgtgcactat 1020

actgtctctt gccttcagca aactttggcg ggagaggcag aagaaaatgg tgatattaag 1080

catcttatga acggtttcaa acataatctt tatctgtaa 1119

<210> 6
<211> 372
<212> PRT
<213> Nicotiana tabacum

<400> 6

Met Ala Val Arg Thr Val Asp Phe Arg Ser Asp Thr Val Thr Lys Pro
1               5                   10                  15

Thr Glu Ser Met Arg Asn Ala Met Ala Asn Ala Glu Val Asp Asp Asp
            20                  25                  30

Val Leu Gly Tyr Asp Pro Thr Ala Gln Arg Leu Glu Ala Glu Met Ala
        35                  40                  45

Arg Ile Met Gly Lys Glu Ala Gly Leu Phe Val Pro Ser Gly Thr Met
    50                  55                  60

Gly Asn Leu Ile Cys Leu Leu Thr His Cys Gln Ile Arg Gly Ser Glu
65                  70                  75                  80

Val Ile Leu Gly Asp Asn Ser His Ile His Val Tyr Glu Asn Gly Gly
                85                  90                  95

Ile Ser Thr Ile Gly Gly Val His Pro Arg Thr Val Lys Asn Asn Glu
            100                 105                 110

Asp Gly Thr Met Asp Leu His Leu Ile Glu Ala Ala Ile Arg Asp Ser
        115                 120                 125

Ser Phe Glu Ile Leu Tyr Pro Thr Thr Arg Leu Ile Cys Leu Glu Asn
    130                 135                 140

Ser His Ala Arg Ser Gly Gly Arg Cys Leu Thr Val Glu Tyr Thr Asp
145                 150                 155                 160

Lys Val Gly Glu Leu Ala Lys Lys Tyr Gly Leu Lys Leu His Ile Asp
                165                 170                 175

Gly Ala Arg Ile Phe Asn Ala Ser Val Ala Leu Gly Val Pro Val His
            180                 185                 190

Arg Leu Val Gln Ala Ala Asp Ser Val Ser Ala Cys Leu Ser Lys Gly
        195                 200                 205

Leu Gly Ala Pro Ala Gly Ser Val Ile Val Gly Ser Lys Ser Phe Ile
    210                 215                 220

Ala Arg Ala Lys Ile Leu Arg Lys Thr Leu Gly Gly Gly Met Arg Gln
225                 230                 235                 240

Ile Gly Val Leu Cys Ala Ala Ala Ser Val Ala Leu Gln Glu Asn Leu
                245                 250                 255

Val Lys Leu Glu Gly Asp His Arg Lys Ala Lys Leu Leu Ala Glu Glu
            260                 265                 270

Leu Asn Lys Ile Lys Gly Leu Lys Val Asp Ser Ala Ala Val Glu Thr
        275                 280                 285

Asn Ile Val Tyr Cys His Ile Leu Lys Gly Ser Lys Val Ser Arg Thr
    290                 295                 300

His Leu Cys Lys Ile Leu Glu Gln His Gly Ile Leu Val Leu Pro Glu
305                 310                 315                 320

Gly Pro Met Arg Ile Arg Phe Val Ile His His Gln Ile Ser Glu Ser
                325                 330                 335

Asp Val His Tyr Thr Val Ser Cys Leu Gln Gln Thr Leu Ala Gly Glu
            340                 345                 350

Ala Glu Glu Asn Gly Asp Ile Lys His Leu Met Asn Gly Phe Lys His
        355                 360                 365

Asn Leu Tyr Leu
    370

<210>  7
<211>  26
<212>  DNA
<213>  Artificial sequence

<220>
<223>  Forward primer

<400>  7
ggggtcaaag atcagcgaaa cagaga                                          26

<210>  8
<211>  25
<212>  DNA
<213>  Artificial sequence

<220>
<223>  Reverse primer

<400>  8
tcttcggcca ctcctgctaa agctc                                           25

<210>  9
<211>  25
<212>  DNA
<213>  Artificial sequence

<220>
<223>  Forward primer

<400>  9
tactgaaagg gtcaaaggtc agtag                                           25

<210>  10
<211>  26
<212>  DNA
<213>  Artificial sequence

<220>
<223>  Reverse primer

<400>  10
ttcttctgcc tctcccgcca aagttt                                          26

<210>  11
<211>  23
<212>  DNA
<213>  Artificial sequence

<220>
<223>  Forward primer

<400>  11
cagggaaaat ggtgatgaga acc                                              23


<210>  12
<211>  23
<212>  DNA
<213>  Artificial sequence

<220>
<223>  Reverse primer

<400>  12
atgcaccaat atgaggatcg ctg                                              23


<210>  13
<211>  23
<212>  DNA
<213>  Artificial sequence

<220>
<223>  Forward primer

<400>  13
cagggaaaat ggtgatgaga acc                                              23


<210>  14
<211>  25
<212>  DNA
<213>  Artificial sequence

<220>
<223>  Reverse primer

<400>  14
accagataat gtaccaaata gagag                                            25


<210>  15
<211>  23
<212>  DNA
<213>  Artificial sequence

<220>
<223>  tha1_s1_F primer

<400>  15
gcactatggg caaccttatc agt                                              23


<210>  16
<211>  21
<212>  DNA
<213>  Artificial sequence

<220>
<223>  tha1_s1_R primer

<400>  16
aacacctccg agagtggcaa t                                                21


<210>  17
<211>  16
<212>  DNA
<213>  Artificial sequence

<220>
<223>  tha1_s1_W probe

<400>  17
tcattgccaa attaga                                                      16


<210>  18
<211>  17
<212>  DNA
<213>  Artificial sequence

<220>
<223>  tha1_s1_M probe

<400>  18
tgctgactca ttgctaa                                                     17


<210>  19
<211>  24
<212>  DNA
<213>  Artificial sequence

<220>
<223>  tha1_s2_F primer

<400>  19
cgaggacagt gaagaacaat gaag                                             24


<210>  20
<211>  22
<212>  DNA
<213>  Artificial sequence

<220>
<223>  tha1_s2_R primer

<400>  20
tcagcctagt ggttgggtaa ca                                               22


<210>  21
<211>  16
<212>  DNA
<213>  Artificial sequence

<220>
<223>  tha1_s2_W probe

<400>  21
aagctgcaat tcgaga                                                      16

<210>  22
<211>  14
<212>  DNA
<213>  Artificial sequence

<220>
<223>  tha1_s2_M probe

<400>  22
aagctgcaat ttga                                                          14


<210>  23
<211>  19
<212>  DNA
<213>  Artificial sequence

<220>
<223>  tha1_t_F primer

<400>  23
caatggccaa cgctgaagt                                                     19


<210>  24
<211>  21
<212>  DNA
<213>  Artificial sequence

<220>
<223>  tha1_t_R primer

<400>  24
ggttgcccat agtgcctgaa g                                                  21


<210>  25
<211>  19
<212>  DNA
<213>  Artificial sequence

<220>
<223>  tha1_t_W probe

<400>  25
ccaactgccc aacgccttg                                                     19


<210>  26
<211>  19
<212>  DNA
<213>  Artificial sequence

<220>
<223>  tha1_t_M probe

<400>  26
ccaactgcct aacgccttg                                                     19


<210>  27

<211>  23
<212>  DNA
<213>  Artificial sequence

<220>
<223>  THA1_F primer

<400>  27
taggtcaggg aaaatggtga tga                                               23


<210>  28
<211>  18
<212>  DNA
<213>  Artificial sequence

<220>
<223>  THA1_R primer

<400>  28
cgttccgcat ggcttcag                                                     18


<210>  29
<211>  24
<212>  DNA
<213>  Artificial sequence

<220>
<223>  THA1 probe

<400>  29
accgtggatc ttcgctcgga caca                                              24


<210>  30
<211>  20
<212>  DNA
<213>  Artificial sequence

<220>
<223>  EF-1_F primer

<400>  30
ctaagggtgc tgccagcttt                                                   20


<210>  31
<211>  22
<212>  DNA
<213>  Artificial sequence

<220>
<223>  EF-1_R primer

<400>  31
gtcaagcact ggagcatatc ca                                                22


<210>  32
<211>  26
<212>  DNA
<213>  Artificial sequence

<220>
<223>  EF-1 Probe

<400>  32
atcatgaacc atccaggaca gattgg                                          26

<210>  33
<211>  1080
<212>  DNA
<213>  Nicotiana tabacum

<400>  33
atggtgatga gaaccgtgga tcttcgctcg gacacagtca ctaaaccaac tgaagccatg      60

cggaacgcga tggccaatgc tgaagtggat gacgatgtat tgggttatga tccaactgcc     120

caacgccttg aagccgagat cgcaaggata atgggcaaag aagcagggct atttgttcct     180

tcaggcacta tgggcaacct tatcagtgtg ctgactcatt gctaaattag aggcagtgaa     240

attattcttg gtgattattc gcatatccat atttatgaaa atggaggcat tgccactctc     300

ggaggtgttc atccgaggac agtgaagaac aatgaagatg gaacaatgga tcttgatcta     360

attgaagctg caattcgaga tcctagcttt gagatatgtt acccaaccac taggctgatc     420

tgcttggaga attcacatgc acactcagga ggccgatgcc tttctgcaga gtatacagac     480

aaagtcggag agctagcaaa gaagtatggt ctgaagcttc acattgatgg agctcgtata     540

ttcaatgcat cagttgcact cggagtgcct gttcatagac ttgtacgggc tgctgattca     600

gtttcggtat gcttatcaaa aggtcttggt gctccagtcg ggtctgtggt tgttggttca     660

aagagcttca ttgccaaggc gaaaattctc aggaagactc taggcggtgg aatgaggcag     720

gttggtgtcc tttgtgctgc tgcttttatc gctttccaag agaatcttgt taagctggaa     780

ggagatcaca aaaaggctaa gattttggct gaggaactta acaaaatcaa agggctgaaa     840

gttgatattg ctgccgtaga gactaacatt gtatattgtg atatactgaa ggggtcaaag     900

ataagcgaaa cagagatgtg caagactttg gaacaacatg gtttacttat actaccagaa     960

gggccacaga gaatcagatt tgttattcat caccagattt cagaaaatga tgtgcactat    1020

gcagcgtcgt gcgttcagcg agctttggca ggagtggccg aagaaaatgg tgacaagtaa    1080

<210>  34
<211>  1080
<212>  DNA
<213>  Nicotiana tabacum

<400>  34
atggtgatga gaaccgtgga tcttcgctcg gacacagtca ctaaaccaac tgaagccatg      60

cggaacgcga tggccaatgc tgaagtggat gacgatgtat tgggttatga tccaactgcc     120

caacgccttg aagccgagat cgcaaggata atgggcaaag aagcagggct atttgttcct     180

tcaggcacta tgggcaacct tatcagtgtg ctgactcatt gccaaattag aggcagtgaa     240

attattcttg gtgattattc gcatatccat atttatgaaa atggaggcat tgccactctc 300

ggaggtgttc atccgaggac agtgaagaac aatgaagatg gaacaatgga tcttgatcta 360

attgaagctg caatttgaga tcctagcttt gagatatgtt acccaaccac taggctgatc 420

tgcttggaga attcacatgc acactcagga ggccgatgcc tttctgcaga gtatacagac 480

aaagtcggag agctagcaaa gaagtatggt ctgaagcttc acattgatgg agctcgtata 540

ttcaatgcat cagttgcact cggagtgcct gttcatagac ttgtacgggc tgctgattca 600

gtttcggtat gcttatcaaa aggtcttggt gctccagtcg ggtctgtggt tgttggttca 660

aagagcttca ttgccaaggc gaaaattctc aggaagactc taggcggtgg aatgaggcag 720

gttggtgtcc tttgtgctgc tgcttttatc gctttccaag agaatcttgt taagctggaa 780

ggagatcaca aaaaggctaa gattttggct gaggaactta acaaaatcaa agggctgaaa 840

gttgatattg ctgccgtaga gactaacatt gtatattgtg atatactgaa ggggtcaaag 900

ataagcgaaa cagagatgtg caagactttg gaacaacatg gtttacttat actaccagaa 960

gggccacaga gaatcagatt tgttattcat caccagattt cagaaaatga tgtgcactat 1020

gcagcgtcgt gcgttcagcg agctttggca ggagtggccg aagaaaatgg tgacaagtaa 1080

<210> 35
<211> 1080
<212> DNA
<213> Nicotiana tabacum

<400> 35
atggtgatga gaaccgtgga tcttcgctcg gacacagtca ctaaaccaac tgaagccatg 60

cggaacgcaa tggccaacgc tgaagtggat gacgatgtat tgggttatga tccaactgcc 120

taacgccttg aagccgagat cgcaaggata atgggcaaag aagcagggct atttgttcct 180

tcaggcacta tgggcaacct tatcagtgtg ctgactcatt gccaaattag aggcagtgaa 240

attattcttg gtgattattc acatatccat atttatgaaa atggaggcat tgccactctc 300

ggaggtgttc atccgaggac agtgaagaac aatgaagatg gaacaatgga tcttgattta 360

attgaagctg caattcgaga tcctagcttt gagatatgtt acccgaccac taggctgatc 420

tgcttggaga attcacatgc acactcagga ggcagatgcc tttctgcaga gtatacagac 480

aaagtcggag agctagcaaa gaagtatggt ctgaagcttc acattgatgg agctcgtata 540

ttcaatgcat cagttgcact tggagtgcct gttcatagac ttgtacgtgc tgctgattca 600

gtttcggtat gcttatcaaa aggtcttggt gctccagttg ggtctgtggt tgttggttca 660

aagagcttca ttgccaaggc aaaaattctg aggaagactc taggcggtgg aatgaggcag 720

gttggtgtcc tttgtgctgc tgcttttatc ggtttccaag agaatcttgt taagctggaa 780

ggagatcaca aaaaggctaa gattttggct gaggaactaa acaaaatcaa agggctgaaa 840

gttgatattg ctgccgtaga gactaacatt gtatattgcg atatactgaa ggggtcaaag 900

atcagcgaaa cagagatgtg caagattttg gagcaacatg gtttacttat actaccagaa 960

ggcctaatga gaatcagatt tgttattcat caccagattt cagaaaatga tgtgcactat 1020

gcggtatctt gtgttcagcg agctttagca ggagtggccg aagaaaatgg tgacaagtaa 1080

<210> 36
<211> 552
<212> PRT
<213> Nicotiana tabacum

<400> 36

Met Ala Ser Leu Ser His Phe Cys Gly Val Lys Thr Pro Cys Ser Ser
1               5                   10                  15

Leu Phe Lys Arg Ser Val His Phe Arg Gln Leu Asp Phe Val Ala Ser
            20                  25                  30

Val Pro Phe Ala Ser Ala Pro Ser Lys Tyr Val Lys Ala Ser Cys Cys
        35                  40                  45

Glu Arg Val Thr Cys Lys Ala Gln Ala Ala Asp Val Val Glu Leu Lys
    50                  55                  60

Glu Thr Lys Asn Glu Ala Phe Gly Glu Phe Thr Cys Val Met Lys Phe
65                  70                  75                  80

Gly Gly Ser Ser Val Ala Ser Ala Glu Arg Met Arg Glu Val Ala Asp
                85                  90                  95

Leu Ile Leu Ser Phe Pro Glu Glu Arg Pro Val Ile Val Leu Ser Ala
            100                 105                 110

Met Gly Lys Thr Thr Asn Asn Leu Leu Leu Ala Gly Glu Lys Ala Val
        115                 120                 125

Thr Cys Gly Val Ser Asn Val Cys Asp Leu Gln Glu Leu Asp Phe Ile
    130                 135                 140

Lys Asp Leu His Phe Arg Thr Ile Asp Gln Leu Gly Val Glu Lys Ser
145                 150                 155                 160

Thr Ile Ser Lys His Leu Leu Glu Leu Glu Gln Leu Leu Asn Gly Ile
                165                 170                 175

Ala Met Met Lys Glu Leu Thr Pro Arg Thr Arg Asp Tyr Leu Val Ser
            180                 185                 190

Phe Gly Glu Cys Met Ser Thr Arg Ile Phe Thr Ala Tyr Leu Asn Lys
        195                 200                 205

Leu Gly Val Lys Ala Arg Gln Tyr Asp Ala Phe Glu Met Gly Ile Ile
    210                 215                 220

Thr Thr Asp Glu Phe Thr Asn Ala Asp Ile Leu Glu Ala Thr Tyr Pro
225                 230                 235                 240

Ala Val Ala Lys Arg Leu Ser Gly Asp Trp Met Asn Asn Pro Ala Ile
                245                 250                 255

Pro Ile Val Thr Gly Phe Leu Gly Lys Gly Trp Arg Thr Cys Ala Val
            260                 265                 270

Thr Thr Leu Gly Arg Gly Gly Ser Asp Leu Thr Ala Thr Thr Ile Gly
        275                 280                 285

Lys Ala Leu Gly Leu Arg Glu Ile Gln Val Trp Lys Asp Val Asp Gly
    290                 295                 300

Val Leu Thr Cys Asp Pro Asn Ile Tyr Pro Arg Ala Gln Pro Val Pro
305                 310                 315                 320

Tyr Leu Thr Phe Asp Glu Ala Ala Glu Leu Ala Tyr Phe Gly Ala Gln
                325                 330                 335

Val Leu His Pro Gln Ser Met Arg Pro Ala Arg Glu Gly Asp Ile Pro
            340                 345                 350

Val Arg Val Lys Asn Ser Tyr Asn Pro Lys Ala Pro Gly Thr Leu Ile
        355                 360                 365

Cys Arg Ala Arg Asp Met Ser Lys Ala Val Leu Thr Ser Ile Val Leu
    370                 375                 380

Lys Arg Asn Val Thr Met Leu Asp Ile Val Ser Thr Arg Met Leu Gly
385                 390                 395                 400

Gln Phe Gly Phe Phe Ala Lys Val Phe Ser Ile Phe Glu Asp Leu Gly
                405                 410                 415

Ile Ser Val Asp Val Val Ala Thr Ser Glu Val Ser Ile Ser Leu Thr
            420                 425                 430

Leu Asp Pro Ser Lys Leu Trp Ser Arg Glu Leu Ile Gln Gln Glu Leu

435                 440                 445

Asp His Val Val Glu Glu Leu Glu Lys Ile Ala Val Val Asn Leu Leu
    450                 455                 460

Gln His Arg Ser Ile Ile Ser Leu Ile Gly Asn Val Gln Arg Ser Ser
465                 470                 475                 480

Leu Ile Leu Glu Lys Ala Phe His Val Leu Arg Thr Asn Gly Val Asn
                485                 490                 495

Val Gln Met Ile Ser Gln Gly Ala Ser Lys Val Asn Ile Ser Leu Ile
            500                 505                 510

Val Asn Asp Ser Glu Ala Glu Gln Cys Val Arg Ala Leu His Lys Thr
        515                 520                 525

Phe Phe Glu Ser Asp Leu Ser Glu Leu Val Trp Glu Asn Gly His Val
    530                 535                 540

Thr Pro Leu Ser Thr Leu Ser Thr
545                 550

<210>  37
<211>  18
<212>  DNA
<213>  Artificial sequence

<220>
<223>  Forward primer

<400>  37
actaccagaa gggccaca                                                    18

<210>  38
<211>  17
<212>  DNA
<213>  Artificial sequence

<220>
<223>  Reverse primer

<400>  38
ccaaagctcg ctgaacg                                                     17

<210>  39
<211>  21
<212>  DNA
<213>  Artificial sequence

<220>
<223>  Forward primer

<400>  39
ctaccagaag gcctaatgag a                                                21


<210>  40
<211>  20
<212>  DNA
<213>  Artificial sequence

<220>
<223>  Reverse primer

<400>  40
ctgctaaagc tcgctgaaca                                                  20


<210>  41
<211>  28
<212>  DNA
<213>  Artificial sequence

<220>
<223>  FAM/TAMRA probe

<400>  41
tcaccagatt tcagaaaatg atgtgcac                                         28


<210>  42
<211>  23
<212>  DNA
<213>  Artificial sequence

<220>
<223>  Target region for CRISPR-Cas9

<400>  42
ggaggcattg ccactctcgg agg                                              23


<210>  43
<211>  23
<212>  DNA
<213>  Artificial sequence

<220>
<223>  Forward primer

<400>  43
taggtcaggg aaaatggtga tga                                              23


<210>  44
<211>  18
<212>  DNA
<213>  Artificial sequence

<220>
<223>  Reverse primer

<400>  44
cgttccgcat ggcttcag                                                    18

<210>  45
<211>  22
<212>  DNA
<213>  Artificial sequence

<220>
<223>  Forward primer

<400>  45
ttggttcaaa gagcttcatt gc                                                22


<210>  46
<211>  21
<212>  DNA
<213>  Artificial sequence

<220>
<223>  Reverse primer

<400>  46
ccgataaaag cagcagcaca a                                                 21


<210>  47
<211>  24
<212>  DNA
<213>  Artificial sequence

<220>
<223>  FAM/TAMRA probe

<400>  47
accgtggatc ttcgctcgga caca                                              24


<210>  48
<211>  18
<212>  DNA
<213>  Artificial sequence

<220>
<223>  FAM/NFQ (MGB) probe

<400>  48
aagactctag gcggtgga                                                     18


<210>  49
<211>  1081
<212>  DNA
<213>  Nicotiana tabacum

<400>  49
atggtgatga gaaccgtgga tcttcgctcg gacacagtca ctaaaccaac tgaagccatg      60

cggaacgcga tggccaatgc tgaagtggat gacgatgtat tgggttatga tccaactgcc     120

caacgccttg aagccgagat cgcaaggata atgggcaaag aagcagggct atttgttcct     180

tcaggcacta tgggcaacct tatcagtgtg ctgactcatt gccaaattag aggcagtgaa     240

attattcttg gtgattattc gcatatccat atttatgaaa tatggaggca ttgccactct 300

cggaggtgtt catccgagga cagtgaagaa caatgaagat ggaacaatgg atcttgatct 360

aattgaagct gcaattcgag atcctagctt tgagatatgt tacccaacca ctaggctgat 420

ctgcttggag aattcacatg cacactcagg aggccgatgc ctttctgcag agtatacaga 480

caaagtcgga gagctagcaa agaagtatgg tctgaagctt cacattgatg gagctcgtat 540

attcaatgca tcagttgcac tcggagtgcc tgttcataga cttgtacggg ctgctgattc 600

agtttcggta tgcttatcaa aaggtcttgg tgctccagtc gggtctgtgg ttgttggttc 660

aaagagcttc attgccaagg cgaaaattct caggaagact ctaggcggtg gaatgaggca 720

ggttggtgtc ctttgtgctg ctgcttttat cgctttccaa gagaatcttg ttaagctgga 780

aggagatcac aaaaaggcta agattttggc tgaggaactt aacaaaatca aaggcctgaa 840

agttgatatt gctgccgtag agactaacat tgtatattgt gatatactga aggggtcaaa 900

gataagcgaa acagagatgt gcaagacttt ggaacaacat ggtttactta tactaccaga 960

agggccacag agaatcagat ttgttattca tcaccagatt tcagaaaatg atgtgcacta 1020

tgcagcgtcg tgcgttcagc gagctttggc aggagtggcc gaagaaaatg gtgacaagta 1080

a 1081

<210> 50
<211> 1081
<212> DNA
<213> Nicotiana tabacum

<400> 50
atggtgatga gaaccgtgga tcttcgctcg gacacagtca ctaaaccaac tgaagccatg 60

cggaacgcaa tggccaacgc tgaagtggat gacgatgtat tgggttatga tccaactgcc 120

caacgccttg aagccgagat cgcaaggata atgggcaaag aagcagggct atttgttcct 180

tcaggcacta tgggcaacct tatcagtgtg ctgactcatt gccaaattag aggcagtgaa 240

attattcttg gtgattattc acatatccat atttatgaaa tatggaggca ttgccactct 300

cggaggtgtt catccgagga cagtgaagaa caatgaagat ggaacaatgg atcttgattt 360

aattgaagct gcaattcgag atcctagctt tgagatatgt tacccgacca ctaggctgat 420

ctgcttggag aattcacatg cacactcagg aggcagatgc ctttctgcag agtatacaga 480

caaagtcgga gagctagcaa agaagtatgg tctgaagctt cacattgatg gagctcgtat 540

attcaatgca tcagttgcac ttggagtgcc tgttcataga cttgtacgtg ctgctgattc 600

agtttcggta tgcttatcaa aaggtcttgg tgctccagtt gggtctgtgg ttgttggttc 660

aaagagcttc attgccaagg caaaaattct gaggaagact ctaggcggtg gaatgaggca 720

ggttggtgtc ctttgtgctg ctgcttttat cggtttccaa gagaatcttg ttaagctgga 780

aggagatcac aaaaaggcta agattttggc tgaggaacta aacaaaatca aagggctgaa 840

agttgatatt gctgccgtag agactaacat tgtatattgc gatatactga aggggtcaaa 900

gatcagcgaa acagagatgt gcaagatttt ggagcaacat ggtttactta tactaccaga 960

aggcctaatg agaatcagat ttgttattca tcaccagatt tcagaaaatg atgtgcacta 1020

tgcggtatct tgtgttcagc gagctttagc aggagtggcc gaagaaaatg gtgacaagta 1080

a 1081

<210> 51
<211> 1081
<212> DNA
<213> Nicotiana tabacum

<400> 51
atggtgatga gaaccgtgga tcttcgctcg gacacagtca ctaaaccaac tgaagccatg 60

cggaacgcaa tggccaacgc tgaagtggat gacgatgtat tgggttatga tccaactgcc 120

caacgccttg aagccgagat cgcaaggata atgggcaaag aagcagggct atttgttcct 180

tcaggcacta tgggcaacct tatcagtgtg ctgactcatt gccaaattag aggcagtgaa 240

attattcttg gtgattattc acatatccat atttatgaaa gatggaggca ttgccactct 300

cggaggtgtt catccgagga cagtgaagaa caatgaagat ggaacaatgg atcttgattt 360

aattgaagct gcaattcgag atcctagctt tgagatatgt tacccgacca ctaggctgat 420

ctgcttggag aattcacatg cacactcagg aggcagatgc ctttctgcag agtatacaga 480

caaagtcgga gagctagcaa agaagtatgg tctgaagctt cacattgatg gagctcgtat 540

attcaatgca tcagttgcac ttggagtgcc tgttcataga cttgtacgtg ctgctgattc 600

agtttcggta tgcttatcaa aaggtcttgg tgctccagtt gggtctgtgg ttgttggttc 660

aaagagcttc attgccaagg caaaaattct gaggaagact ctaggcggtg gaatgaggca 720

ggttggtgtc ctttgtgctg ctgcttttat cggtttccaa gagaatcttg ttaagctgga 780

aggagatcac aaaaaggcta agattttggc tgaggaacta aacaaaatca aagggctgaa 840

agttgatatt gctgccgtag agactaacat tgtatattgc gatatactga aggggtcaaa 900

gatcagcgaa acagagatgt gcaagatttt ggagcaacat ggtttactta tactaccaga 960

aggcctaatg agaatcagat ttgttattca tcaccagatt tcagaaaatg atgtgcacta 1020

tgcggtatct tgtgttcagc gagctttagc aggagtggcc gaagaaaatg gtgacaagta 1080

a 1081

## Description

### Technical Field

The present invention relates to a method of producing a cured tobacco material having an enhanced threonine content, a cured tobacco material, an extract of the cured tobacco material, and a tobacco product.

### Background Art

Threonine is an amino acid that provides various beneficial effects in plants. For example, threonine in a tobacco plant is one of the components that contribute greatly to the flavor (aroma and taste) of smoking when the tobacco plant processed into a tobacco source material is smoked. Developing a tobacco variety for production of a tobacco source material having an enhanced threonine content has been an important goal in tobacco breeding.

One known approach to enhance the threonine content of a plant is to modify aspartate kinase (AK), which is an enzyme (key enzyme) serving as a key to the threonine biosynthesis. AK is the first enzyme in the biosynthetic pathway of various amino acids derived from aspartic acid, such as threonine. Usually, the activity of AK is strictly regulated by feedback inhibition from the end product such as threonine, lysine, or S-adenosylmethionine (Non-patent Literatures 1 and 2).

It is known that plants' leaves that express AK which has been modified to be protected against such feedback inhibition from the amino acid (end product) show a high level of threonine concentration (Non-patent Literature 3 and Patent Literature 1).

It is also known that, also when there is an obstacle in the pathway of biosynthesis of lysine or methionine from aspartic acid, the threonine content of a plant becomes high. For example, a T-DNA insertion mutant of *Arabidopsis thaliana,* in which the function of dihydrodipicolinate synthase (DHDPS) (which is an enzyme responsible for the first reaction in the lysine biosynthetic pathway) is impaired, shows a high level of threonine concentration in the whole body (Non-patent Literature 4). Also, a shoot of *Arabidopsis thaliana* in which the expression of cystathionine gamma-synthase (CGS) (which is a key enzyme in the methionine biosynthetic pathway) is inhibited by antisense RNA has a high threonine content (Non-patent Literature 5).

However, a plant modified to have an enhanced threonine content is known to be accompanied by severe growth inhibition, and this is not only true of the above examples (Non-patent literatures 6, 7, and 8).

As such, a plant having an enhanced threonine content is accompanied by an unacceptable characteristic that would lead to a significant reduction in yield. This has been a major issue in developing a tobacco variety for use in production of, for example, a tobacco source material with high level of threonine content.

Patent Literature 2 discloses one method to solve the above issue, by which AK protected against feedback inhibition is expressed under the control of a senescence-induced promoter. According to this method, the threonine content does not increase before senescence, and thus this method is promising in improving the poor plant growth.

On the other hand, unlike the aforementioned examples, it has not been reported that a modification to inhibit threonine degradation has resulted in increasing the threonine content of a plant's leaf. For example, *Arabidopsis thaliana* contains two threonine aldolases (THA) each responsible for catabolism of threonine to glycine. Of these, a loss of function of THAI that is expressed mainly in seeds and seedlings enhances the threonine content of seeds; however, the loss does not influence the threonine content of shoots. On the other hand, a mutation that impairs the function of THA2, which is expressed in vascular bundle tissues in the whole body, causes a lethal albino phenotype (Patent Literature 3 and Non-patent Literatures 9 and 10). Furthermore, suppressed expression of threonine deaminase (TD), which is a key enzyme in another threonine degradation pathway, strongly inhibits the growth of a plant (Non-patent Literature 11).

As described above, it is known that the approaches to enhance the threonine level by inhibiting threonine degradation are difficult to employ in the development of a practical tobacco variety.

### Citation List

### [Patent Literatures]

[Patent Literature 1]
   Specification of United States Patent Application No. 6730832 (Publication Date: May 4, 2004)
[Patent Literature 2]
   Specification of PCT International Publication No. WO2010/018234 (Publication Date: February 18, 2010)
[Patent Literature 3]
   Specification of PCT International Publication No. WO2005/047472 (Publication Date: May 26, 2006)

[Non-patent Literature 1]
   Heldt and Piechulla, Plant biochemistry, Fourth Edition, Academic Press, 291-293 (2010)
[Non-patent Literature 2]
   Jander and Joshi, The Arabidopsis book/American Society of Plant Biologists, 7 (2009)
[Non-patent Literature 3]
   Frankard et al., Theoretical and Applied Genetics, 82, 273-282 (1991)
[Non-patent Literature 4]
   Craciun et al., FEBS letters, 487, 234-238 (2000)
[Non-patent Literature 5]
   Kim et al. Plant Science, 151(1), 9-18 (2000)
[Non-patent Literature 6]
   Shaul and Galili, Plant Physiol 100: 1157-1163 (1992)
[Non-patent Literature 7]
   Karchi et al., The Plant Journal, 3, 721-727 (1993)
[Non-patent Literature 8]
   Sarrobert et al., The Plant Journal, 24, 357-368 (2000)
[Non-patent Literature 9]
   Jander et al., The Plant Journal, 39, 465-475 (2004)
[Non-patent Literature 10]
   Joshi et al., Plant Cell. 18, 3564-3575 (2006)
[Non-patent Literature 11]
   Kang et al., The Plant Cell, 18(11), 3303-3320 (2006)

### Summary of Invention

### Technical Problem

There is still a demand for research or development of a tobacco variety that is suitable for production of a tobacco source material with high level of threonine content and that is not accompanied by growth inhibition. It is also demanded that a tobacco variety having such features can be selected from non-genetically-modified varieties.

The present invention was made in view of the above issues, and an object thereof is to provide a method of producing a cured tobacco material having an enhanced threonine content, without producing any drawbacks in a leaf tobacco production that involves cultivating a tobacco plant, harvesting the tobacco plant, and then curing the tobacco plant.

### Solution to Problem

In order to attain the above object, the present invention includes any one of the following aspects.
<1> A method of producing a cured tobacco material having an enhanced threonine content,
   including producing the cured tobacco material from (i) a modified tobacco plant which has been modified such that expression or activity of an endogenous threonine aldolase is reduced or (ii) a part of the modified tobacco plant,
   said endogenous threonine aldolase being at least one of the following threonine aldolases (a) and (b):
   (a) a threonine aldolase comprised of a polypeptide encoded by a gene containing a polynucleotide which has a sequence identity of not less than 90% to a polynucleotide which has a base sequence shown in SEQ ID NO:1; and
   (b) a threonine aldolase comprised of a polypeptide encoded by a gene containing a polynucleotide which has a sequence identity of not less than 90% to a polynucleotide which has a base sequence shown in SEQ ID NO:3.
<2> A method of producing a cured tobacco material having an enhanced threonine content,
   including producing the cured tobacco material from (i) a modified tobacco plant which has been modified such that expression or activity of an endogenous threonine aldolase is reduced or (ii) a part of the modified tobacco plant,
   said endogenous threonine aldolase being at least one of the following threonine aldolases (a) and (b):
   (a) a threonine aldolase comprised of a polypeptide which has a sequence identity of not less than 90% to a polypeptide which has an amino acid sequence shown in SEQ ID NO:2; and
   (b) a threonine aldolase comprised of a polypeptide which has a sequence identity of not less than 90% to a polypeptide which has an amino acid sequence shown in SEQ ID NO:4.

### Advantageous Effects of Invention

According to the present invention, it is possible to produce a cured tobacco material having an enhanced threonine content, and possible to provide the cured tobacco material, an extract prepared from the cured tobacco material, and a tobacco product produced using the cured tobacco material and/or the extract.

### Brief Description of Drawings

Fig. 1 shows metabolism pathways of amino acids derived from aspartic acid.
Fig. 2 shows the expressions of NtTHA1 and NtTHA2 genes from various tissues of *Nicotiana tabacum.*
Fig. 3 shows base sequence alignment of wild type alleles and mutant alleles of NtTHA1 genes.
Fig. 4 shows amino acid sequence alignment of translation products of wild type alleles and mutant alleles of NtTHA1 genes.
Fig. 5 shows the expression of NtTHA1 genes in NtTHA1-gene double mutants.
Fig. 6 shows NtTHA1-gene double mutants and control tobacco plants grown in a field.
Fig. 7 shows a standard curing schedule for flue curing.
Fig. 8 shows the threonine content of fresh leaves taken from NtTHA1-gene double mutants and a control tobacco plant.
Fig. 9 shows the threonine content of flue-cured leaves of the NtTHA1-gene double mutants and the control tobacco plant.
Fig. 10 shows threonine content in cases where leaves taken from the NtTHA1-gene double mutant and the control tobacco plant were cured (flue-curing) at each temperature.
Fig. 11 shows the expression of the NtTHA1 genes in individuals of respective genotypes.
Fig. 12 shows the free threonine content of cured leaves derived from the individuals of the respective genotypes.
Fig. 13 shows tobacco leaves which are being air-cured.
Fig. 14 shows transitions of ambient temperature and humidity around tobacco leaves during air-curing.
Fig. 15 shows transitions of free threonine content of tobacco leaves of a double mutant and a control during air-curing.
Fig. 16 shows the free threonine content of cured leaves of the double mutant and the control.
Fig. 17 shows expression level of the NtTHA1 genes in genome-edited tobacco plants.
Fig. 18 shows the free threonine content of cured leaves of genome-edited tobacco plants.

### Description of Embodiments

The following description will discuss embodiments of the present invention. Note, however, that the following description is not intended to limit the present invention.

### [Definitions of terms and the like]

As used herein, the term "polynucleotide" can be interpreted as meaning "nucleic acid" or "nucleic acid molecule", and is intended to mean a polymer of nucleotides. The term "base sequence" can be interpreted as meaning "nucleic acid sequence" or "nucleotide sequence", and is intended to mean the sequence of deoxyribonucleic acid or ribonucleic acid, unless otherwise noted.

As used herein, the term "polypeptide" can be interpreted as meaning "protein".

As used herein, the term "homozygote" refers to a tobacco plant in which a certain gene locus that codes for a threonine aldolase is a homozygous locus occupied by a pair of identical alleles. The term "heterozygote" refers to a tobacco plant in which a certain gene locus that codes for a threonine aldolase is a heterozygous locus occupied by a pair of different alleles.

In this specification, the phrase "A and/or B" is intended to encompass both "A and B" and "A or B", and can be interpreted as meaning "at least one of A and B".

As used herein, the phrase "polynucleotides are complementary to each other" can be interpreted as meaning, when the base sequences of the polynucleotides are compared to each other, "the base sequences of the polynucleotides are complementary to each other".

As used herein, the term "control", in regard to a tobacco plant, refers to a tobacco plant in which the expression or activity of an endogenous threonine aldolase in accordance with the present invention has not been changed from normal state, for example, a tobacco plant which has not been modified such that the expression or activity of an endogenous threonine aldolase is reduced. Thus, a control tobacco plant is capable of providing a standard for comparison with a tobacco plant with reduced expression or activity of the endogenous threonine aldolase. A control can be a wild type tobacco plant of the same species. Alternatively, the control may be a tobacco plant containing an empty version of a recombinant expression vector (this will be described later).

As used herein, the term "control", in regard to a threonine aldolase protein, refers to a threonine aldolase protein in which the activity of a threonine aldolase in accordance with the present invention has not been changed from normal state, for example, a threonine aldolase protein which has not been modified such that the activity of a threonine aldolase is reduced. Thus, a control threonine aldolase protein is capable of providing a standard for comparison with a threonine aldolase protein with reduced threonine aldolase activity.

As used herein, the term "control", in regard to a cured tobacco material, refers to a cured tobacco material obtained by curing a tobacco plant in which the expression or activity of an endogenous threonine aldolase in accordance with the present invention has not been changed from normal state, for example, obtained by curing a tobacco plant which has not been modified such that the expression or activity of an endogenous threonine aldolase is reduced or by curing a part of the tobacco plant. Thus, a control cured tobacco material is capable of providing a standard for comparison with a cured tobacco material prepared by curing a tobacco plant with reduced expression or activity of an endogenous threonine aldolase or by curing a part of the tobacco plant.

### [1. Method of producing cured tobacco material having enhanced threonine content]

The following description will discuss a production method in accordance with the present invention.

A production method in accordance with the present invention is a method of producing a cured tobacco material having an enhanced threonine content, the method including producing the cured tobacco material from (i) a tobacco plant which has been modified such that the expression or activity of an endogenous threonine aldolase is reduced or (ii) a part of the tobacco plant, wherein the endogenous threonine aldolase is at least one of the following threonine aldolases (a) and (b).
(a) A threonine aldolase comprised of a polypeptide encoded by a gene containing a polynucleotide which has a sequence identity of not less than 90% to a polynucleotide which has the base sequence shown in SEQ ID NO:1
(b) A threonine aldolase comprised of a polypeptide encoded by a gene containing a polynucleotide which has a sequence identity of not less than 90% to a polynucleotide which has the base sequence shown in SEQ ID NO:3

Alternatively, the production method in accordance with the present invention is a method of producing a cured tobacco material having an enhanced threonine content, the method including producing the cured tobacco material from (i) a tobacco plant which has been modified such that the expression or activity of an endogenous threonine aldolase is reduced or (ii) a part of the tobacco plant, wherein the endogenous threonine aldolase is at least one of the following threonine aldolases (a) and (b).
(a) A threonine aldolase comprised of a polypeptide which has a sequence identity of not less than 90% to a polypeptide which has the amino acid sequence shown in SEQ ID NO:2
(b) A threonine aldolase comprised of a polypeptide which has a sequence identity of not less than 90% to a polypeptide which has the amino acid sequence shown in SEQ ID NO:4

Each threonine aldolase described above is a protein encoded by a gene containing any of the polynucleotides which will be described later in detail in the (Gene) section. The threonine aldolase will be described later in detail in the (Polypeptide as threonine aldolase) section.

As used herein, the term "cured tobacco material" refers to a material obtained by curing a harvested tobacco plant or a part of the harvested tobacco plant.

In one embodiment, the production method of the present invention involves a curing step including curing a tobacco plant modified such that the expression or activity of an endogenous threonine aldolase is reduced or curing a part of the tobacco plant. The curing step will be described later in detail in the (Curing step) section.

As used herein, the term "cured tobacco material having an enhanced threonine content" means that the threonine content of a tobacco plant cured through the curing step or the threonine content of a part of the tobacco plant cured through the curing step is significantly enhanced as compared to a control.

For example, the threonine content of a cured tobacco plant or the threonine content of a cured part of a tobacco plant is 20% or more, more preferably 30% or more, even more preferably 40% or more, even more preferably 50% or more, even more preferably 60% or more, particularly preferably 80% or more, most preferably 100% greater than that of a control, per dry weight. Specifically, the threonine content per dry weight of a plant cured through the curing step or the threonine content per dry weight of a part of a plant cured through the curing step is preferably not less than 100 pg/g (DW), more preferably not less than 120 pg/g (DW), even more preferably not less than 140 pg/g (DW), even more preferably not less than 160 pg/g (DW), even more preferably not less than 180 pg/g (DW), even more preferably not less than 200 µg/g (DW), particularly preferably not less than 220 pg/g (DW), most preferably not less than 240 pg/g (DW). In one example, the cured tobacco material is a leaf material, and this cured tobacco material is 20% or more greater in threonine content than a cured tobacco material prepared from a control tobacco plant with no modification of a threonine aldolase or from a part of the control tobacco plant.

As used herein, the "threonine content of a cured tobacco material" is intended to mean the amount of free threonine in the cured tobacco material.

### (Tobacco plant or a part thereof)

First, the following description will discuss a tobacco plant or a part thereof for use in the production method of the present invention. As used herein, the term "tobacco plant or a part thereof" is intended to encompass a whole plant body, plant organs (such as leaves, roots, stems, axillary buds, flowers, and seeds), plant tissue fragments (such as epithelium, phloem, parenchyma, xylem, and vascular bundles), and plant cells (such as cell culture, callus, and protoplast) of a tobacco plant. The "tobacco plant or a part thereof" is preferably selected from whole plant body, plant organs (such as leaves, roots, stems, axillary buds, flowers, and seeds), and plant tissue fragments (such as epithelium, phloem, parenchyma, xylem, and vascular bundles), more preferably selected from whole plant body, plant organs (such as leaves, roots, stems, axillary buds, flowers, and seeds), and the like, even more preferably selected from whole plant body and leaves, most preferably a leaf. The leaf for use in the production method of the present invention may be a leaf at the tip of a stem of a plant (such a leaf is called "Tips") or may be a leaf positioned higher than the middle of a stem (such a leaf is called "Leaf"). The leaf may be a leaf on the stem.

A tobacco plant for use in the production method of the present invention is a tobacco plant modified such that the expression or activity of a threonine aldolase is reduced. As used herein, the phrase "expression or activity is reduced" is intended to mean that "expression is reduced" or "activity is reduced", which means that the level of "expression" of a threonine aldolase or the level of "activity" of the threonine aldolase is less than that of a control plant (e.g., a wild type plant of the same species) because of some hereditary factor. The phrase "expression or activity is reduced" is intended to also mean that "expression or activity is lost".

The "expression" of a threonine aldolase means that the threonine aldolase in a cell of a tobacco plant is translated and is present as protein (threonine aldolase protein).

As used herein, the phrase "reduced expression of a threonine aldolase" means that the amount of the threonine aldolase protein in a tobacco plant is less than that in a control tobacco plant (e.g., a wild type plant of the same species).

In a certain embodiment, a reduction in expression of the threonine aldolase can be such that: the amount of the threonine aldolase protein is not more than 80%, not more than 70%, not more than 60%, not more than 50%, not more than 40%, not more than 30%, not more than 20%, not more than 10%, not more than 5%, or not more than 1% of that in a control tobacco plant (e.g., a wild type plant of the same species).

The phrase "reduced activity of a threonine aldolase" means that the level of activity of the threonine aldolase protein itself is significantly less than that of a control tobacco plant (e.g., a wild type plant of the same species). In a certain embodiment, a reduction in activity of the threonine aldolase protein can be such that: the level of activity of the threonine aldolase protein is not more than 80%, not more than 70%, not more than 60%, not more than 50%, not more than 40%, not more than 30%, not more than 20%, not more than 10%, not more than 5%, or not more than 1% of that of a control in a wild type plant of the same species.

### (Gene [threonine aldolase gene])

A gene in accordance with the production method of the present invention is a gene that codes for a threonine aldolase which catalyzes catabolism of threonine to glycine. One example of such a gene is a gene that codes for a threonine aldolase which catalyzes catabolism of threonine to glycine and that, when the threonine aldolase has activity, catalyzes catabolism of threonine to glycine in the biosynthetic pathway of an amino acid derived from aspartic acid. The gene in accordance with the production method of the present invention hereinafter may be referred to as "threonine aldolase gene".

The gene in accordance with the production method of the present invention contains any of the following polynucleotides.
(a) A polynucleotide which has a sequence identity of not less than 90% to a polynucleotide which has the base sequence shown in SEQ ID NO:1
(b) A polynucleotide that codes for a protein which has a sequence identity of not less than 90% to the amino acid sequence shown in SEQ ID NO:2 and which has enzymatic activity to catalyze catabolism of threonine to glycine
(c) A polynucleotide that codes for a protein which has the same amino acid sequence as shown in SEQ ID NO:2 except that 1 to 35 amino acids are substituted, deleted, inserted and/or added and which has enzymatic activity to catalyze catabolism of threonine to glycine
(d) A polynucleotide that hybridizes with a polynucleotide comprised of a complementary sequence of the foregoing polynucleotide (a) under stringent conditions and that codes for a protein having enzymatic activity to catalyze catabolism of threonine to glycine
(e) A polynucleotide which has a sequence identity of not less than 90% to a polynucleotide which has the base sequence shown in SEQ ID NO:3
(f) A polynucleotide that codes for a protein which has a sequence identity of not less than 90% to the amino acid sequence shown in SEQ ID NO:4 and which has enzymatic activity to catalyze catabolism of threonine to glycine
(g) A polynucleotide that codes for a protein having the same amino acid sequence as shown in SEQ ID NO:4 except that 1 to 35 amino acids are substituted, deleted, inserted and/or added and having enzymatic activity to catalyze catabolism of threonine to glycine
(h) A polynucleotide that hybridizes with a polynucleotide comprised of a complementary sequence of the foregoing polynucleotide (e) under stringent conditions and that codes for a protein having enzymatic activity to catalyze catabolism of threonine to glycine

The sequence identity of each of the aforementioned base sequences is preferably not less than 91%, more preferably not less than 92%, even more preferably not less than 93%, even more preferably not less than 94%, even more preferably not less than 95%, even more preferably not less than 96%, particularly preferably not less than 97%, most preferably not less than 98% or not less than 99%. The number of amino acids substituted, deleted, inserted and/or added is preferably 1 to 32, more preferably 1 to 28, even more preferably 1 to 25, particularly preferably 1 to 21, most preferably 1 to 17.

The stringent conditions are, for example, the conditions disclosed in the Reference Literature "Molecular cloning-a Laboratory manual 2nd edition (Sambrook et al., 1989)". A more specific example of the stringent conditions is such that: a polynucleotide is allowed to hybridize with a probe in a solution containing 6×SSC (Composition of 1×SSC: 0.15 M sodium chloride, 0.015 M sodium citrate, pH7.0), 0.5% SDS, 5×Denhardt's, and 100 mg/mL herring sperm DNA, by incubating the polynucleotide and the probe in the solution at 65°C for 8 to 16 hours. It is noted that this polynucleotide preferably has a sequence identity of not less than 91%, more preferably not less than 92%, even more preferably not less than 93%, even more preferably not less than 94%, even more preferably not less than 95%, even more preferably not less than 96%, particularly preferably not less than 97%, most preferably not less than 98% or not less than 99% to the base sequence of the foregoing polynucleotide (a) or (e).

The threonine aldolase gene in accordance with the present invention can be present in the form of RNA (e.g., mRNA) or DNA (e.g., cDNA or genomic DNA). DNA may be double strand DNA or single strand DNA. The base sequence shown in SEQ ID NO:1, which is one example of a polynucleotide in accordance with the present invention, is a coding region (CDS) that codes for the polypeptide shown in SEQ ID NO:2. The base sequence shown in SEQ ID NO:3, which is another example of the polynucleotide in accordance with the present invention, is a coding region (CDS) that codes for the polypeptide shown in SEQ ID NO:4. The threonine aldolase gene in accordance with the present invention may be a gene that contains some additional sequence such as the sequence of an untranslated region (UTR).

Examples of the threonine aldolase gene include NtTHA1s gene (SEQ ID NO:1) in the S genome of *Nicotiana tabacum* (a tobacco plant) and NtTHAlt gene (SEQ ID NO:3) in the T genome of *Nicotiana tabacum. Nicotiana tabacum* is an allotetraploid, and these genes are orthologous genes derived from respective ancestor genomes.

In one example, a gene in accordance with the production method of the present invention contains both of or at least one of: one of the foregoing polynucleotides (a) to (d); and one of the foregoing polynucleotides (e) to (h).

There is no particular limitation on a part in which the threonine aldolase gene in accordance with the present invention is expressed and a growth stage in which the threonine aldolase gene is expressed. For example, the threonine aldolase gene in accordance with the present invention in a control plant is expressed in the whole body. It is preferable that the gene is expressed at least in a leaf and at least in the curing step.

### (Polypeptide as threonine aldolase)

A polypeptide in accordance with the production method of the present invention is a translation product of any of the genes described in the foregoing (Gene) section, and is a plant's threonine aldolase that has at least the activity of catalyzing catabolism of threonine to glycine. One example of the "threonine aldolase that catalyzes catabolism of threonine to glycine" is L-threonine aldolase (EC 4.1.2.5). L-threonine aldolase catalyzes the reaction that produces glycine and acetaldehyde from L-threonine.

A polypeptide as a threonine aldolase in accordance with the production method of the present invention is, more specifically, at least one of the following polypeptides (a) to (d). Note that each of these polypeptides is a translation product of a gene, and the definitions of the phrase "hybridize under stringent conditions" and the like as used in the following description are the same as those described in the foregoing (Gene) section.
(a) A polypeptide as a threonine aldolase, comprised of a polypeptide which has a sequence identity of not less than 90% to a polypeptide which has the amino acid sequence shown in SEQ ID NO:2
(b) A polypeptide as a threonine aldolase, comprised of a polypeptide which has the same amino acid sequence as shown in SEQ ID NO:2 except that 1 to 35 amino acids are substituted, deleted, inserted and/or added
(c) A polypeptide as a threonine aldolase, encoded by a polynucleotide that hybridizes with a polynucleotide comprised of a complementary sequence of a polynucleotide that codes for the foregoing polypeptide (a) under stringent conditions
(d) A polypeptide as a threonine aldolase, comprised of a polypeptide which has a sequence identity of not less than 90% to a polypeptide which has the amino acid sequence shown in SEQ ID NO:4
(e) A polypeptide as a threonine aldolase, comprised of a polypeptide which has the same amino acid sequence as shown in SEQ ID NO:4 except that 1 to 35 amino acids are substituted, deleted, inserted and/or added
(f) A polypeptide as a threonine aldolase, encoded by a polynucleotide that hybridizes with a polynucleotide comprised of a complementary sequence of a polynucleotide that codes for the foregoing polypeptide (d) under stringent conditions

The sequence identity of each of the aforementioned amino acid sequences is preferably not less than 91%, more preferably not less than 92%, even more preferably not less than 93%, even more preferably not less than 94%, even more preferably not less than 95%, even more preferably not less than 96%, particularly preferably not less than 97%, most preferably not less than 98% or not less than 99%. The number of amino acids substituted, deleted, inserted and/or added is preferably 1 to 32, more preferably 1 to 28, even more preferably 1 to 25, even more preferably 1 to 21, particularly preferably 1 to 17.

In one example, a polypeptide in accordance with the production method of the present invention includes both of: one of the foregoing polypeptides (a) to (c); and one of the foregoing polypeptides (e) to (f). Alternatively, the polypeptide in accordance with the production method of the present invention includes one of the foregoing polypeptides (a) to (f).

### (Aspects of tobacco plant for use in production method of the present invention)

A tobacco plant for use in the production method of the present invention is a tobacco plant modified such that the expression or activity of an endogenous threonine aldolase is reduced.

Note that a "reduction in expression or activity of an endogenous threonine aldolase" occurs because the tobacco plant contains a mutant allele as an endogenous gene that codes for the threonine aldolase or because of an exogenous polynucleotide introduced into the tobacco plant in order to suppress the expression of the endogenous gene that codes for the threonine aldolase.

That is, in one embodiment, a tobacco plant for use in the production method of the present invention is (i) a plant containing a mutant allele as an endogenous gene that codes for the threonine aldolase or (ii) a plant having an exogenous polynucleotide introduced therein to suppress the expression of that gene.

Note here that the mutant allele related to the threonine aldolase, contained in the tobacco plant for use in the production method of the present invention, can contain a mutation(s) selected from the group consisting of insertion, deletion, frameshift mutation, nonsense mutation, nonstop mutation, and splice-site mutation. The type of mutation can be determined by comparison with a wild-type allele in a control plant. Examples of the mutant allele include those containing an artificial mutation introduced by a technique selected from the group consisting of mutation induction, genome editing, and a knockout technique, and also include those containing a naturally occurring spontaneous mutation.

Examples of a technique of the mutation induction, genome editing, or knockout technique include exposure to a chemical or radiation, a technique using CRISPR system, a technique using transcription activator-like effector nucleases, a technique using zinc finger nucleases, oligonucleotide-directed mutagenesis, T-DNA insertion, and transposon insertion. The mutation induction, genome editing, and knockout technique will be described later in detail in the (Production of tobacco plant with reduced expression or activity of threonine aldolase) section.

The term "spontaneous mutation" refers to a mutation that occurs due to some natural cause such as radiation in nature or gene replication error, without artificial operations.

The foregoing mutant allele in one embodiment has the same meaning as a loss-of-function allele.

A tobacco plant for use in the production method of the present invention may contain a large-scale deletion of a chromosomal region containing a gene coding for a threonine aldolase.
A reduction in expression or activity of a threonine aldolase in the modified tobacco plant may be a constant reduction, an intermittent reduction, or a transient reduction. For example, a reduction in expression or activity of a threonine aldolase may be a reduction that occurs only when the tobacco plant is exposed to a temperature equal to or above a specific temperature. An example of such a threonine aldolase is one that contains a temperature-sensitive mutation and that loses its activity when heated.

Examples of the tobacco plant of the present invention also include: plant cells having an exogenous polynucleotide introduced therein to suppress the expression of an endogenous threonine aldolase; plants containing such a plant cell; tobacco varieties having the characteristics of such a plant; and clones thereof. Specifically, examples of the plant of the present invention include: "T0 generation", which refers to transgenic plants that are of the same genetic generation as the plant that was initially transformed; their offspring such as "T1 generation" (plants grown from seeds harvested from selfed T0 generation plants); hybrid plants obtained by crossing using any of those listed above as a parent, and their offspring; and plants having a construct (such as a vector containing an exogenous polynucleotide) introduced therein to suppress the expression of an endogenous threonine aldolase, and their offspring.

The "variety" of tobacco in this specification refers to a certain group of tobacco plants that show little genetic variation between individuals in terms of at least one feature distinguishable from other plants belonging to the same species. The variety can be maintained by a usual breeding technique. The variety of tobacco for use in the production method of the present invention is also characterized in that the expression or activity of an endogenous threonine aldolase has been reduced. In this specification, the term "variety" can be interpreted as meaning cultivar or line.

Note that, in a case where a tobacco plant's characteristic of reduced expression or activity of a threonine aldolase (this characteristic may be hereinafter referred to as "characteristic" for short) is given by a mutant allele related to a threonine aldolase, the term "tobacco variety" in this specification can usually be an inbred variety or a F1 hybrid variety. However, provided that the above characteristic is satisfied, the other characteristics do not necessarily have to be equal between individuals, unlike general inbred varieties or F1 hybrid variety. The foregoing tobacco variety can be produced by selfing of a tobacco plant having the foregoing characteristic, or by crossing between two different tobacco plants having the foregoing characteristic. A tobacco plant for use in the production method of the present invention, when used commercially, is particularly preferably a plant belonging to a tobacco variety characterized by reduced expression or activity of a threonine aldolase.

A tobacco plant with reduced expression or activity of a threonine aldolase can be grown from a seed obtained by selfing of a tobacco plant having the foregoing character or a seed obtained from crossing between two different tobacco plants having the foregoing character. A tobacco plant grown from such a seed usually has the foregoing character. The tobacco plant can alternatively be bred by reproducing an organ or an individual from a tissue or a cell through a tissue culture process such as callus induction.

### (Genotype of threonine aldolase gene)

In a case where a reduction in expression or in activity of an endogenous threonine aldolase in a tobacco plant is a reduction caused by the foregoing mutant allele, the tobacco plant, in terms of a gene locus that codes for a threonine aldolase, can be a homozygote in which a pair of alleles contains the same kind of mutation as each other or can be a heterozygote in which a pair of alleles contains respective different kinds of mutation.

The above gene mutation may be such that, for example, one mutation is present in one region in one gene locus, or two or more mutations are present in two or more different regions in one gene locus. In a case where two or more mutations are present in two or more different regions in one gene locus, such mutations may be respective different kinds of mutation. Note that such a mutation needs only be present in at least one of a pair of threonine aldolase alleles on homologous chromosomes (that is, heterozygous); however, it is preferable that both of the pair of alleles contain mutations.

In a case where a tobacco plant is an allotetraploid such as *Nicotiana tabacum,* it is preferable that a gene locus of at least one of the two orthologous genes is: homozygous such that a pair of alleles contains the same kind of mutation as each other; or heterozygous such that a pair of alleles contains respective different kinds of mutation.

It is particularly preferable, in such an allotetraploid, that all the four gene copies in the two gene loci related to a threonine aldolase are mutant alleles.

One example of a tobacco plant in accordance with the production method of the present invention is a double mutant of *Nicotiana tabacum* in which: both the NtTHAls gene shown in SEQ ID NO:1 and the NtTHAlt gene shown in SEQ ID NO:3 contain nonsense mutations; and both of these mutated NtTHA1 genes, i.e., mutant NtTHAls gene (e.g., SEQ ID NO:33 or SEQ ID NO:34) and mutant NtTHAlt gene (e.g., SEQ ID NO:35), are each homozygous. Another example of a tobacco plant in accordance with the production method of the present invention is a double mutant of *Nicotiana tabacum* in which: both the NtTHAls gene shown in SEQ ID NO:1 and the NtTHAlt gene shown in SEQ ID NO:3 contain one base insertion in each of their alleles; and both of these mutated NtTHA1 genes, i.e., mutant NtTHAls gene (e.g., SEQ ID NO:49) and mutant NtTHAlt gene (e.g., SEQ ID NO:50 or SEQ ID NO:51), are each homozygous. Generally, such tobacco plants cannot be obtained without artificial operations such as crossing or screening.

A tobacco plant modified such that the expression or activity of an endogenous threonine aldolase in accordance with the present invention is reduced may be a tobacco plant in which aspartate kinase (AK) less sensitive to feedback inhibition is expressed. The production method of the present invention, which involves using such a tobacco plant or a part thereof, is thereby capable of producing a cured tobacco material having a further enhanced threonine content.

In one embodiment, a tobacco plant in accordance with the present invention has a mutation introduced in its endogenous AK gene, and an AK protein less sensitive to feedback inhibition is expressed in the tobacco plant. Alternatively, the tobacco plant may be a tobacco plant that contains an AK protein, less sensitive to feedback inhibition, expressed by a polynucleotide which has been introduced into the plant using an appropriate recombinant expression vector and which codes for a mutant AK gene. As such, another example of the tobacco plant in accordance with the present invention can be a tobacco plant modified such that the expression or activity of an endogenous threonine aldolase is reduced and such that AK less sensitive to feedback inhibition is expressed.

Note that the recombinant expression vector for use in expression of AK can be suitable one of those listed in the following <Recombinant expression vector and transformant> section.

In one embodiment, the AK protein less sensitive to feedback inhibition, expressed in a tobacco plant, has the amino acid sequence shown in SEQ ID NO:36. This amino acid sequence is the same as that of AK protein in a wild type tobacco plant, except that leucine at the 405th position is substituted by phenylalanine. The AK protein less sensitive to feedback inhibition, expressed in a tobacco plant, may be such that its amino acid sequence is the same as that of AK protein in a wild type tobacco plant except that two or more amino acids are mutated, for example, substituted.

### (Tobacco plant obtained through judging method or screening method)

In one embodiment, whether or not a tobacco plant is suitable for use in the production method of the present invention can be judged based on the following indicator (a) or (b), that is, reduced expression or reduced activity of an endogenous threonine aldolase. In another embodiment, a tobacco plant suitable for use in the production method of the present invention can be bred through screening based on any of the following indicators (a) to (e) associated with a reduction in expression or activity of an endogenous threonine aldolase.
(a) Reduced expression of a threonine aldolase
(b) Reduced activity of a threonine aldolase
(c) Enhanced threonine content of a heat-treated leaf
(d) Presence or absence of a mutant allele related to a threonine aldolase
(e) A DNA marker linked to the mutant allele of (d) (linked DNA marker)
(f) Presence or absence of an exogenous polynucleotide introduced in order to suppress the expression of the above threonine aldolase gene

### <Method of judging that tobacco plant is suitable for use in production method of the present invention>

In one embodiment, when using a certain tobacco plant or a part thereof as a source material in the production method of the present invention, it is possible to judge whether or not the tobacco plant or the part thereof is suitable for use in the production method of the present invention by checking whether the expression or activity of a threonine aldolase in the candidate tobacco plant has been reduced.

The following description will discuss the judging method.

The judging method involves checking whether the expression of an endogenous threonine aldolase has been reduced or whether the activity of the threonine aldolase protein itself has been reduced. In one embodiment, the judgement can be made using, as an indicator, (a) reduced expression of an endogenous threonine aldolase or (b) reduced activity of the endogenous threonine aldolase. A specific means to make the judgement is not particularly limited, and may be, for example, a technique using a molecular genetic means or a technique using a biochemical means. In another embodiment, the judging method may further include (c) checking whether or not a heat-treated leaf has enhanced threonine content, in addition to the above checking (a) or (b).

### 1. Checking whether expression of threonine aldolase has been reduced

A reduction in expression of a threonine aldolase can be verified by determining the amount of a threonine aldolase protein in a tobacco plant or a part thereof. The determination of the amount of the protein can be carried out by, for example, western blotting, ELISA, or the like using an antibody. If necessary, the amount of the protein as a reference is used to determine whether or not the amount of the protein is reduced. The amount of the protein as a reference refers to, for example, the amount of a threonine aldolase protein in a control tobacco plant of the same species (e.g., a tobacco plant in which the expression or activity of a threonine aldolase has not been changed from normal state, for example, a tobacco plant not modified such that the expression or activity of the threonine aldolase is reduced).

Generally, a reduction in amount of a protein can be associated with a reduction in gene expression. The reduced expression level resulting from gene mutation is, in many cases, based on the degradation of mutant mRNA by a mechanism responsible for monitoring mRNA quality, such as nonsense-mediated mRNA decay (NMD), nonstop decay (NSD), and no go decay (NGD). Thus, the reduction in amount of a threonine aldolase protein can be verified by verifying a reduction in expression of a threonine aldolase gene. The reduced gene expression that would lead to a reduction in amount of a protein can be verified by checking the gene expression via a transcription product analysis using a specific probe or primer, such as Northern analysis or RT-PCR analysis.

The foregoing checking of reduced expression of a threonine aldolase may be carried out site-specifically in a specific growth stage, depending on need.

### 2. Check whether activity of threonine aldolase has been reduced

A reduction in activity of a threonine aldolase can be verified by, for example, any of the following methods 2-1 to 2-2.

### 2-1. Complementation test using glycine-requiring yeast

A reduction in enzymatic activity of a threonine aldolase protein itself in a certain tobacco plant can be determined by a complementation test using cDNA of a threonine aldolase gene isolated from the plant. In the complementation test, YM13 (*gly*1 *shm*1 *shm*2), which is a glycine-requiring yeast strain, can be suitably used as a host (Monschau et al. 1997, FEMS microbiology letters 150: 55-60., Joshi et al. 2006. The Plant Cell 18: 3564-3575.) In a case where a translation product of the isolated threonine aldolase gene has enzymatic activity, functional complementation occurs; therefore, the host having this gene introduced therein can be grown in a glycine-free culture medium. From the viewpoint of easy testing, generally the complementation test using yeast is carried out in preference to the following determination of polypeptide's activity.

### 2-2. Determination of activity of polypeptide

A polypeptide encoded by a threonine aldolase gene is isolated from a tobacco plant of interest or a part thereof. Alternatively, a clone of a threonine aldolase gene from a plant of interest is introduced into an expression vector, the expression vector is introduced into a host cell, and thereby a polypeptide is expressed transiently. The cell is cultured, subjected to extraction, and subjected to purification, and is used to check the presence or absence of an enzymatic activity that catalyzes catabolism of threonine (substrate) to glycine. If the enzymatic activity is absent or the enzymatic activity is lower than that of a control plant of the same species (e.g., homozygote for wild-type threonine aldolase gene), the activity of a threonine aldolase is regarded as reduced.

The recombinant expression vector and host cell that can be suitably used in foregoing expression of a polypeptide can be any of those listed in the (Recombinant expression vector and transformant) section or any of those known in the field of the art.

The methods of synthesis, extraction, and purification of a polypeptide are not particularly limited, and known methods can be suitably used. The method of synthesizing a polypeptide is, for example: allowing the polypeptide to be expressed in a leaf or the like of a tobacco plant transiently via an *Agrobacterium* species; or transfecting *Escherichia coli* with an expression vector for the polypeptide, and culturing the *Escherichia coli* to thereby allow the polypeptide to be expressed.

The method of determining the enzymatic activity that catalyzes glycine synthesis from threonine (substrate) is, for example, a method of: adding threonine to a polypeptide obtained by the foregoing method and incubating it to obtain a reaction solution; and determining the amount of acetaldehyde in the reaction solution (Reference Literature: Liu et al. 1998. European Journal of Biochemistry, 255, 220-226.)

In another embodiment, the method of determining the enzymatic activity is, for example, a method of: adding threonine (e.g., L-Thr) to a polypeptide obtained by the foregoing method and incubating it to obtain a reaction solution; purifying the reaction solution; and subjecting the resultant solution to LC/MS/MS analysis.

The method of determining the amount of each of the foregoing substances is, for example, a determination method using a mass spectrometer, a determination method using HPLC, or the like, among which a determination method using a mass spectrometer is preferred because of its high sensitivity and accuracy. Examples of the mass spectrometer for use in the determination include LC/MS/MS and GC/MS.

### 2-3. Determination of threonine content

Besides the foregoing methods <2-1> and <2-2>, the judgement of whether or not a tobacco plant is suitable for use in the production method of the present invention can be easily carried out by: heat-treating the tobacco plant or a part thereof; determining the amount of free threonine in the heat-treated tobacco plant or part thereof; and checking the determination result. For example, whether or not the tobacco plant is suitable can be judged by checking whether or not the amount of free threonine in the heat-treated tobacco plant or part thereof is significantly greater than when a control plant or a part thereof is heat-treated.

The phrase "significantly greater" is intended to mean that the amount is preferably 25% or more, more preferably 50% or more, 75% or more, 100% or more greater.

The foregoing heat treatment of the tobacco plant or a part thereof for determination of threonine content can be carried out by, for example, exposing a leaf cut from the tobacco plant to a condition involving a certain temperature and humidity for a certain period of time or longer. For example, the heat treatment can be carried out by exposing the leaf to a condition involving a temperature of 36 to 40 and a humidity of 80 to 90% for 24 to 48 hours.

### <Screening method for use in breeding tobacco plant suitable for production method of the present invention>

In one embodiment, a tobacco plant suitable for use in the production method of the present invention can be a tobacco plant bred through a screening method that involves carrying out screening based on an indicator(s) selected from the group consisting of the foregoing indicators (a) to (e).

As described earlier, whether or not a tobacco plant is suitable for use in the production method of the present invention can be judged based on the foregoing indicator (a) or (b), that is, reduced expression of an endogenous threonine aldolase or reduced activity of the endogenous threonine aldolase.

Note here that (a) the reduced expression of a threonine aldolase or (b) the reduced activity of a threonine aldolase, as described above, can be used as an indicator based on which screening is carried out during breeding of a tobacco plant suitable for use in the production method of the present invention. For example, (a) the reduced expression of a threonine aldolase or (b) the reduced activity of a threonine aldolase in a plant obtained from test cross is used as an indicator, and thereby the screening can be carried out effectively. Specific analytic means to verify (a) the reduced expression of a threonine aldolase or (b) the reduced activity of a threonine aldolase have already been described in regard to the foregoing judging method.

In another embodiment, a tobacco plant suitable for use in the production method of the present invention can be a tobacco plant bred through screening based on any of the following indicators: (d) presence or absence of a mutant allele related to a threonine aldolase; (e) a DNA marker linked to the mutant allele of (d); and (f) presence or absence of an exogenous polynucleotide introduced to suppress the expression of a threonine aldolase gene. That is, a tobacco plant for use in the production method of the present invention can be, for example, a tobacco plant bred through screening carried out by any of the following means 1 to 3.

### 1. Screening using, as indicator, presence/absence of mutant allele related to threonine aldolase

A mutation that causes a reduction in expression or activity of a threonine aldolase, in a threonine aldolase gene, is detected. The mutation is, for example, a mutation(s) selected from the group consisting of insertion, deletion, frameshift mutation, nonsense mutation, nonstop mutation, and splice-site mutation. The mutation can be detected by a variety of methods, a suitable example of which is a method using PCR-RFLP or real-time PCR (Cycling Probe Method, TaqMan (registered trademark) SNP Genotyping Assays). The genotype of a gene locus related to a threonine aldolase can be constantly monitored over the course of a breeding process in a general breeding program involving crossing, screening, and/or the like. Thus, a tobacco variety with reduced expression or activity of a threonine aldolase can be easily screened and bred by using the mutation of a mutant allele(s) itself as an indicator in the breeding program which makes use of the mutant allele(s) related to a threonine aldolase.

### 2. Screening using, as indicator, presence/absence of exogenous polynucleotide introduced to suppress expression of mutant allele related to threonine aldolase

In one aspect, a reduced expression of a threonine aldolase may be due to an exogenous polynucleotide introduced to suppress the expression of a threonine aldolase gene. In such a case, the screening and breeding of a tobacco plant having the foregoing characteristic can be carried out using, as an indicator, the presence or absence of an exogenous polynucleotide introduced to suppress the expression of a threonine aldolase gene. Alternatively, a constituent element (e.g., promoter sequence) of a construct introduced into the plant along with the exogenous polynucleotide may also be used as an indicator based on which the screening is carried out.

### 3. Screening using linked DNA marker as indicator

The presence or absence of a mutant allele, which is related to a threonine aldolase and which is relevant to a reduction in expression or activity of the threonine aldolase, can be checked using a DNA marker linked to the mutant allele. In one example, the foregoing linked DNA marker is present within a distance of about 10 M, preferably about 5 cM, more preferably about 2 cM, even more preferably about 1 cM, from the position where the threonine aldolase gene resides.

Examples of a polymorphic linked DNA marker usable for a marker assisted selection (MAS) in carrying out screening of tobacco plants include SNP, RFLP, RAPD, AFLP and microsatellites.

As has been described, a tobacco plant with reduced expression or activity of a threonine aldolase, for use in the production method of the present invention, may be a plant which has previously been judged as to whether or not it is suitable for use in the production method of the present invention by the foregoing judging method, and/or a plant belonging to a tobacco variety bred through the foregoing tobacco plant screening.

### (Production of tobacco plant with reduced expression or activity of threonine aldolase)

A tobacco plant modified such that the expression or activity of a threonine aldolase is reduced, in accordance with the present invention, includes a tobacco plant produced in the following manner.

Examples of a method of producing a tobacco plant with reduced expression or activity of a threonine aldolase include: a method which makes use of a mutant allele of a gene that codes for a threonine aldolase; and a method by which the expression of the gene is suppressed by introducing an exogenous polynucleotide.

As described earlier, a reduced expression or activity of a threonine aldolase is a genetic characteristic that occurs because of some hereditary factor. Therefore, a tobacco plant having this characteristic can be produced through a general breeding program involving crossing, screening, and/or the like.

### 1. Method which makes use of mutant allele

A mutant allele of a gene that codes for a threonine aldolase, which leads to a reduction in expression or activity of a threonine aldolase, contains a mutation(s) selected from the group consisting of insertion, deletion, frameshift mutation, nonsense mutation, nonstop mutation, and splice-site mutation.

The above mutation is usually intended to mean a mutation that causes a change in amino acid sequence that codes for a threonine aldolase protein, but may be a mutation that causes suppression of transcriptional activity of the threonine aldolase gene. Such a mutation is, for example, a mutation in a transcription regulatory sequence (e.g., promoter sequence or enhancer sequence).

The above mutant allele contains an artificial mutation caused by a technique selected from mutagen treatment, genome editing, and a knockout technique, or contains a naturally occurring spontaneous mutation. Specific examples of the mutagen treatment include: treatment with ethyl methane sulfonate (EMS); techniques involving irradiation with various kinds of ray such as electromagnetic wave, ultraviolet ray, X-ray, γ ray, corpuscular ray, neutron ray, α ray, and β ray; and techniques involving irradiation with a heavy ion beam or the like.

A plant in which a mutation is introduced into its threonine aldolase gene by a mutagen treatment can be searched for from a mutant group by, for example, some method such as TILLING (Till et al., 2003) or amplicon sequence analysis. Also, a tobacco plant whose threonine aldolase gene contains a spontaneous mutation can be searched for from a genetic resource collection of tobacco by a similar method. By analyzing the base sequence of a threonine aldolase gene (e.g., genomic DNA, mRNA, cDNA, or the like) of such a tobacco plant, it is possible to determine the type of the introduced mutation. This is also helpful to estimating whether or not the mutant allele contributes to a reduction in expression or activity of a threonine aldolase.

Examples of a polynucleotide of a nonsense mutant gene obtained through an EMS treatment include tobacco-derived polynucleotides having the base sequences shown in SEQ ID NO:33, SEQ ID NO:34, and SEQ ID NO:35, respectively, obtained in Examples provided in this specification.

Specific examples of the genome editing include genome editing techniques such as transcription activator-like effector nuclease (TALEN) technique, zinc finger nuclease (ZFN) technique, and clustered regularly interspaced short palindromic repeat (CRISPR) system (e.g., CRISPR/Cas9 and CRISPR/Cpf1). Examples of a polynucleotide of a single-base-insertion mutant gene obtained through genome editing using CRISPR/Cas9 include tobacco-derived polynucleotides having the base sequences shown in SEQ ID NO:49, SEQ ID NO:50, and SEQ ID NO:51, respectively, obtained in Examples provided in this specification.

In the CRISPR/Cas9 system, a base sequence immediately upstream of XGG on a genome and a sequence complementary to the base sequence are recognized by guide RNA, and cleaved by Cas9 within the base sequence. The base sequence recognized by the guide RNA is, for example, a part of a polynucleotide having a sequence identity of not less than 90% to a polynucleotide having the base sequence shown in SEQ ID NO:1 or SEQ ID NO:3 or a part of a polynucleotide that codes for a polypeptide having a sequence identity of not less than 90% to a polypeptide having the amino acid sequence shown in SEQ ID NO:2 or SEQ ID NO:4. The part of the polynucleotide is comprised of 10 or more, preferably 17 or more, more preferably 20 or more successive bases immediately upstream of XGG.

In the TALEN technique, a pair of DNA-binding domains of dimerized artificial nucleases binds to the respective base sequences which have a 5- to 7-base spacer therebetween and which are opposite from each other with the FokI cleavage domains therebetween. Each of the DNA-binding domains is structured such that each of the bases of the base sequence to which the DNA-binding domain binds and the modules is constituted by 33 or 34 amino acid residue repeats (modules). Each of the base sequences to which the DNA-binding domains bind is a part of a polynucleotide having a sequence identity of not less than 90% to a polynucleotide having the base sequence shown in SEQ ID NO:1 or SEQ ID NO:3 or a part of a polynucleotide that codes for a polypeptide having a sequence identity of not less than 90% to a polypeptide having the amino acid sequence shown in SEQ ID NO:2 or SEQ ID NO:4. Such parts of the polynucleotide are each comprised of 6 or more, preferably 10 or more, more preferably 21 or more successive bases and are present opposite from each other with a 5- to 7-base spacer therebetween and with the FokI cleavage domains therebetween.

In the ZFN technique, as with the TALEN technique, a pair of DNA-binding domains of dimerized artificial nucleases binds to the respective base sequences which have a 5- to 7-base spacer therebetween and which are opposite from each other with the FokI cleavage domains therebetween. Each of the DNA-binding domains is constituted by a plurality of zinc finger modules. Each of the above base sequences is a part of a polynucleotide having a sequence identity of not less than 90% to a polynucleotide having the base sequence shown in SEQ ID NO:1 or SEQ ID NO:3 or a part of a polynucleotide that codes for a polypeptide having a sequence identity of not less than 90% to a polypeptide having the amino acid sequence shown in SEQ ID NO:2 or SEQ ID NO:4. Such parts of the nucleotide are each comprised of 9 or more, preferably 12 or more, more preferably 18 or more successive bases and are present opposite from each other with a 5- to 7-base spacer therebetween and with the FokI cleavage domains therebetween.

Specific examples of the knockout technique include: gene disruption techniques using various kinds of transposon such as retrotransposon; and gene disruption techniques using T-DNA or the like. Specific examples of these techniques include inserting, into a tobacco plant, the retrotransposon tnt1 of tobacco, a transposon from some other plant, or T-DNA of the Ti plasmid of an *Agrobacterium* species.

### 2. Method of suppressing gene expression by introducing exogenous polynucleotide

The following description will discuss a method of producing a tobacco plant in which the expression of a threonine aldolase gene is suppressed by introducing an exogenous polynucleotide.

The exogenous polynucleotide can be a polynucleotide that suppresses the expression of the threonine aldolase gene. Such a tobacco plant is produced by, for example, introducing a construct containing the polynucleotide into a plant. The construct is, for example, a vector with an expression cassette that contains a polynucleotide which suppresses gene expression, or the like.

The technique of suppressing the expression of the endogenous threonine aldolase gene by introducing an exogenous polynucleotide into a plant is, for example, an RNA interference technique, an antisense RNA technique, a co-suppression technique, a technique using a complex of a carrier (such as antibody or peptide) and a nucleic acid, a technique by which a nucleic acid is introduced into a cell using a nucleic acid carrier (e.g., functional peptides such as an in vivo signal peptide), a technique of inhibiting a transcriptional regulator by a decoy nucleic acid, or the like. That is, the foregoing polynucleotide that suppresses the expression of the gene is preferably a nucleotide molecule for use in such a technique and is a molecule selected from the group consisting of antisense RNA molecules, RNAi molecules, and cosuppressor molecules.

As used herein, the antisense technique refers to a technique by which antisense RNA complementary to mRNA transcribed from a target gene is expressed, the antisense RNA is allowed to bind complementarily to the target gene to thereby inhibit the translation of a target protein, and, as a result, the expression of the gene is suppressed. Specifically, it is possible to produce the antisense RNA complementary to the mRNA by: linking a polynucleotide related to the threonine aldolase gene or a fragment thereof to a promoter that is capable of functioning in a plant such that the polynucleotide or the fragment thereof is positioned downstream of, in the antisense direction, of the promoter; and introducing it into a plant cell or tissue.

The RNA interference technique refers to a technique by which the expression of a target gene is suppressed by making use of a phenomenon of degrading target RNA in the following mechanism. First, dsRNA (double-strand RNA) is allowed to form within a cell by using a vector that contains DNA serving as a template for the dsRNA. Next, the dsRNA is cleaved with a double-strand-RNA-specific RNase (Dicer) to form siRNA (small interfering RNA). Then, the siRNA is allowed to constitute a part of a RNA-induced silencing complex (RISC) molecule to form a complex, and, finally, the target mRNA is recognized by the RISK by homology, and thereby the mRNA is degraded.

The vector for use in the RNA interference is preferably a vector that expresses the RNAi-inducing dsRNA in the form of a hairpin. Such an RNAi vector that expresses the dsRNA can be an RNAi vector in the form of a hairpin in which DNAs, corresponding to the portions contributing to the formation of the dsRNA, are positioned at the opposite ends of a spacer sequence (such as an intron) of several bases or longer such that the inversed repeat (IR) is achieved. The structure of the vector will be described later in detail.

The RNAi vector may be a tandem vector arranged such that sense RNA and antisense RNA are transcribed by respective different promoters and that the transcription products hybridize within a cell to produce dsRNA. Alternatively, the RNAi may be caused by constructing a plurality of expression vectors from which the sense RNA and the antisense RNA are transcribed, respectively.

The co-suppression technique refers to a phenomenon in which, upon introduction of a gene having a sequence equal or similar to a target endogenous gene into a plant through transformation, both the introduced exogenous gene and the target endogenous gene are suppressed from expression. One way to obtain a plant in which the threonine aldolase gene is co-suppressed may be, for example, to transform a target plant by using a vector prepared such that the gene or DNA having a sequence similar to the gene are expressed.

A polynucleotide that suppresses the expression of such a gene is introduced into a plant using a recombinant expression vector that contains the above polynucleotide. A method of introducing the recombinant expression vector containing the polynucleotide that suppresses the expression of such a gene into a plant is not limited to a particular method, and may be selected from, for example, a polyethylene glycol method, an electroporation method, a method using an *Agrobacterium* species, a particle gun method, and the like, depending on need. This recombinant expression vector containing the polynucleotide may be introduced into a plant cell, callus, plant tissue, or a plant body.

The exogenous polynucleotide to be introduced in order to suppress the expression of the threonine aldolase gene may be one that contains a sequence of a promoter region or an enhancer region of the endogenous threonine aldolase gene.

### <Recombinant expression vector and transformant>

The following description will discuss a recombinant expression vector applied to a tobacco plant in accordance with the present invention and a transformant having an exogenous polynucleotide in accordance with the present invention introduced therein in an expressible manner.

A vector for construction of a recombinant expression vector is not limited to a particular kind, and may be appropriately selected from those that are expressible in a host cell. Specifically, a recombinant expression vector obtained in the following manner may be used: an appropriate promoter sequence is selected according to the type of host cell; and the promoter sequence and a polynucleotide related to the threonine aldolase gene in accordance with the present invention are incorporated into, for example, a plasmid, a phagemid, a cosmid, or the like.

The recombinant expression vector is preferably an expression suppressing vector. In the recombinant expression vector, the polynucleotide in accordance with the present invention is operably linked to an element necessary for transcription (such as a promoter). The polynucleotide in accordance with the present invention may optionally be linked to, for example, an enhancer, a selective marker, a splicing signal, a poly(A) addition signal, and/or a 5'-UTR sequence. The promoter is a DNA sequence that shows transcriptional activity in a host cell, and can be appropriately selected depending on the type of host cell.

Examples of the promoter sequence that is capable of functioning in a host cell include: 35S promoter from cauliflower mosaic virus; nopaline synthase gene promoter from an *Agrobacterium* species; ubiquitin gene promoter derived from corn; and actin gene promoter derived from rice. Examples of the promoter sequence that is capable of functioning in a host cell further include: senescence-induced promoter of SAG12 gene from *Arabidopsis thaliana* (Ori et al., The Plant Cell11.6: 1073-1080 (1999), PCT International Application Publication No. WO 2010/018234 A1); and heat-induced promoter of HSP18.2 gene from *Arabidopsis thaliana* (Yoshida, K., et al., Applied microbiology and biotechnology 44.3-4 466-472 (1995)).

Alternatively, the foregoing promoter in accordance with the present invention may be a sequence of a promoter region of the endogenous threonine aldolase gene in accordance with the present invention.

The preparation of a recombinant expression vector and a transformant is carried out with the use of, for example, an isolated polynucleotide of the threonine aldolase gene. A method of obtaining (isolating) a polynucleotide related to the threonine aldolase gene is not particularly limited, and, for example, the following method may be employed: preparing a probe that specifically hybridizes with a part of the base sequence of the threonine aldolase gene; and carrying out screening on a genomic DNA library or a cDNA library with the probe.

Another example of a method of obtaining the polynucleotide related to the threonine aldolase gene is a method using an amplifying means such as PCR. For example, primers are prepared from the 5'- and 3'-side sequences (or sequences complementary thereto) of cDNA of the threonine aldolase gene, and PCR or the like is carried out using these primers and using genomic DNA (or cDNA) or the like as a template, and thereby a DNA region between the primers is amplified. In this way, a large amount of DNA fragments containing the polynucleotide related to the threonine aldolase gene can be obtained.

In the recombinant expression vector, the polynucleotide in accordance with the present invention may optionally be linked to an appropriate terminator. For example, the polynucleotide may be operably linked to a terminator of nopaline synthase (NOS) gene and a terminator of CaMV35S RNA gene. The type of terminator may be selected appropriately depending on the type of host cell.

An enhancer is used to improve the expression efficiency of a target gene. Examples of the enhancer include: an enhancer region including an upstream sequence of the CaMV35S promoter; and omega sequence from tobacco mosaic virus.

The recombinant expression vector in accordance with the present invention may further contain a selective marker. Examples of the selective marker include drug resistance genes resistant to a drug such as ampicillin, kanamycin, tetracycline, chloramphenicol, neomycin, hygromycin or spectinomycin.

A reporter gene is not limited to a particular kind, provided that it is possible to check whether or not a plant cell has been transformed by the expression of a gene. Examples of the reporter gene include: β-glucuronidase (GUS); luciferase (LUC); fluorescent proteins such as green fluorescent protein (GFP) and cyan fluorescent protein (CFP); and beta-galactosidase (LacZ).

The following arrangement may be employed: an expression cassette containing a reporter gene, and a target gene, are carried on respective different recombinant expression vectors. In a case where an expression cassette containing a reporter gene and a target gene are carried on respective different recombinant expression vectors, the vectors may be co-transfected into a host.

In the recombinant expression vector, the polynucleotide in accordance with the present invention may optionally be bound to a suitable spacer sequence. Furthermore, one example of the recombinant expression vector is a vector structured such that the gene in accordance with the present invention is bound downstream of a temperature sensitive promoter, or the like.

A vector applied to a tobacco plant in accordance with the present invention can be a gene expression suppressing vector. The gene expression suppressing vector is obtained by inserting a polynucleotide for suppressing the expression of any of the genes described in the (Gene) section into a suitable vector.

Among recombinant vectors for use in transformation of a tobacco plant, an expression suppressing vector is a vector to suppress the expression of an endogenous gene in a plant cell, and can be used to produce a transgenic plant. This vector contains, for example, a polynucleotide having a base sequence selected from the group consisting of: base sequences having a sequence identity of preferably not less than 90%, more preferably not less than 91%, even more preferably not less than 92%, even more preferably not less than 93%, even more preferably not less than 94%, even more preferably not less than 95%, even more preferably not less than 96%, even more preferably not less than 97%, particularly preferably not less than 98%, most preferably not less than 99%, to at least one of a polynucleotide having the base sequence shown in SEQ ID NO:1 and a polynucleotide having the base sequence shown in SEQ ID NO:3. Such a vector is, for example, an RNA interference inducing vector, an antisense vector, or a co-suppression vector.

The RNA interference inducing vector is a vector that contains DNA serving as a template for double-strand RNA (dsRNA) that triggers the RNA interference. A polynucleotide corresponding to a part from which the double-strand RNA triggering the RNA interference is formed is a polynucleotide for suppressing the expression of an endogenous threonine aldolase gene. This polynucleotide is a polynucleotide (sense RNA portion) shown in a base sequence comprised of the following bases or a polynucleotide (antisense RNA portion) shown in a base sequence complementary to that polynucleotide: at least 21 to 30 successive bases (for example, 21 or more successive bases, 22 or more successive bases, 23 or more successive bases, 24 or more successive bases, 25 or more successive bases, 26 or more successive bases, 27 or more successive bases, 28 or more successive bases, 29 or more successive bases, 30 or more successive bases) of a polynucleotide having the base sequence shown in SEQ ID NO:1, a polynucleotide having the base sequence shown in SEQ ID NO:3, or a polynucleotide having a sequence identity of preferably not less than 90%, more preferably not less than 91%, even more preferably not less than 92%, even more preferably not less than 93%, even more preferably not less than 94%, even more preferably not less than 95%, even more preferably not less than 96%, even more preferably not less than 97%, particularly preferably not less than 98%, most preferably not less than 99%, to the polynucleotide having the base sequence shown in SEQ ID NO:1 or the polynucleotide having the base sequence shown in SEQ ID NO:3. The base sequence comprised of "at least 21 to 30 successive bases" more specifically refers to a base sequence comprised of 21 or more successive bases, 23 or more successive bases, 25 or more successive bases, 30 or more successive bases, 35 or more successive bases, 40 or more successive bases, 45 or more successive bases, 50 or more successive bases, 60 or more successive bases, 70 or more successive bases, 80 or more successive bases, 90 or more successive bases, or 100 or more successive bases.

An example of the RNA interference inducing vector is a vector structured such that the sequence of the DNA serving as a template triggering the RNA interference is bound downstream of a senescence-induced promoter or a temperature sensitive promoter, or the like vector.

An antisense vector is a vector that contains a polynucleotide which suppresses the expression of a threonine aldolase gene by an antisense method. A polynucleotide molecule for suppressing the expression of the endogenous threonine aldolase gene, for introduction into an antisense vector, may be an antisense nucleotide molecule having a part of a polynucleotide having the base sequence shown in SEQ ID NO:1, an antisense nucleotide molecule having a part of a polynucleotide having the base sequence shown in SEQ ID NO:3, or an antisense nucleotide molecule having a part of a base sequence having a sequence identity of preferably not less than 90%, more preferably not less than 91%, even more preferably not less than 92%, even more preferably not less than 93%, even more preferably not less than 94%, even more preferably not less than 95%, even more preferably not less than 96%, even more preferably not less than 97%, particularly preferably not less than 98%, most preferably not less than 99%, to at least one of the polynucleotide having the base sequence shown in SEQ ID NO:1 and the polynucleotide having the base sequence shown in SEQ ID NO:3. The polynucleotide molecule may be, for example, an antisense nucleotide molecule having a base sequence comprised of 15 or more successive bases but less than the whole length, 20 or more successive bases but less than the whole length, preferably 100 or more successive bases but less than the whole length, more preferably 500 or more successive bases but less than the whole length, of any of the above sequences.

A co-suppression vector is a vector that contains a cosuppressor molecule which suppresses the expression of the threonine aldolase gene by a co-suppression method. A nucleotide molecule for use in the co-suppression is a polynucleotide for suppressing the expression of the endogenous threonine aldolase gene, and is comprised of 50 or more successive bases, preferably 100 successive bases, more preferably 500 successive bases of a polynucleotide having the base sequence shown in SEQ ID NO:1, a polynucleotide having the base sequence shown in SEQ ID NO:3, or a polynucleotide having a sequence identity of preferably not less than 90%, more preferably not less than 91%, even more preferably not less than 92%, even more preferably not less than 93%, even more preferably not less than 94%, even more preferably not less than 95%, even more preferably not less than 96%, even more preferably not less than 97%, particularly preferably not less than 98%, most preferably not less than 99%, to the polynucleotide having the base sequence shown in SEQ ID NO:1 or the polynucleotide having the base sequence shown in SEQ ID NO:3. The nucleotide molecule for use in the co-suppression may be a nucleotide having a base sequence comprised of, for example, 50 or more successive bases but less than the whole length, preferably 100 or more successive bases but less than the whole length, more preferably 500 or more successive bases but less than the whole length, of any of the above base sequences.

A transformant can be a plant body, plant cell, plant tissue fragment, plant organ, or the like, which has the foregoing recombinant expression vector or the polynucleotide related to the threonine aldolase gene introduced therein in an expressible manner.

In a case where a reduced expression of a threonine aldolase results from an exogenous polynucleotide introduced in order to suppress the expression of the gene, the tobacco plant is preferably homozygous for the introduced polynucleotide sequence, but may be hemizygous.

### (Curing step)

A method of producing a cured tobacco material in accordance with one embodiment of the present invention involves a curing step including curing a tobacco plant or a part thereof.

In this specification, "curing" of a tobacco plant or a part thereof is intended to not only remove moisture from a plant but also promote changes of the metabolic compounds in the plant.

A curing method used to produce a cured tobacco material having an enhanced threonine content, in this specification, is not particularly limited, and may be a generally-used tobacco leaf curing method or a method falling under this category. Examples of the tobacco leaf curing method include, but are not limited to, flue curing, air curing, fire curing, sun curing, and combinations of any of these curing methods. In one embodiment, curing is carried out by a method(s) selected from the group consisting of flue curing, air curing, fire curing, and sun curing. Generally, the tobacco leaf curing method usually depends on the type of tobacco. For example, Virginia tobacco, Burley tobacco and Orient tobacco are cured by the process of flue-curing, air-curing, sun-curing respectively.

Equipment or apparatus for use in curing a tobacco plant or a part thereof is not particularly limited. For example, in a case where the curing is carried out by flue curing, a space or equipment in which temperature and humidity can be controlled is generally used. In a case where curing is carried out by air curing, a facility such as a shed is generally used, in which the curing of a harvested tobacco plant or a part thereof can proceed without exposure to rain, direct sunlight, and the like.

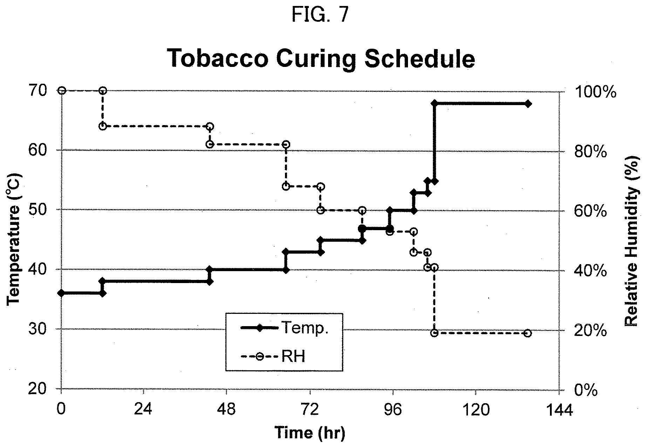

A curing program for curing a tobacco plant or a part thereof can be changed in its configuration appropriately depending on the state of the tobacco plant or part thereof to be cured, such as stage, maturity, and organ. In a case where a leaf of a tobacco plant is cured, for example, a curing program used in generally-used flue curing may be employed. The curing program is, for example, a program illustrated in Fig. 7 (discussed in Examples).

Furthermore, a certain kind of heat treatment enhances the threonine content of a harvested tobacco plant or a part thereof. The degradation of protein in a leaf is accelerated by a mechanism called heat-induced leaf senescence, under the conditions to be exposed to heat stress. The protein degradation product resulted from such heat stress contributes directly or indirectly to enhancement of the threonine content of the tobacco plant or part thereof. Especially in a case where the tobacco plant is a tobacco plant modified such that the expression or activity of an endogenous threonine aldolase is reduced, the enhancement of the threonine content resulting from the foregoing heat treatment is more noticeable than when a control plant is heat-treated. In addition, tobacco leaves, which are subjected to a curing treatment by a generally-used method, are usually exposed to the heat stress. The conditions (such as temperature and time) under which the heat treatment for enhancing the threonine content is carried out may be selected using, for example, the protein degradation as an indicator. The amount of protein can be determined by a Bradford method, a bicinchoninic acid (BCA) method, or the like. The temperature and time may be selected using, as an indicator, the fact that not less than 10%, not less than 20%, not less than 30%, not less than 40%, not less than 50%, or not less than 60% of the total protein has been degraded. Furthermore, the heat-induced leaf senescence also promotes the degradation of chlorophyll; therefore, the conditions under which the heat treatment is carried out can be selected using, as an indicator, the chlorophyll degradation in a leaf. The amount of chlorophyll can be measured easily with the use of a chlorophyll meter or the like. For example, the following method may be employed: temperature and time are selected using, as an indicator, the fact that not less than 5%, not less than 10%, not less than 15%, or not less than 20% of the total chlorophyll has been degraded.

A specific condition under which the heat treatment is carried out is, for example, exposure to a moist atmosphere at about 38 degrees Celsius for 12 hours or longer. The degradation of protein is influenced by the temperature and time at and for which the treatment is carried out, and therefore the temperature and time should be adjusted appropriately. In the above heat treatment, the temperature to which the tobacco plant or part thereof is subjected preferably falls within the range of not lower than 25°C and not higher than 60°C, more preferably falls within a temperature range selected from the following ranges:
28°C to 60°C, 30°C to 60°C, 32°C to 60°C, 34°C to 60°C, 36°C to 60°C, 38°C to 60°C, 40°C to 60°C, 42°C to 60°C, 44°C to 60°C, 46°C to 60°C, 48°C to 60°C, 50°C to 60°C, 52°C to 60°C, 54°C to 60°C, 56°C to 60°C,
28°C to 58°C, 30°C to 58°C, 32°C to 58°C, 34°C to 58°C, 36°C to 58°C, 38°C to 58°C, 40°C to 58°C, 42°C to 58°C, 44°C to 58°C, 46°C to 58°C, 48°C to 58°C, 50°C to 58°C, 52°C to 58°C, 54°C to 58°C,
28°C to 56°C, 30°C to 56°C, 32°C to 56°C, 34°C to 56°C, 36°C to 56°C, 38°C to 56°C, 40°C to 56°C, 42°C to 56°C, 44°C to 56°C, 46°C to 56°C, 48°C to 56°C, 50°C to 56°C, 52°C to 56°C,
28°C to 54°C, 30°C to 54°C, 32°C to 54°C, 34°C to 54°C, 36°C to 54°C, 38°C to 54°C, 40°C to 54°C, 42°C to 54°C, 44°C to 54°C, 46°C to 54°C, 48°C to 54°C, 50°C to 54°C,
28°C to 52°C, 30°C to 52°C, 32°C to 52°C, 34°C to 52°C, 36°C to 52°C, 38°C to 52°C, 40°C to 52°C, 42°C to 52°C, 44°C to 52°C, 46°C to 52°C, 48°C to 52°C,
28°C to 50°C, 30°C to 50°C, 32°C to 50°C, 34°C to 50°C, 36°C to 50°C, 38°C to 50°C, 40°C to 50°C, 42°C to 50°C, 44°C to 50°C, 46°C to 50°C,
28°C to 48°C, 30°C to 48°C, 32°C to 48°C, 34°C to 48°C, 36°C to 48°C, 38°C to 48°C, 40°C to 48°C, 42°C to 48°C, 44°C to 48°C,
28°C to 46°C, 30°C to 46°C, 32°C to 46°C, 34°C to 46°C, 36°C to 46°C, 38°C to 46°C, 40°C to 46°C, 42°C to 46°C, 28°C to 44°C, 30°C to 44°C, 32°C to 44°C, 34°C to 44°C, 36°C to 44°C, 38°C to 44°C, 40°C to 44°C,
28°C to 42°C, 30°C to 42°C, 32°C to 42°C, 34°C to 42°C, 36°C to 42°C, 38°C to 42°C,
28°C to 40°C, 30°C to 40°C, 32°C to 40°C, 34°C to 40°C, 36°C to 40°C,
28°C to 38°C, 30°C to 38°C, 32°C to 38°C, 34°C to 38°C,
28°C to 36°C, 30°C to 36°C, 32°C to 36°C,
28°C to 34°C, 30°C to 34°C, and 28°C to 32°C.

Furthermore, in the above heat treatment, the time for which the plant or part thereof is exposed preferably falls within the range of from 2 hours to 96 hours, more preferably falls within a time range selected from the following ranges:
2 hours to 96 hours, 4 hours to 96 hours, 8 hours to 96 hours, 12 hours to 96 hours, 16 hours to 96 hours, 24 hours to 96 hours, 36 hours to 96 hours, 48 hours to 96 hours, 72 hours to 96 hours,
2 hours to 72 hours, 4 hours to 72 hours, 8 hours to 72 hours, 12 hours to 72 hours, 16 hours to 72 hours, 24 hours to 72 hours, 36 hours to 72 hours, 48 hours to 72 hours,
2 hours to 48 hours, 4 hours to 48 hours, 8 hours to 48 hours, 12 hours to 48 hours, 16 hours to 48 hours, 24 hours to 48 hours, 36 hours to 48 hours,
2 hours to 36 hours, 4 hours to 36 hours, 8 hours to 36 hours, 12 hours to 36 hours, 16 hours to 36 hours, 24 hours to 36 hours,
2 hours to 24 hours, 4 hours to 24 hours, 8 hours to 24 hours, 12 hours to 24 hours, 16 hours to 24 hours,
2 hours to 16 hours, 4 hours to 16 hours, 8 hours to 16 hours, 12 hours to 16 hours,
2 hours to 12 hours, 4 hours to 12 hours, 8 hours to 12 hours,
2 hours to 8 hours, 4 hours to 8 hours, and
2 hours to 4 hours.

The humidity at which the heat treatment is carried out is not particularly limited. For example, in a case where a curing apparatus is used, the humidity inside the curing apparatus is preferably 60 to 95%, more preferably 70 to 90%.

The threonine content of a tobacco plant or a part thereof is enhanced through the above heat treatment; however, usually, this treatment alone is not sufficient to complete the curing. Generally, the curing needs to be completed by removing most of the remaining moisture and terminating the changes of the components. Thus, in one embodiment, the above heat treatment may be carried out in combination with some other drying method which is mainly for moisture removal. Alternatively, the above heat treatment may be carried out in combination with a generally-used tobacco leaf curing method. For example, the above heat treatment may be carried out at an early stage of an existing curing program.

In this specification, the temperature at which a curing treatment is carried out refers to the average temperature in a curing environment (e.g., in a curing shed or curing apparatus) in the curing step. The average temperature can be obtained by measuring temperature(s) at one or more points or one or more positions within a curing shed or a curing apparatus.

In the heat treatment, the heating temperature may be raised stepwise over time. A program for a single heat treatment may contain one or more temperature-raising periods and/or one or more temperature-lowering periods. The heating may be carried out continuously or intermittently. That is, a certain interval may be provided between heating periods.

The heat treatment may be carried out by burning fuel or the like, or may be carried out using heat from sunlight or the like or using electricity. A closed facility such as a simple plastic greenhouse may be used. Hot air may be used.

The heat treatment can be a treatment that includes a stage during which a tobacco plant or a part thereof is exposed to an environment of a certain temperature for a certain period of time.

In one example, a tobacco plant or a part thereof for use in the production method in accordance with the present invention is such that its threonine content is finally enhanced through the curing step.

### (Type of tobacco plant suitable for use in production method)

The production method in accordance with the present invention can be applied to tobacco plants belonging to the *Nicotiana* genus. A tobacco plant is not limited to a particular kind, provided that the plant belongs to the *Nicotiana* genus. Examples of the tobacco plant include *Nicotiana tomentosiformis, Nicotiana sylvestris, Nicotiana rustica* (Aztec tobacco), *Nicotiana tabacum, Nicotiana plumbaginifolia, Nicotiana alata* (flowering tobacco), *Nicotiana acaulis, Nicotiana acuminata, Nicotiana acuminata var. multzjlora, Nicotiana africana, Nicotiana amplexicaulis, Nicotiana arentsii, Nicotiana attenuata, Nicotiana benavidesii, Nicotiana benthamiana, Nicotiana bigelovii, Nicotiana bonariensis, Nicotiana cavicola, Nicotiana clevelandii, Nicotiana cordifolia, Nicotiana corymbosa, Nicotiana debneyi, Nicotiana excelsior, Nicotianaforgetiana, Nicotiana fragrans, Nicotiana glauca, Nicotiana glutinosa, Nicotiana goodspeedii, Nicotiana gossei, Nicotiana hybrid, Nicotiana ingulba, Nicotiana kawakamii, Nicotiana knightiana, Nicotiana langsdorfi, Nicotiana linearis, Nicotiana longiflora, Nicotiana maritima, Nicotiana megalosiphon, Nicotiana miersii, Nicotiana noctiflora, Nicotiana nudicaulis, Nicotiana obtusifolia, Nicotiana occidentalis, Nicotiana occidentalis subsp. Hesperis, Nicotiana otophora, Nicotiana paniculata, Nicotiana pauczjlora, Nicotiana petunioides, Nicotiana plumbaginifolia, Nicotiana quadrivalvis, Nicotiana raimondii, Nicotiana repanda, Nicotiana rosulata, Nicotiana rosulata subsp. Ingulba, Nicotiana rotundifolia, Nicotiana setchellii, Nicotiana simulans, Nicotiana solanifolia, Nicotiana spegauinii, Nicotiana stocktonii, Nicotiana suaveolens, Nicotiana thyrsiflora, Nicotiana tomentosa, Nicotiana trigonophylla, Nicotiana umbratica, Nicotiana undulata, Nicotiana velutina,* and *Nicotiana wigandioides.* Examples of the variety include various varieties such as flue-cured variety (Virginia variety), Burley variety, cigar variety, Orient variety, and differentiated local varieties suited to the local climates. In one embodiment, a plant belonging to the *Nicotiana* genus selected from the group consisting of *Nicotiana tabacum* and *Nicotiana rustica* is used in the production method in accordance with the present invention.

### [2. Cured tobacco material]

The present invention also provides a cured tobacco material produced by the foregoing production method. As described earlier, a "cured tobacco material" as used herein contains a cured tobacco plant or a part thereof obtained by curing a harvested tobacco plant or a part thereof.

A cured tobacco material produced by the production method of the present invention can be used in any tobacco product production applications, such as: smoking products including e-cigarettes, heat-not-burn tobacco, paper-wrapped cigarettes, cigars, and pipe smoking tobacco; and smokeless tobacco products including snuff tobacco and chewing tobacco.

The cured tobacco material produced by the production method of the present invention can be used in production of any of the above-listed products after being subjected to an additional processing treatment accompanied by changes of components and/or physical properties, such as steaming, roasting, or swelling, which are non-limiting examples. In another embodiment, the cured tobacco material produced by the production method of the present invention can be used in production of any of the above-listed products after being processed into the form of, for non-limiting example, shred tobacco, granules, fine powder, or liquid (extract-containing liquid).

### [2. Extract]

An extract prepared from a cured tobacco material in accordance with the present invention falls within the scope of the present invention. The extract can be prepared by a preparation method that involves an extracting step including extracting one or more components from a cured tobacco material.

The preparation of an extract is carried out by, for example: adding a suitable solvent such as an aqueous solvent to a cured tobacco material produced by the production method of the present invention; and allowing separation to occur between a solvent-soluble component and a cured tobacco material residue. For non-limiting example, the cured tobacco material for use in preparing the extract may have been subjected to an additional processing treatment accompanied by changes of components and/or physical properties, such as steaming, roasting, or swelling. The obtained extract may be subjected to column cleanup, filtration, or the like depending on the purpose, for removal of unwanted components or for concentrating a target component. Concentration via freeze-drying or via distillation under reduced pressure, or the like method, may be carried out. The extract may be dried if needed. The extract prepared through such a step can be an extract obtained by removing unwanted components from a cured tobacco material, or an extract that contains only desired components. Thus, the extract can be used in production of a variety of products. For example, a method of preparing an extract involves a step including adding an aqueous solvent such as water to a cured tobacco material obtained by the foregoing production method and allowing separation to occur between a water-soluble tobacco extract and a cured tobacco material residue. In a case where a tobacco product is produced from a tobacco extract, the extract, which has been subjected to removal of unwanted components or the like, and the residue can be mixed together again and cured to be used in the production of the tobacco product.

### [4. Tobacco product]

The present invention also provides a tobacco product produced using, as a source material(s), (i) a cured tobacco material produced by the production method of the present invention and/or (ii) an extract prepared from the cured tobacco material. The tobacco product is produced from the cured tobacco material, from a further processed cured tobacco material, or from an extract. The cured tobacco material, the further processed cured tobacco material, or the extract may constitute the entire tobacco material in the tobacco product, or may constitute a part of the tobacco material in the tobacco product. Alternatively, a cured tobacco material which has been produced by the method of the present invention and further processed into the form of powder, liquid, or the like may be added to some other tobacco material in the tobacco product. In a case where the cured tobacco material obtained in the present invention is used as a part of the tobacco material in the tobacco product, the cured tobacco material can be used in any proportion.

The tobacco product can further contain, in addition to the cured tobacco material, some other component(s) such as a flavoring agent, filler, a binder, a pH adjustor, a buffer, a coloring agent, a disintegration auxiliary agent, an antioxidant, a humectant, and/or a preservative.

As has been described, the present invention makes it possible to produce a cured tobacco material having an enhanced threonine content, and the cured tobacco material can be used as a source material for production of various tobacco products.

### [5. Examples of specific aspects in accordance with the present invention]

The present invention includes any of the following <1> to <18>.
<1> A method of producing a cured tobacco material having an enhanced threonine content,
   including producing the cured tobacco material from (i) a modified tobacco plant which has been modified such that expression or activity of an endogenous threonine aldolase is reduced or (ii) a part of the modified tobacco plant,
   said endogenous threonine aldolase being at least one of the following threonine aldolases (a) and (b):
   (a) a threonine aldolase comprised of a polypeptide encoded by a gene containing a polynucleotide which has a sequence identity of not less than 90% to a polynucleotide which has a base sequence shown in SEQ ID NO:1; and
   (b) a threonine aldolase comprised of a polypeptide encoded by a gene containing a polynucleotide which has a sequence identity of not less than 90% to a polynucleotide which has a base sequence shown in SEQ ID NO:3.
<2> A method of producing a cured tobacco material having an enhanced threonine content,
   including producing the cured tobacco material from (i) a modified tobacco plant which has been modified such that expression or activity of an endogenous threonine aldolase is reduced or (ii) a part of the modified tobacco plant,
   said endogenous threonine aldolase being at least one of the following threonine aldolases (a) and (b):
   (a) a threonine aldolase comprised of a polypeptide which has a sequence identity of not less than 90% to a polypeptide which has an amino acid sequence shown in SEQ ID NO:2; and
   (b) a threonine aldolase comprised of a polypeptide which has a sequence identity of not less than 90% to a polypeptide which has an amino acid sequence shown in SEQ ID NO:4.
<3> The method according to <1> or <2>, wherein a reduction in expression or in activity of the endogenous threonine aldolase in the modified tobacco plant is caused by (i) a mutant allele of a gene coding for the endogenous threonine aldolase or (ii) an exogenous polynucleotide which has been introduced into the modified tobacco plant in order to suppress the expression of the endogenous threonine aldolase.
<4> The method according to <3>, wherein the mutant allele contains a mutation selected from the group consisting of insertion, deletion, frameshift mutation, nonsense mutation, nonstop mutation, and splice-site mutation.
<5> The method according to <4>, wherein the mutation includes (i) an artificial mutation introduced by a method selected from the group consisting of mutation induction, genome editing, and a knockout technique or (ii) a naturally occurring spontaneous mutation.
<6> The method according to <5>, wherein: the artificial mutation is introduced by the mutation induction or the genome editing; and the mutation induction or the genome editing is carried out by a technique selected from the group consisting of exposure to a chemical or radiation, a technique using CRISPR system, a technique using transcription activator-like effector nucleases, a technique using zinc finger nucleases, oligonucleotide-directed mutagenesis, T-DNA insertion, and transposon insertion.
<7> The method according to any one of <3> through <6>, wherein the modified tobacco plant is (i) a homozygote in which alleles of the gene coding for the endogenous threonine aldolase contain the same kind of mutation as each other or (ii) a heterozygote in which alleles of the gene coding for the endogenous threonine aldolase contain respective different kinds of mutation.
<8> The method according to <3>, wherein the exogenous polynucleotide includes a molecule selected from the group consisting of antisense RNA molecules, RNAi molecules, and cosuppressor molecules
<9> The method according to any one of <1> through <8>, wherein the modified tobacco plant is a plant which belongs to a tobacco variety and which is obtained by (i) selfing of a tobacco plant which has been modified such that expression or activity of an endogenous threonine aldolase is reduced or (ii) crossing between tobacco plants each of which has been modified such that expression or activity of an endogenous threonine aldolase is reduced.
<10> The method according to <9>, wherein the modified tobacco plant is a tobacco plant bred through screening based on an indicator selected from the group consisting of the following indicators (a) to (f):
   (a) reduced expression of the endogenous threonine aldolase;
   (b) reduced activity of the endogenous threonine aldolase;
   (c) enhanced threonine content of a heat-treated leaf;
   (d) presence or absence of a mutant allele related to the endogenous threonine aldolase;
   (e) a DNA marker linked to the mutant allele recited in (d); and
   (f) presence or absence of an exogenous polynucleotide introduced in order to suppress the expression of the endogenous threonine aldolase.
<11> The method according to any one of <1> through <10>, wherein the part of the modified tobacco plant is selected from the group consisting of leaves, roots, stems, axillary buds, flowers, seeds, plant cell culture, calli, and protoplasts.
<12> The method according to any one of <1> through <11>, wherein the enhanced threonine content of the cured tobacco material is achieved by curing of the modified tobacco plant or the part of the modified tobacco plant.
<13> The method according to <12>, wherein the curing is a curing selected from the group consisting of flue curing, air curing, fire curing, and sun curing.
<14> The method according to any one of <1> through <13>, wherein the modified tobacco plant is a tobacco plant selected from the group consisting of *Nicotiana tabacum* and *Nicotiana rustica.*
<15> The method according to any one of <1> through <14>, wherein: the cured tobacco material is a leaf material; and the cured tobacco material is 20% or more greater in threonine content than a cured tobacco material prepared from a control tobacco plant or a part thereof, the control tobacco plant being a plant which has not been modified such that expression or activity of a threonine aldolase is reduced.
<16> A cured tobacco material produced by a method as set forth in any one of <1> through <15>.
<17> An extract prepared from a cured tobacco material as set forth in claim 16.
<18> A tobacco product produced from a cured tobacco material as set forth in <16> and/or an extract as set forth in <17>.

The present invention is not limited to the embodiments, but can be altered by a skilled person in the art within the scope of the claims. The present invention also encompasses, in its technical scope, any embodiment derived by combining technical means disclosed in differing embodiments.

### Examples

### [Example 1: Selection and isolation of target gene from Nicotiana tabacum]

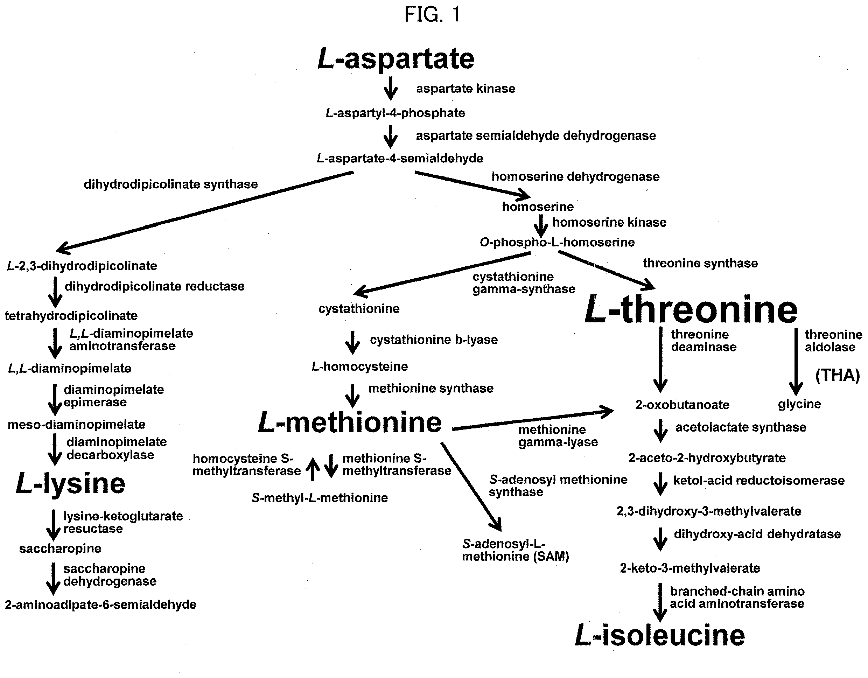

A threonine aldolase (THA) is an enzyme involved in metabolism of threonine in living beings, and is known to be encoded by each of the two genes: THA1 (At1g08630) and THA2 (At3g04520) in *Arabidopsis thaliana* (Non-patent Literature 10). Fig. 1 shows metabolism pathways of amino acids derived from aspartic acid.

Through a BLAST search on a public nucleic acid database, three kinds of tobacco gene having a homology to a THA gene of *A. thaliana* were identified. Of these, two kinds of tobacco gene resided on the S genome of tobacco, whereas one kind of tobacco gene resided on the T genome of tobacco. Since one gene on the S genome and one gene on the T genome have a sequence identity of as high as 99% to each other in terms of amino acids, it was inferred that these two genes have the same function. Of these genes, the one on the S genome was named NtTHA1s, whereas the one on the T genome was named NtTHA1t. The remaining gene was found to have an identity of 87% to the other two genes in terms of amino acids, and was named NtTHA2. The coding regions (CDS) of NtTHA1s, NtTHA1t, and NtTHA2 are shown in SEQ ID NOs:1, 3, and 5, respectively, and the amino acid sequences of their translation products are shown in SEQ ID NOs:2, 4, and 6, respectively.

### [Example 2: Verification of expression of THA genes in tobacco]

For verification of the expression of the THA genes in tobacco, RNA was extracted from a seedling 10 days after being seeded, a lamina of a nursery plant 4 weeks after being seeded, a root of a nursery plant 5 weeks after being seeded, heat-treated laminae, and seeds, of cultivar Tsukuba No. 1, and the extracted RNA was subjected to RT-PCR. The heat treatment of each lamina was carried out by exposing a leaf cut from an individual 8 weeks after being seeded to a condition involving a temperature of 37°C and a humidity of 85% for 0, 8, 24, or 48 hours. The RNA of seed was prepared from 10, 25, 50 or 100 seeds. Total RNA was extracted with the use of an RNeasy Plant Mini Kit (QIAGEN). Synthesis of cDNA was carried out with the use of a High Capacity RNA-to-cDNA Master Mix (Thermo Fisher Scientific Inc.) This was carried out in accordance with the manual coming with the extraction kit.

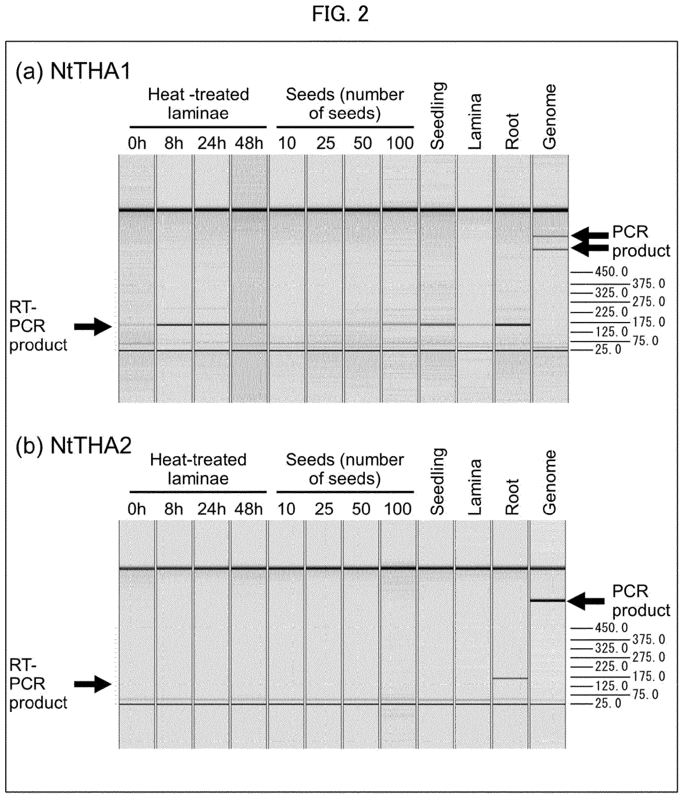

RT-PCR of the NtTHA1 and NtTHA2 genes was carried out using specific primer sets shown in Table 1 and a PrimeSTAR (registered trademark) Max DNA Polymerase (TAKARA BIO INC.) For detection of the NtTHAl genes, a primer set that concurrently amplifies two orthologous genes (NtTHA1_s and NtTHA1_t) was used. Genomic DNA from cultivar Tsukuba No. 1 was used as a template for positive control of PCR amplification. The obtained PCR products were subjected to electrophoresis and checked. Fig. 2 shows the expressions of the NtTHAl and NtTHA2 genes in various tissues of *Nicotiana tabacum.* (a) of Fig. 2 shows the expression of the NtTHAl genes, and (b) of Fig. 2 shows the expression of the NtTHA2 gene. The NtTHAl genes were found to be expressed in all the samples analyzed. The NtTHAl genes were found to be expressed especially strongly in root, seedling, and heat-treated laminae. On the other hand, the NtTHA2 gene was found to be expressed only in root.

**[Table 1]**

| Gene | Forward or Reverse | Sequence (SEQ ID NO) |
|---|---|---|
| NtTHA1 | F | GGGGTCAAAGATCAGCGAAACAGAGA (SEQ ID NO:7) |
| | R | TCTTCGGCCACTCCTGCTAAAGCTC (SEQ ID.NO:8) |
| NtTHA2 | F | TACTGAAAGGGTCAAAGGTCAGTAG (SEQ ID NO:9) |
| | R | TTCTTCTGCCTCTCCCGCCAAAGTTT (SEQ ID NO: 10) |

### [Example 3: Screening for genetically mutated plant]

### (Screening for plant in which NtTHA1_s gene is mutated and plant in which NtTHA1_t gene is mutated)

Lines whose NtTHA1_s gene and NtTHA1_t gene contain some mutation were screened from the EMS mutant population (M2) of *Nicotiana tabacum* (Tsukuba No. 1). The screening was carried out by SingleStrand Conformation Polymorphism (SSCP) analysis. PCR fragments to be subjected to the SSCP analysis were amplified using NtTHA1-gene-specific primer sets shown in Table 2 and a Multiplex PCR Kit (QIGEN). Then, each amplified product was analyzed with the use of a capillary electrophoresis apparatus (3130xl PRISM (registered trademark) DNA analyzer, Thermo Fisher Scientific Inc.) filled with a POP (trademark) Conformational Analysis Polymer (Thermo Fisher Scientific Inc.) The obtained data was analyzed using a GeneMapper (registered trademark) Version 4.0 (Thermo Fisher Scientific Inc.), and the lines from which a polymorphic PCR product was amplified were screened as candidates for a target mutated plant.

**[Table 2]**

| Gene | Forward or Reverse | Sequence (SEQ ID NO) |
|---|---|---|
| NtTHA1s | F | CAGGGAAAATGGTGATGAGAACC (SEQ ID NO:11) |
| | R | ATGCACCAATATGAGGATCGCTG (SEQ ID NO:12) |
| NtTHA1t | F | CAGGGAAAATGGTGATGAGAACC (SEQ ID NO:13) |
| | R | ACCAGATAATGTACCAAATAGAGAG (SEQ ID NO: 14) |

Each of the PCR products amplified from the screened candidate lines was cloned with the use of a TOPO (registered trademark) TA Cloning Kit (Thermo Fisher Scientific Inc.) The resulting colony was cultured overnight in a LB/Km liquid culture medium, and then a plasmid was extracted with the use of a QiaPrep Spin Miniprep Kit (QIAGEN). Then, using the obtained plasmid and a BigDye (registered trademark) Terminator v3.1 Cycle Sequencing Kit (Thermo Fisher Scientific Inc.), the base sequence of the PCR product was determined. The obtained data was analyzed with the use of ATGC (Ver.7) Sequence Assembly Software (GENETYX CORPORATION), and the mutation introduced in each of the NtTHAl genes, if any, was identified. All the processes in the above procedures were carried out in accordance with the manuals coming with the kits.

Finally, two kinds of mutant allele (tha1-s1, tha1-s2), which are the same as those of the NtTHA1_s gene except that nonsense mutations are introduced, were identified. Furthermore, one kind of mutant allele (tha1-t), which is the same as that of the NtTHA1_t gene except that a nonsense mutation is introduced, was identified. Each mutation was found within exon 1 (450 bp). Among the above two kinds of mutant allele, tha1-s1 was found to (i) have the same base sequence as that of a wild type NtTHA1s shown in SEQ ID NO:1 except that the base C at the 223rd position is substituted by T (SEQ ID NO:33) and (ii) encode the same amino acid sequence as that of the wild type NtTHA1s shown in SEQ ID NO:2 except that the amino acid at the 75th position is a stop codon. Tha1-s2 was found to (i) have the same base sequence as that of the wild type NtTHA1s shown in SEQ ID NO:1 except that the base C at the 376th position is substituted by T (SEQ ID NO:34) and (ii) encode the same amino acid sequence as that of the wild type NtTHA1s shown in SEQ ID NO:2 except that the amino acid at the 126th position is a stop codon. Furthermore, tha1-t was found to (i) have the same base sequence as that of a wild type NtTHA1t shown in SEQ ID NO:3 except that the base C at the 121st position is substituted by T (SEQ ID NO:35) and (ii) encode the same amino acid sequence as that of the wild type NtTHA1t shown in SEQ ID NO:4 except that the amino acid at the 41st position is a stop codon.

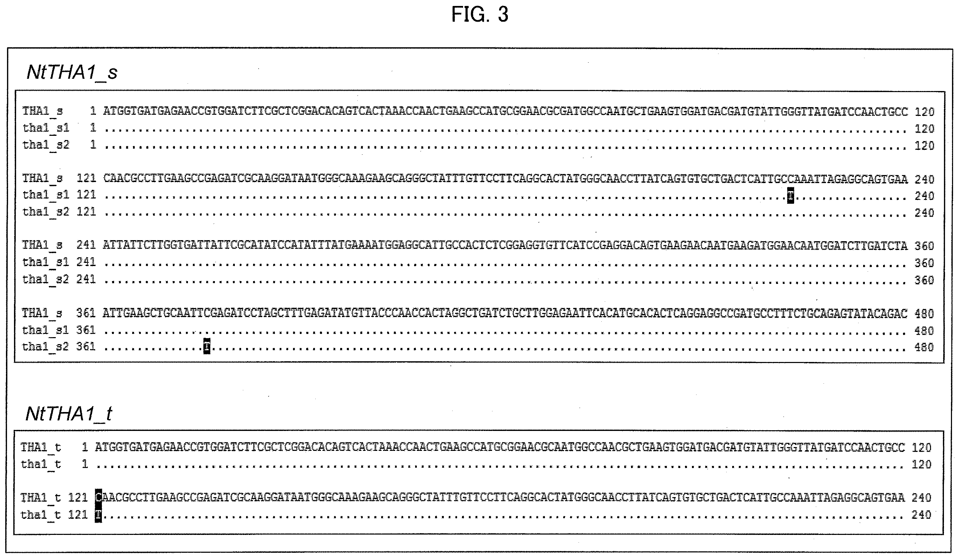

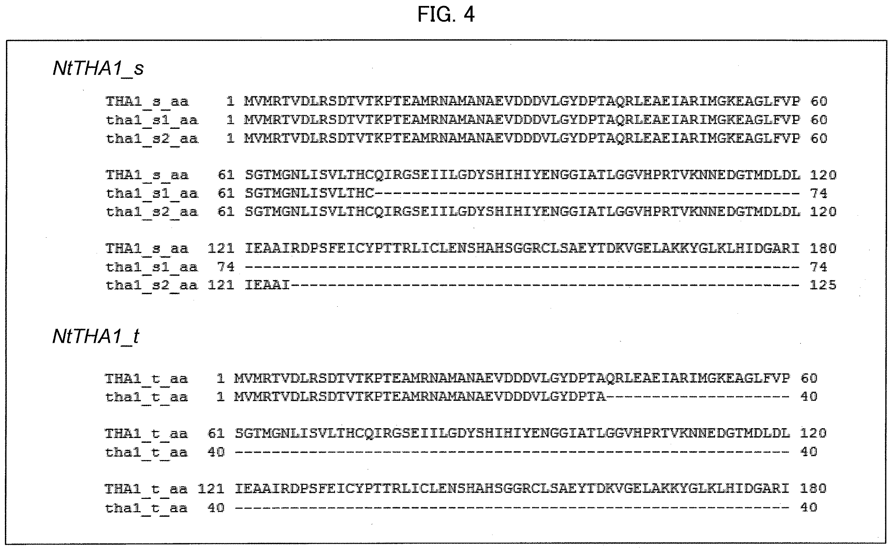

Fig. 3 shows base sequence alignment of the wild type alleles and the mutant alleles of the NtTHAl genes. Fig. 4 shows amino acid sequence alignment of translation products of the wild type alleles and the mutant alleles of the NtTHAl genes.

### (Breeding of double mutant)

For determining the influence of the loss of function of the NtTHAl genes, a double mutant plant which is homozygous for tha1-s1 or tha1-s2 and which is also homozygous for tha1-t was bred.

First, as described above, M2 plants each of which is homozygous for tha1-s1, tha1-s2, or tha1-t were screened. Next, two F2 populations derived from crossing between these M2 plants were prepared. From each population, some individuals in which each of the NtTHAl gene loci is homozygous (each locus is occupied by the same mutant alleles) were screened, and two types of double mutant lines (tha1-s1/tha1-t and tha1-s2/tha1-t) were obtained. Furthermore, from each of the above populations, a plant in which each of the NtTHAl gene loci is homozygous (each locus is occupied by wild type alleles) (THA1-s/THA1-t) was screened and used as a control.

The genotype of each of the two NtTHAl gene loci was determined through SNP Genotyping Assay with the use of a TaqMan (registered trademark) MGBprobe. The assay was carried out with the use of a TaqMan (registered trademark) Genotyping Master Mix (Thermo Fisher Scientific Inc.) and StepOnePlus Real-Time PCR System (trademark) (Thermo Fisher Scientific Inc.) The primer and probe sets used in the assay are shown in Table 3.

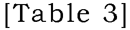

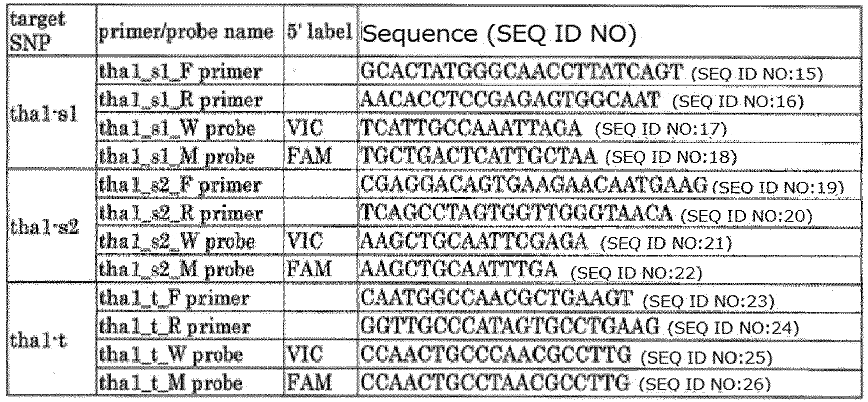

### [Example 4: Analysis of gene expression]

The expression of the NtTHAl genes in the double mutants was assessed in the following manner. First, RNA was extracted from a nursery plant 6 weeks after being seeded, and cDNA was synthesized with the use of a PrimeScript (trademark) RT reagent kit with gDNA Eraser (TAKARA BIO INC.). Real-Time PCR was carried out using the obtained cDNA as a template and using a primer and probe set shown in Table 4 and a TaqMan (registered trademark) Fast Advanced Master Mix (Thermo Fisher Scientific Inc.) The analysis was carried out with the use of a StepOnePlus Real-Time PCR System (Thermo Fisher Scientific Inc.) The processes of PCR were carried out in accordance with the manuals coming with the kit and apparatus.

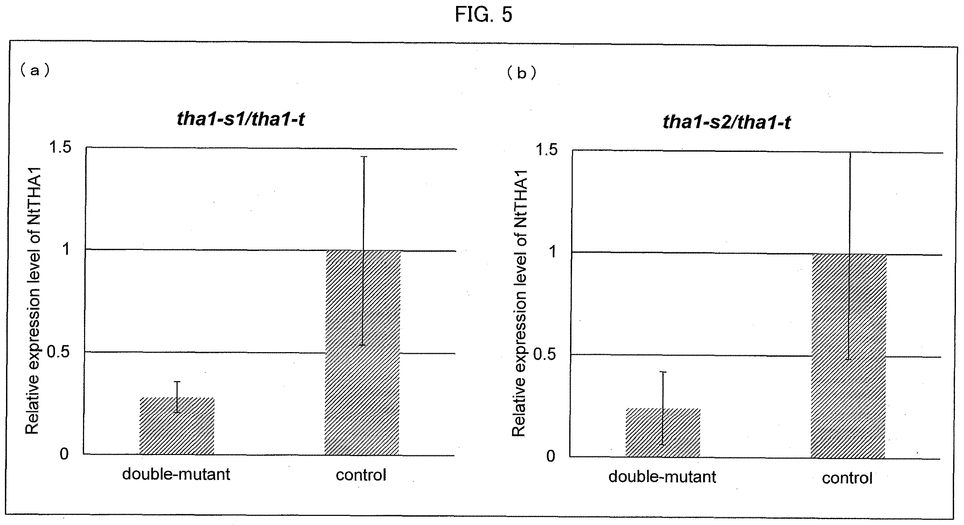

The expression level of the NtTHAl genes was analyzed by a comparative CT method using, as an internal standard, elongation factor 1-alpha (EF-1 alpha) gene (accession No. AF120093). A primer and probe set was designed such that the primer and probe each have a base sequence that is completely identical between NtTHA1s and NtTHA1t, with the use of a Primer Express (registered trademark) 3.0 (Thermo Fisher Scientific Inc.) (Table 4). The expression level of the NtTHAl genes in the double mutants and the control plant is shown in Fig. 5. (a) of Fig. 5 shows the expression level in the tha1-s1/tha1-t double mutant, and (b) of Fig. 5 shows the expression level in the tha1-s2/tha1-t double mutant. Each vertical axis in Fig. 5 shows the expression level (relative expression level) relative to the expression level of each NtTHAl gene in the control plant (THA1-s/THA1-t). It was found that the expression level in each double mutant is significantly lower than the control plant.

**[Table 4]**

| target | primer/probe name | 5' label | Sequence (SEQ ID NO) |
|---|---|---|---|
| NtTHA1 | THA1_F primer | | TAGGTCAGGGAAAATGGTGATGA (SEQ ID NO:27) |
| | THA1_R primer | | CGTTCCGCATGGCTTCAG (SEQ ID NO:28) |
| | THA1 probe | FAM | ACCGTGGATCTTCGCTCGGACACA (SEQ ID NO:29) |
| EF·1 alpha | EF·1_F primer | | CTAAGGGTGCTGCCAGCTTT (SEQ ID NO:30) |
| | EF·1_R primer | | GTCAAGCACTGGAGCATATCCA (SEQ ID NO:31) |
| | EF·1 probe | VIC | ATCATGAACCATCCAGGACAGATTGG (SEQ ID NO:32) |

### [Example 5: Cultivation of plant and analysis of free threonine content]

### (Observation of growth state and appearance)

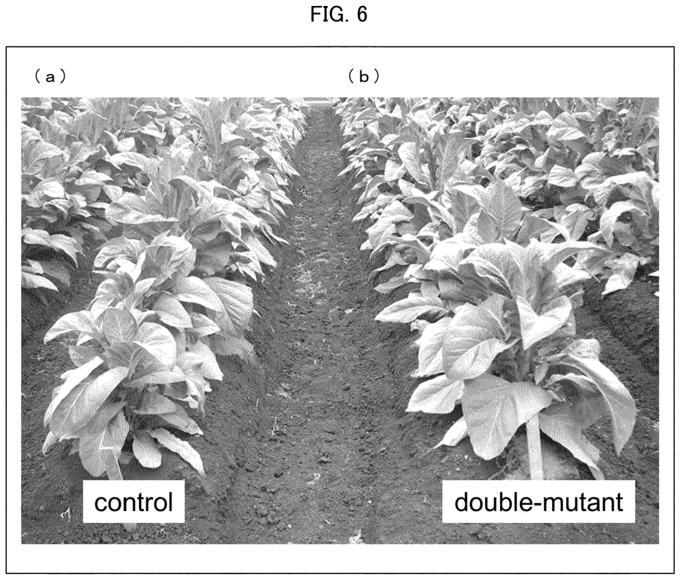

The double mutant (tha1-s2/tha1-t) screened as described above and the control plant were cultivated in a field in accordance with a general tobacco cultivation method. (b) of Fig. 6 shows the double mutant, and (a) of Fig. 6 shows the control plant.

As is clear from Fig. 6, there was no difference found in growth state or appearance between the double mutant and control plant.

### (Analysis of free threonine content of plant)

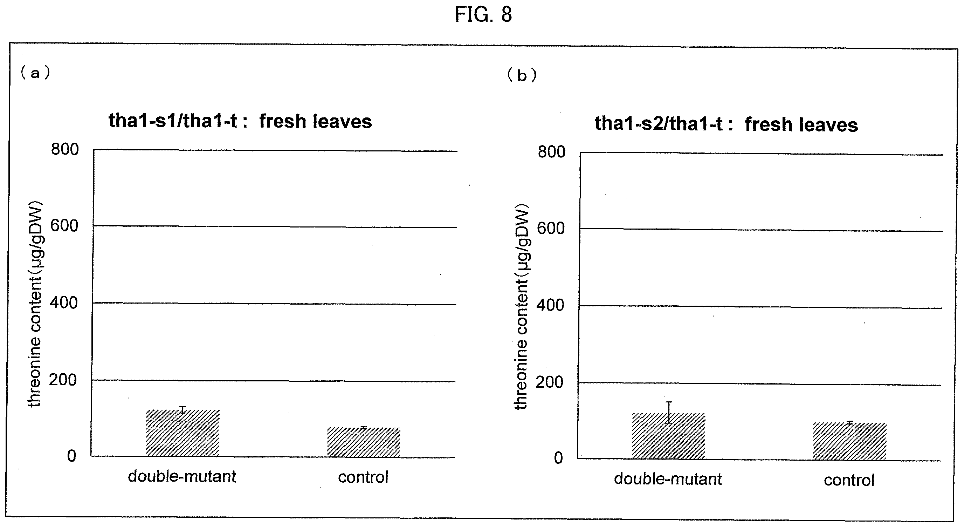

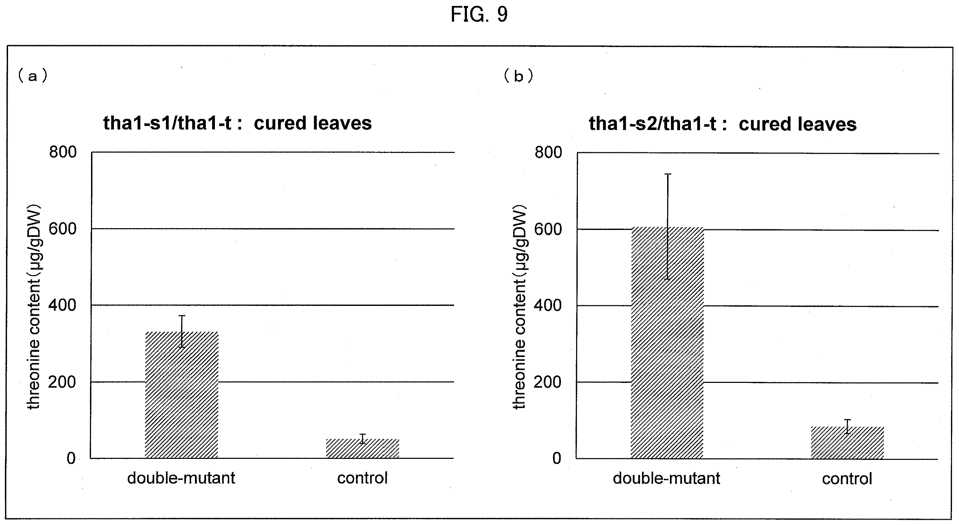

Next, leaves were taken from each of the double mutants and from the control plant, and the free threonine content of fresh leaves and flue-cured leaves were analyzed. A standard curing schedule (Tabacco Curing Schedule) for the flue-curing is shown in Fig. 7. The horizontal axis in Fig. 7 shows time (hr), the left vertical axis in Fig. 7 shows temperature (°C), and the right vertical axis in Fig. 7 shows relative humidity (RH, %). Fig. 8 shows the free threonine content of fresh leaves of the double mutants and the control plant. Fig. 9 shows the free threonine content of the flue-cured leaves (cured leaves) of the double mutants and the control plant.
(a) of Fig. 8 and (a) of Fig. 9 show the free threonine content (threonine content) of the tha1-s1/tha1-t double mutant and the control plant. (b) of Fig. 8 and (b) of Fig. 9 show the free threonine content (threonine content) of the tha1-s2/tha1-t double mutant and the control plant. Each vertical axis in Figs. 8 and 9 shows free threonine content (µg/gDW) per dry weight.

As shown in Fig. 8, the threonine content of a fresh leaf immediately after taken from each double mutant was found not to be significantly different from that of the control plant. On the other hand, as shown in Fig. 9, the free threonine content of a flue-cured leaf of each double mutant was found to be much greater than that of the control.

### [Example 6: Analysis of influence of flue-curing temperature on free threonine content]

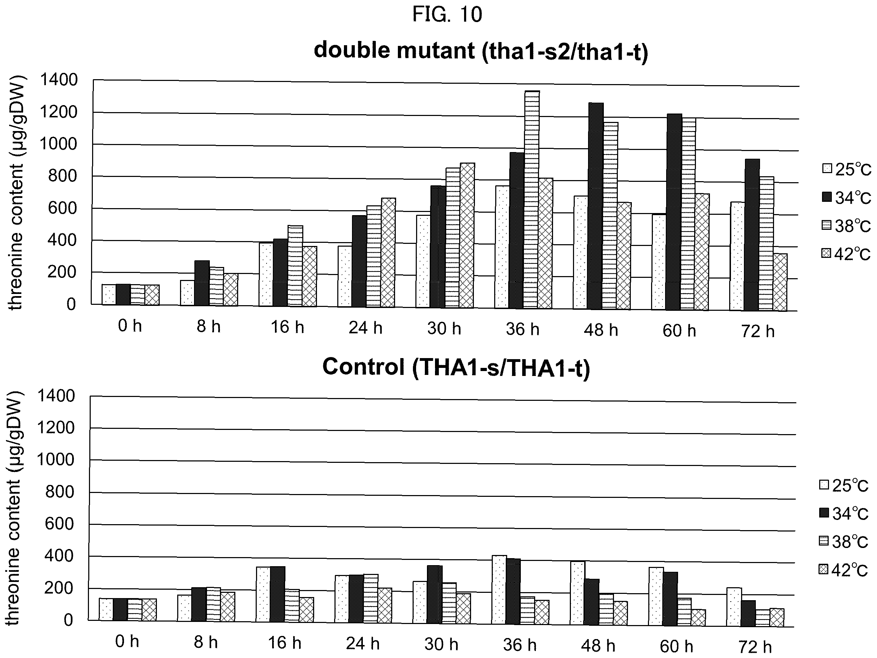

What influence the flue-curing temperature imposes on the free threonine content of the leaves of the double mutant (tha1-s2/tha1-t) and the control plant (THA1-s/THA1-t) was studied. Three tobacco leaves which were being cured were randomly selected 0h, 8h, 16h, 24h, 30h, 36h, 48h, 60h, and 72h after the start of the curing, and a lamina measuring about 10 cm square was taken from each leaf. A collection of three laminae was used as one sample, and the sample was measured for free threonine content. The flue-curing chamber maintained a constant temperature (25°C, 34°C, 38°C, or 42°C) and was operated at a humidity of 85%. Fig. 10 shows threonine content in cases where the leaves taken from the NtTHAl-genes double mutant and the control tobacco plant were flue-cured at each temperature. The leaves of the double mutant were found to have higher free threonine content than the wild type at each temperature 24 hours after the start of the curing and later.

### [Example 7: Analysis of various genotypes]

For further study of the expression of the NtTHA1 genes in individuals of different genotypes, primers and probe to specifically detect the NTtHA1s gene or the NTtHA1t gene were prepared (Table 5). The "position" column in Table 5 indicates positions in the sequence shown in SEQ ID NO:1 or 3.

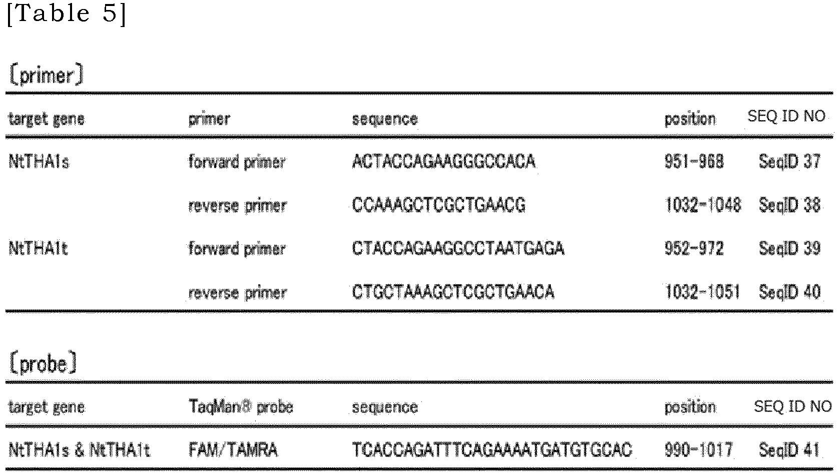

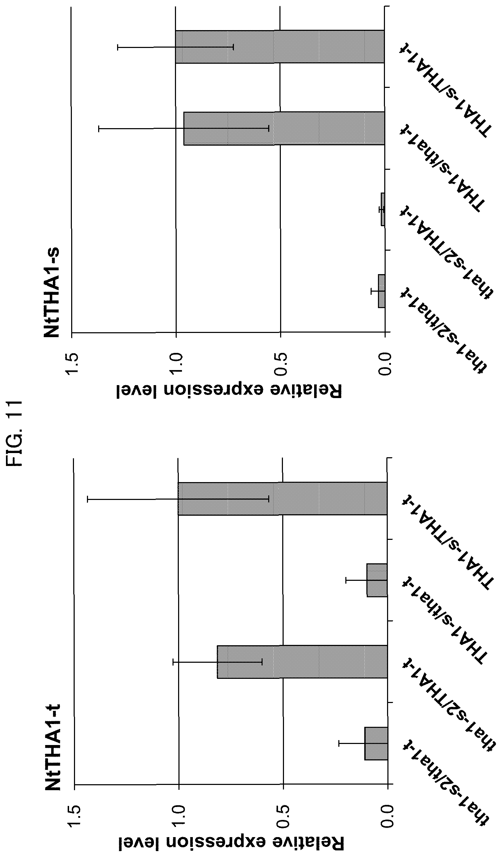

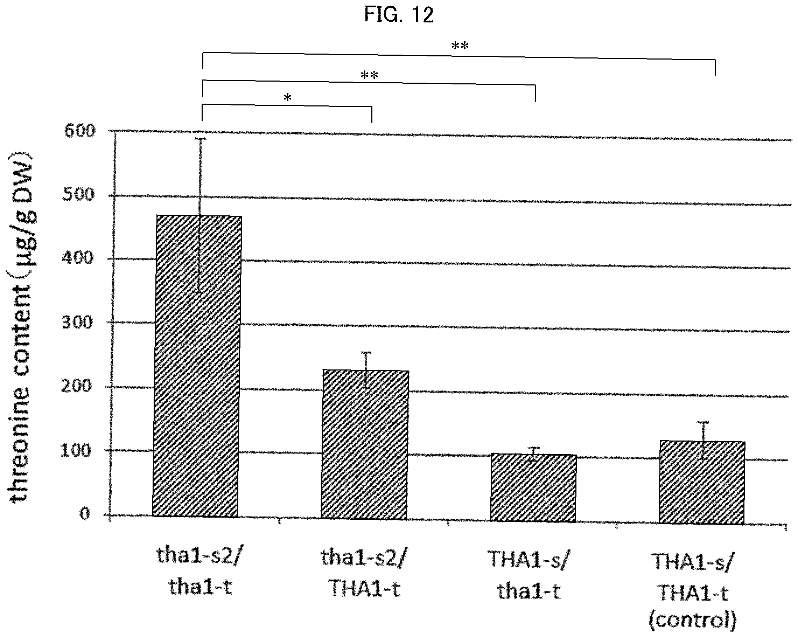

The expression of the NtTHAl genes was studied on plants of four genotypes: (tha1-s2/tha1-t, tha1-s2/THA1-t, THA1-s/tha1-t, THA1-s/THA1-t), in each of which each NtTHAl gene locus is homozygous (each NtTHAl gene locus is occupied by two wild type alleles or by two mutant alleles). Fig. 11 shows the expression of the NtTHAl genes in the individuals of the respective genotypes. As shown in Fig. 11, the expression of the NtTHA1s gene in the tha1-s2 homozygotes was found to have been reduced. Similarly, the expression of the NtTHA1t gene in the tha1-t homozygotes was found to have been reduced. Leaves taken from these plants were flue-cured, and the cured leaves were analyzed for their free threonine content. Fig. 12 shows the free threonine content of cured leaves derived from the individuals of the respective genotypes. The free threonine content of the cured leaves of the double mutant was found to be significantly higher than those of the other genotypes.

### [Example 7: Air curing]

A double mutant (tha1-s1/tha1-t) and a tobacco variety of an air-curing type (TN90) were crossed to obtain seeds of BC1F2. DNA was extracted from seedlings of the BC1F2 plants, and the genotype of each NtTHA1 gene was studied. Two-hundred or more individuals of the double mutant (tha1-s1/tha1-t) and 200 or more individuals of a control plant (THA1-s/THA1-t) were screened and cultivated in a field in accordance with a general tobacco cultivation method.

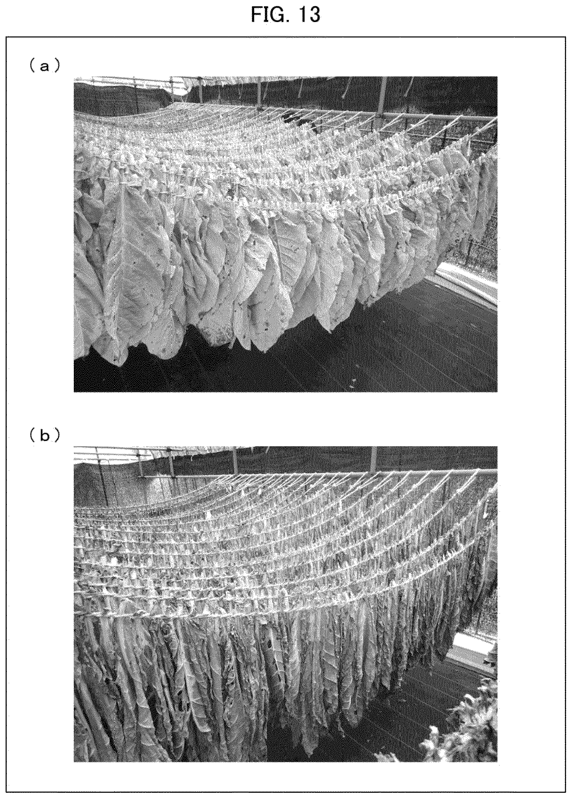

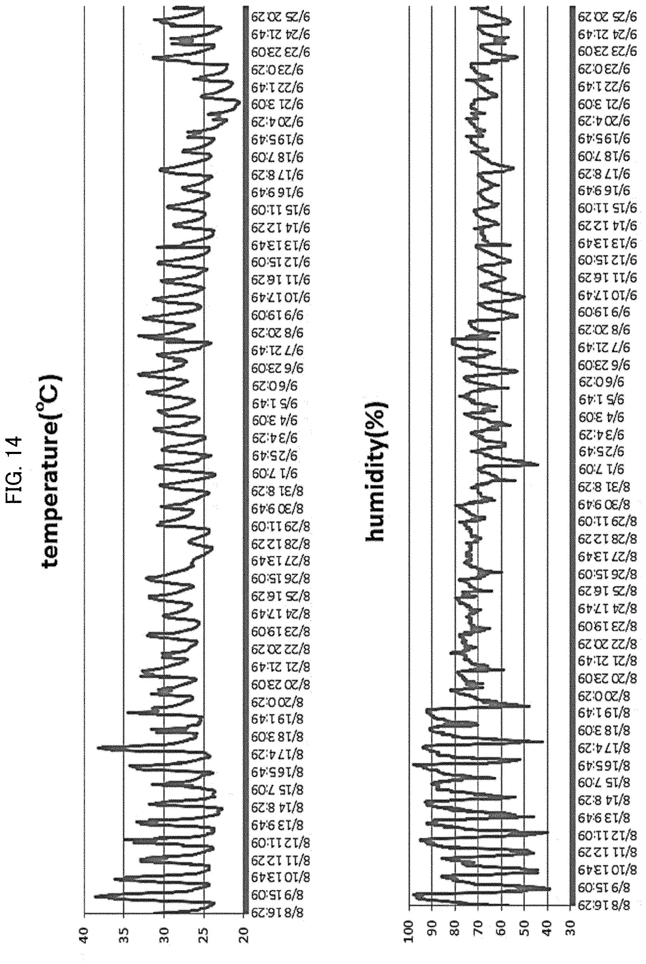

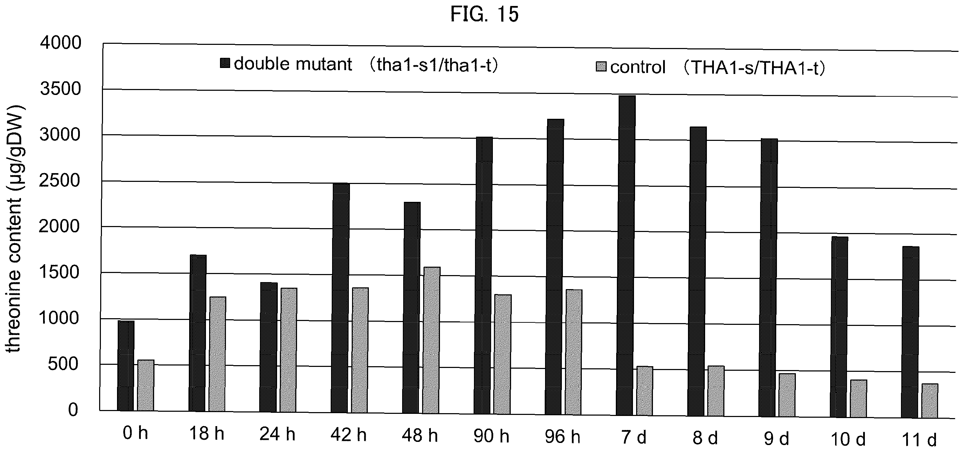

The air curing of tobacco leaves was carried out in accordance with a generally-used method. First, harvested tobacco leaves were hung inside a greenhouse covered in a sun-shade net for 11 days, and thereby curing was promoted. The tobacco leaves were further cured in a curing shed for 37 days. Fig. 13 shows the tobacco leaves which are being air-cured. (a) of Fig. 13 shows the leaves immediately after the start of hanging, and (b) of Fig. 13 shows the leaves 10 days after the start of hanging. Fig. 14 shows transitions of ambient temperature and humidity around the tobacco leaves during air-curing. Three tobacco leaves were randomly selected 0h, 18h, 24h, 42h, 48h, 90h, 96h, 7d, 8d, 9d, 10d, and 11d after the start of the hanging, and a lamina measuring about 10 cm square was taken from each leaf. A collection of three laminae was used as one sample, and the sample was measured for free threonine content. Fig. 15 shows transitions of free threonine content of the tobacco leaves during air-curing. As shown in Fig. 15, the free threonine content of the double mutant stayed high as compared to the control, over the course of air curing.

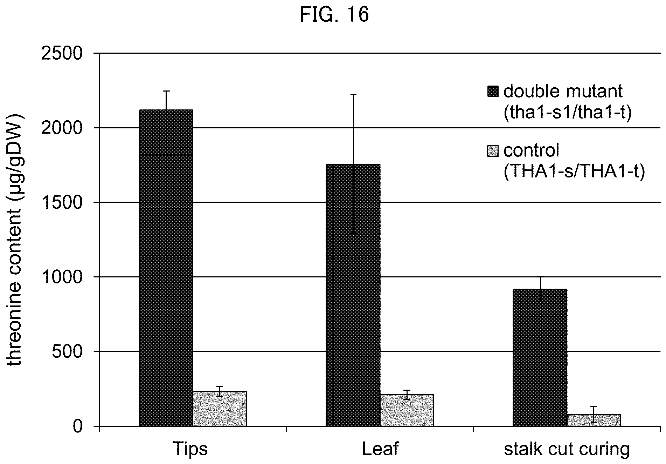

Leaves at the tip of a stem (in Fig. 16, indicated as "Tips"), leaves positioned higher than the middle of a stem (in Fig. 16, indicated as "Leaves"), and leaves remaining on a stem were also cured in the same manner. Fig. 16 shows the free threonine content of these cured leaves of the double mutant and the control. The cured leaves of every position of the double mutant were found to have higher free threonine content than those of the control (Fig. 16). The stalk-cut cured leaves of the double mutant were also found to have higher free threonine content than the control.

### [Example 8: Genome editing]

Genome editing was carried out with use of CRISPR-Cas9 in accordance with the method of Li et al. (Li et al., Nat Biotechnol. 2013 Aug; 31(8): 688-912013). A target sequence (5'-GGAGGCATTGCCACTCTCGGAGG-3': SEQ ID NO:42) was selected from a region common to the NtTHA1s gene (SEQ ID NO:1) and the NtTHA1t gene (SEQ ID NO:3). This sequence is a base sequence extending from the 283rd position to the 305th position of SEQ ID NO:1 or SEQ ID NO:3, and the AGG at the 3' terminus (underlined) is a PAM sequence. An all-in-one plant expression vector for expression of a plant codon-optimized SpCas9 (pcoCas9) and synthetic-guide RNA (sgRNA) in tobacco was prepared by: inserting pcoCas9 gene into pRI201-AN (Takara Bio Inc.) such that the pcoCas9 gene resides downstream of the CaMV 35S promoter and between NdeI and SalI; and inserting a sgRNA expression cassette linked to a Arabidopsis U6 polymerase III promoter between KpnI and BamHI.

A cotyledon fragment from tobacco (*Nicotiana tabacum,* SR-1) 10 days after being seeded was infected with an *Agrobacterium* strain (LBA4404) containing the above vector, and thereby a transformant was prepared. Three T0 (M1) individuals each containing one base insertion in both the NtTHA1s gene and the NtTHA1t gene were screened, and were selfed. From each line, a T1 (M2) plant which contains no pcoCas9/sgRNA construct and in which both the NtTHA1s gene and the NtTHA1t gene are homozygous mutant genes was screened. The M2 plants were selfed to obtain M3 seeds. The NtTHAl genes of each plant of these lines were found to contain one base (T or G) insertion between the 280th base and the 281st base (Table 6). SEQ ID NO:49 shows the base sequence having the same sequence as a wild type NtTHA1s shown in SEQ ID NO:1 except that a single base (T) is inserted between the 280th base and the 281st base. SEQ ID NO:50 shows the base sequence having the same sequence as a wild type NtTHA1t shown in SEQ ID NO:3 except that a single base (T) is inserted between the 280th base and the 281st base, and SEQ ID NO:51 shows the base sequence having the same sequence as the wild type NtTHA1t shown in SEQ ID NO:3 except that a single base (G) is inserted between the 280th base and the 281st base.

**[Table 6]**

| allele | line | mutation | position |
|---|---|---|---|
| NtTHA1_s | 3_10 | T-insertion | 280/281 |
| | 18_16 | T-insertion | 280/281 |
| | 48_78 | T-insertion | 280/281 |
| NtTHA1_t | 3_10 | T-insertion | 280/281 |
| | 18_16 | G-insertion | 280/281 |
| | 48_78 | T-insertion | 280/281 |

For study of the expression of the NtTHAl genes in genome-edited tobacco plants, new primer and probe sets (Set A and Set B) were designed upstream and downstream of a mutant position, respectively (Table 7). The design was carried out with reference to NCBI Reference Sequence (RefSeq) Accession Nos. XM_016599601.1 and XM_016606047.1. Each of the sets enables concurrent detection of both the NtTHA1_s gene and the NtTHA1_t gene. The probe of Set A matches the base sequence of the 13rd to 36th bases of SEQ ID NO:1 or SEQ ID NO:3. The probe of Set B matches the base sequence of the 694th to 711th bases of SEQ ID NO:1 or SEQ ID NO:3.

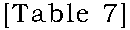

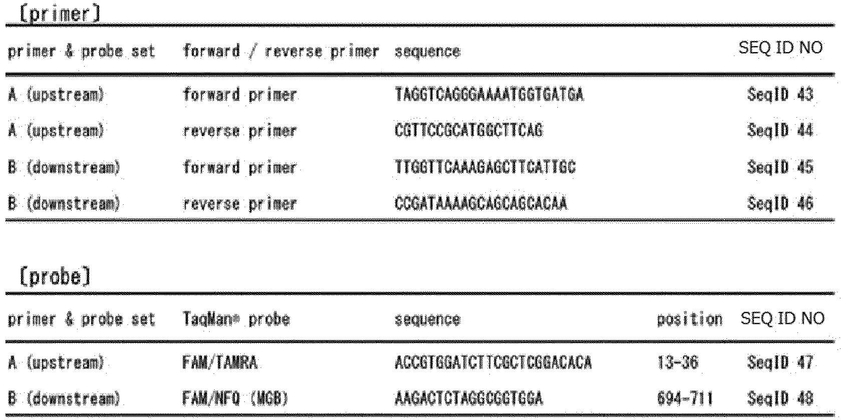

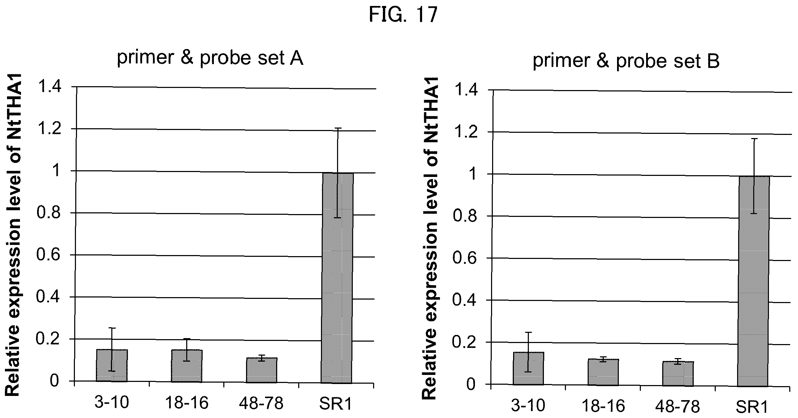

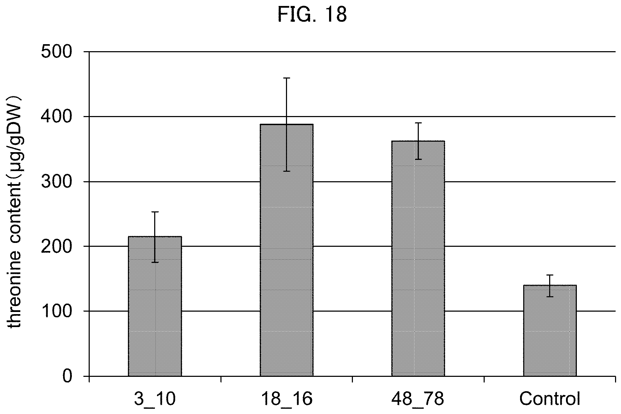

Leaves were taken from M3 seedlings and studied for expression level of the NtTHAl genes (3 individuals per line). Fig. 17 shows expression level of the NtTHAl genes in genome-edited tobacco plants. As shown in Fig. 17, the genome-edited individuals were found to have significantly lower expression level of the NtTHAl genes than the wild type SR-1. Furthermore, these seedlings were transferred to 12 cm terra-cotta pots, and grown in a greenhouse. Three leaves were taken from each of three individuals in the flower initiation stage, were subjected to flue-curing, and analyzed for free threonine content. The temperature and humidity at which the flue curing was carried out were 38°C/ 100%RH/ 18h, 38°C/88%RH/30h, 45°C/85%RH/48h, or 50°C/20%RH/72h. Fig. 18 shows the free threonine content of cured leaves of the genome-edited tobacco plants. The free threonine content of flue-cured leaves was found to be higher in the genome-edited individuals than in the wild type SR-1 (Fig. 18).

### Industrial Applicability

The present invention is applicable to production of a cured tobacco material having an enhanced threonine content and production of a tobacco product that contains the cured tobacco material and/or an extract thereof.

## Claims

1. A method of producing a cured tobacco material having an enhanced threonine content,
comprising producing the cured tobacco material from (i) a modified tobacco plant which has been modified such that expression or activity of an endogenous threonine aldolase is reduced or (ii) a part of the modified tobacco plant,
said endogenous threonine aldolase being at least one of the following threonine aldolases (a) and (b):
(a) a threonine aldolase comprised of a polypeptide encoded by a gene containing a polynucleotide which has a sequence identity of not less than 90% to a polynucleotide which has a base sequence shown in SEQ ID NO: 1; and
(b) a threonine aldolase comprised of a polypeptide encoded by a gene containing a polynucleotide which has a sequence identity of not less than 90% to a polynucleotide which has a base sequence shown in SEQ ID NO:3.

2. A method of producing a cured tobacco material having an enhanced threonine content,
comprising producing the cured tobacco material from (i) a modified tobacco plant which has been modified such that expression or activity of an endogenous threonine aldolase is reduced or (ii) a part of the modified tobacco plant,
said endogenous threonine aldolase being at least one of the following threonine aldolases (a) and (b):
(a) a threonine aldolase comprised of a polypeptide which has a sequence identity of not less than 90% to a polypeptide which has an amino acid sequence shown in SEQ ID NO: 2; and
(b) a threonine aldolase comprised of a polypeptide which has a sequence identity of not less than 90% to a polypeptide which has an amino acid sequence shown in SEQ ID NO:4.

3. The method according to claim 1 or 2, wherein a reduction in expression or in activity of the endogenous threonine aldolase in the modified tobacco plant is caused by (i) a mutant allele of a gene coding for the endogenous threonine aldolase or (ii) an exogenous polynucleotide which has been introduced into the modified tobacco plant in order to suppress the expression of the endogenous threonine aldolase.

4. The method according to claim 3, wherein the mutant allele contains a mutation selected from the group consisting of insertion, deletion, frameshift mutation, nonsense mutation, nonstop mutation, and splice-site mutation.

5. The method according to claim 4, wherein the mutation includes (i) an artificial mutation introduced by a technique selected from the group consisting of mutation induction, genome editing, and a knockout technique or (ii) a naturally occurring spontaneous mutation.

6. The method according to claim 5, wherein:
the artificial mutation is introduced by the mutation induction or the genome editing; and
the mutation induction or the genome editing is carried out by a technique selected from the group consisting of exposure to a chemical or radiation, a technique using CRISPR system, a technique using transcription activator-like effector nucleases, a technique using zinc finger nucleases, oligonucleotide-directed mutagenesis, T-DNA insertion, and transposon insertion.

7. The method according to any one of claims 3 through 6, wherein the modified tobacco plant is (i) a homozygote in which alleles of the gene coding for the endogenous threonine aldolase contain the same kind of mutation as each other or (ii) a heterozygote in which alleles of the gene coding for the endogenous threonine aldolase contain respective different kinds of mutation.

8. The method according to claim 3, wherein the exogenous polynucleotide includes a molecule selected from the group consisting of antisense RNA molecules, RNAi molecules, and cosuppressor molecules

9. The method according to any one of claims 1 through 8, wherein the modified tobacco plant is a plant which belongs to a tobacco variety and which is obtained by (i) selfing of a tobacco plant which has been modified such that expression or activity of an endogenous threonine aldolase is reduced or (ii) crossing between tobacco plants each of which has been modified such that expression or activity of an endogenous threonine aldolase is reduced.

10. The method according to claim 9, wherein the modified tobacco plant is a tobacco plant bred through screening based on an indicator selected from the group consisting of the following indicators (a) to (f):
(a) reduced expression of the endogenous threonine aldolase;
(b) reduced activity of the endogenous threonine aldolase;
(c) enhanced threonine content of a heat-treated leaf;
(d) presence or absence of a mutant allele related to the endogenous threonine aldolase;
(e) a DNA marker linked to the mutant allele recited in (d); and
(f) presence or absence of an exogenous polynucleotide introduced in order to suppress the expression of the endogenous threonine aldolase.

11. The method according to any one of claims 1 through 10, wherein the part of the modified tobacco plant is selected from the group consisting of leaves, roots, stems, axillary buds, flowers, seeds, plant cell culture, calli, and protoplasts.

12. The method according to any one of claims 1 through 11, wherein the enhanced threonine content of the cured tobacco material is achieved by curing of the modified tobacco plant or the part of the modified tobacco plant.

13. The method according to claim 12, wherein the curing is a curing selected from the group consisting of flue curing, air curing, fire curing, and sun curing.

14. The method according to any one of claims 1 through 13, wherein the modified tobacco plant is a tobacco plant selected from the group consisting of *Nicotiana tabacum* and *Nicotiana rustica.*

15. The method according to any one of claims 1 through 14, wherein:
the cured tobacco material is a leaf material; and
the cured tobacco material is 20% or more greater in threonine content than a cured tobacco material prepared from a control tobacco plant or a part thereof, the control tobacco plant being a plant which has not been modified such that expression or activity of a threonine aldolase is reduced.

16. A cured tobacco material produced by a method as set forth in any one of claims 1 through 15.

17. An extract prepared from a cured tobacco material as set forth in claim 16.

18. A tobacco product produced from a cured tobacco material as set forth in claim 16 and/or an extract as set forth in claim 17.
